# EUROPEAN PATENT APPLICATION

(11) **EP 0 887 405 A1**
(43) Date of publication of application: **30.12.1998**
(21) Application number: 97401480.5
(22) Date of filing: 25.06.1997
(51) Int. Cl.: C12N 15/11, C12N 15/82, C12N 15/68

(54) **Process for producing stabilised RNA molecules within cells, RNA molecules thus produced and nucleic acid precursors of such molecules**

(71) Applicant: Gene Shears Pty Limited, North Ryde, NSW 2113 (AU)
(72) Inventor: Brown, John, William, Slessor, Inchture, Perthshire PH14 9TQ (GB); Leader, David,John, Dundee DD1 5PP (GB)
(74) Representative: Almond-Martin, Carol

(57) **Abstract**

The invention concerns a process for producing stabilised non-translated RNA molecules within a cell, comprising introducing into a plant or yeast cell a nucleic acid molecule comprising a 〈〈 precursor DNA 〉〉, said precursor DNA containing a coding sequence, or a cluster of coding sequences, each coding sequence encoding :
- a non-translated RNA sequence to be stabilised, which is capable of functionally interacting with a cellular component, and
- stabilising sequences comprising at least one protein binding site and, optionally, sequences capable of giving rise to secondary structure associated with said protein binding site, said stabilising sequences being heterologous with respect to the non-translated RNA sequence to be stabilised, the precursor DNA being under the transcriptional control of regulatory sequences functional in said cell,
   whereby a transcript is produced by transcription of the precursor DNA, and one or more stabilised RNA units comprising the non-translated RNA sequence in association with the stabilising sequences is, or are, then produced by processing of the transcript by the cell's endogenous processing machinery.

## Description

The present invention relates to a process for qualitatively and quantitatively enhancing the production and stability of RNA molecules, particularly non-translated RNA.

Specifically, the present invention concerns a process for producing stabilised RNA molecules within cells from purpose-constructed precursor nucleic acid molecules. The invention also relates to cells containing stabilised non-translated RNA and to the stabilised RNA molecules themselves. The invention further relates to novel promoter sequences particularly adapted for use in producing multiple, stabilised RNA molecules.

Unprotected RNA molecules are unstable in the cellular environment. They are subject to nucleolytic attack by a variety of RNAses, for example exonucleases, and therefore usually require the presence of one or more specialised structural features to provide a minimum protection against degradation. This is the case for both naturally expressed endogenous RNA and for heterologous or synthetic RNA, either transcribed in the cell, or introduced by injection.

These natural protective structural features include 5' caps, for example, 5'-m₇G and trimethylguanosine (5'-m₃G), 3' polyadenylation sequences, secondary structure and protein binding domains. A given RNA molecule may possess one or more of these characteristics. For example, messenger RNA transcribed from RNA polymerase II promoters has both a 5'-m₇G cap and a 3' polyadenylated tail, effectively protecting each extremity of the mRNA from exonucleolytic degradation for a time sufficient to allow translation of the RNA into protein.

Untranslated RNA which naturally occurs in the cell, such as rRNA, tRNA, small nuclear RNA (snRNA), small cytoplasmic RNA etc., does not have a 3' poly A tail, and, depending on the cellular mechanism employed in its production, may or may not have a 5' cap. Secondary structure of the RNA molecule and protein binding domains are thought to play a role in the resistance of these types of RNA to nucleolytic degradation.

Untranslated RNA artificially introduced into the cell as a result of human intervention, for example by injection, or by transcription of recombinant sequences, can be susceptible to degradation because it often does not possess any of the natural 〈〈 protective 〉〉 structures required to resist RNAses. Several attempts have therefore been made to improve the stability of foreign untranslated RNA, such as ribozymes or antisense molecules, by adding stabilising sequences or structures.

For example, a number of workers have added 5'-cap structures and termination sequences, such as polyadenylated tails or T7 termination signals to single or multiple hammerhead ribozymes in an attempt to improve stability of the ribozyme transcript (see for example, Scanlon et al., 1991, Sioud et al., 1992, Chen et al., 1992). However, in some cases, the addition of extensive 3' sequences may interfere with the function of the ribozyme by preventing correct substrate-ribozyme hybrid formation.

An alternative strategy was developed by De Young et al (De Young et al., 1994), who adapted a small nuclear RNA (snRNA) gene, U1, to express ribozymes against a peptide hormone. U1 is ubiquitously expressed under the control of a strong RNA polymerase II promoter, giving rise to a 5'-m₃G-capped transcript. The 3' end of the U1 molecule is protected from exonuclease degradation by secondary structure and a conserved 3' sequence, the 〈〈 3' end box 〉〉. The U1-ribozyme vector system of De Young et al involved the expression, from the U1 promoter, of a sequence comprising nine, 5'-bases of U1 initiation sequence, a hammerhead ribozyme against atrial natriuretic factor and, 3' to the ribozyme, 61 bases corresponding to the conserved U1 3'end box. The addition of these U1 sequences had variable effects on catalytic activity. Their effect on stability was not directly measured.

Other workers have taken advantage of polymerase III-based transcription systems, particularly tRNA, to enhance stability of non-translated foreign RNA. For example, ribozymes can be embedded at various sites within the tRNA molecule, or related sequences, thus conferring on the ribozyme some of the natural stability of the tRNA. The insertion sites must be chosen to minimise possible interactions between the ribozyme and the tRNA since such interactions would be likely to have adverse effects on ribozyme function (see for example, Cotten and Birnsteil, 1989, Bouvet et al., 1994, Baier et al., 1994 and Perriman et al., 1995)

The present invention provides an alternative to these known systems of improving RNA stability. In addition, the present invention also has the advantage of enabling the production of multiple stabilised RNA molecules from a single promoter, in a splicing-independent manner. Furthermore, the present invention also provides novel regulatory sequences for producing stabilised RNA molecules, or other transcripts, particularly in plant cells.

The present invention is based on the discovery, by the inventors, of a previously unknown natural mechanism for producing stable, small nucleolar RNAs (snoRNAs), particularly in plants. This mechanism involves expression of snoRNAs as part of a larger precursor transcript, followed by exo- and endonucleolytic degradation of the precursor transcript, eliminating all non-protected sequences and giving rise to stable, degradation-resistant snoRNAs. The inventors have shown that this system can be adapted to the production of stable, heterologous RNA, particularly ribozymes and antisense RNA.

Small nucleolar RNAs (snoRNAs) are involved in many aspects of rRNA processing and maturation. All snoRNAs identified to date fall into one of three classes : Box C/ Box D snoRNAs (associated with fibrillarin) - see Maxwell and Fournier et al., 1995 ; Kiss-Laszlo et al., 1996 ; Nicoloso et al., 1996), H/ACA snoRNAs (see Balakin et al., 1996 ; Ganot et al ;, 1997), and MRP snoRNAs. This latter class contains MRP RNA as its sole example. Some examples of Box C/Box D snoRNAs are U8, U22, and U14 for vertebrates, and U3, U14, U18 and U24 for yeast. Examples of H/ACA type snoRNAs are U17/E1, E2, E3, U19, U23 for vertebrates, and snR3, snR5, snR6 for yeast.

In animals and yeast, a large number of snoRNAs are encoded within introns of protein coding genes. These introns contain only single snoRNA genes and their processing is largely splicing-dependent and involves exonucleolytic release of the snoRNA from the excised intron. It is thought that the exonucleolytic cleavage of the excised intron is blocked by the binding of protein factors to specific sites within the snoRNA, and by stems or stem-loops at the 5' and 3' ends (Tycowski et al., 1993 ; Caffarelli et al., 1996 ;Watkins et al., 1997)

The present inventors have established that, in contrast to the animal systems, snoRNA genes in plants are not necessarily in introns and many are found in small clusters, expressed polycistronically from novel promoters. Stable snoRNAs are produced by endonucleolytic cleavage of the polycistronic transcript in the intergenic regions, followed by exonucleolytic trimming-back of the resulting 5'-phosphate and 3'-hydroxy ends, up to the snoRNA. Thus, snoRNA production in plants differs in two significant respects from snoRNA production in animals : firstly the expression of the snoRNA is often polycistronic, and secondly the processing is splicing-independent.

The processing of the polycistronic transcript in plants to give a plurality of individual, mature snoRNAs is particularly surprising in view of experiments reported by Kiss and Filipowicz (1995) and Cavaillé and Bachellerie (1996) in which artificially constructed tandem U17 or U20 genes, in animal cells, gave rise to a trancript containing the tandem coding sequences, correctly processed at the 5' and 3' ends, but not effectively processed in the intergenic region. The cellular machinery for processing of such polycistronic transcripts has therefore been shown, by the present inventors, to be different between animals and plants, animal cells being apparently unable to completely process polycistronic transcripts of this type.

Furthermore, when expressed in intronic and non-intronic transcripts in animal cells, the levels of U17 and U20 were extremely low in the non-intronic form compared to the intronic form, pointing to largely splicing-dependent processing. The inventors have shown that analogous plant snoRNA constructs expressed in plant cells give accumulation of snoRNAs to similar levels from both intronic and non-intronic forms, pointing to splicing-independent processing.

The present inventors, after having identified this novel expression and processing mechanism in plants, have characterised the sequence and structure requirements for production of fully processed, stabilised snoRNA. In the framework of the present invention, it has been found that the sequence and structure requirements are such as to enable use of this system in the production of mature, stabilised heterologous RNA. Specifically, :
- in plants, the endonucleolytic cleavage of the intergenic regions of the polycistronic transcript does not appear to involve any specific signals or sequences. The subsequent digestion of the RNA by 5' and 3' exonucleases is thought to be blocked by the presence of proteins binding to protein binding sites within the snoRNA, thus preventing over-digestion of the snoRNA. In some cases, secondary structure associated with the snoRNA may also help to prevent over-digestion by exonucleases. The resulting processed snoRNA is stable with respect to nucleolytic degradation, and accumulation of snoRNA is observed ;
- as far as the protein binding sites are concerned, it has surprisingly been demonstrated that for natural snoRNA's processing can be achieved by protein binding sites having very different sequences and structures. Indeed the clusters of snoRNA genes found in plants comprise, within a given cluster, genes belonging to the two main categories of snoRNAs, Box C/D-type snoRNAs and H/ACA-type snoRNAs. These two families of snoRNAs have different protein binding sites and structures. Thus, it appears sufficient that the snoRNA possess one or more protein binding site(s) capable of binding a protein or proteins occurring in the cell under consideration ;
- in addition to protein binding sites, some plant snoRNAs have also secondary structure, particularly a base-paired stem, to help prevent overdigestion by exonucleases. For these snoRNA's, it has been established that the sequence of the regions involved in the stem structures is not naturally conserved and can be varied without affecting protective activity ;
- sequences within the snoRNA genes, other than the protein binding sites, do not appear to be critical for achieving the processing and stabilising effects. Internal deletions within the snoRNA therefore do not affect the production of mature, correctly processed and stabilised RNA.

Thus, the basic requirement for controlled, efficient endonucleolytic and exonucleolytic processing of the polycistronic transcript to give multiple molecules of stable snoRNA, is the presence, within the snoRNA, of at least one protein binding site, usually at least 2 protein binding sites, optionally together with sequences giving rise to associated secondary structure.

The stabilisation of molecules of heterologous non-translated RNA can thus be achieved simply by incorporating into, or adding to, the said heterologous molecule, the stabilisation sequences used in the plant snoRNAs, that is one or more protein binding sites, and / or sequences giving rise to appropriate secondary structure such as one or more stem/loop structures. These stabilising sequences are positioned, with respect to the non-translated RNA to be stabilised, so as not to hinder the function of the heterologous RNA. Endonucleolytic and exonucleolytic processing of the transcript is then carried out to the desired degree by the cell's natural processing machinery.

The RNA stabilising and processing system of the invention presents a number of advantages with respect to known stabilisation systems. In particular :
- the processing is splicing-independent, enabling the system to be used for producing stabilised RNA either from an intron or from non-intron sequences. The particular type of expression required can therefore be chosen to suit a given situation ;
- the production of stabilised RNA from intron sequences may not prevent the production of functional mRNA, i.e. the splicing-independent processing of intron-encoded non-translated RNA is not incompatible with the splicing of pre-mRNA to give rise to a functional protein-encoding mRNA. If desired, the production of stabilised non-translated RNA may therefore be co-ordinated with functional expression of a protein by inclusion of the RNA to be stabilised in an intron of a functional protein-encoding gene ;
- polycistronic transcripts from gene clusters can be processed efficiently by the cell. Multiple molecules of the same or different RNAs can therefore be transcribed, processed and stabilised from a single promoter, making expression simple and highly efficient ;
- the processing and stabilising effects may be obtained with a variety of different protein binding sites. Thus, the choice of an appropriate protein binding site can be made as a function of the cellular environment or compartment concerned, particularly as a function of the type of proteins present within any given cell or cell compartment ;
- endonucleolytic cleavage does not require the presence of specific sequences. Flanking sequences and intergenic spacers can therefore be chosen to have any desired sequence.

The present invention therefore relates to stabilised RNA molecules which can be produced in a yeast or plant cell from larger mono- or polycistronic precursor RNA transcripts. The RNA precursors are themselves usually produced in the cell by transcription of a purpose-built synthetic DNA molecule introduced into the cell by any known technique. Alternatively, the stabilised RNA molecules may be produced in vitro and delivered to the cell by known means, including delayed-release and slow-release systems.

The stabilised RNA molecules of the invention are composite molecules in so far as they comprise both the RNA whose stabilisation is required and stabilising sequences / structures which confer on the RNA the property of resistance to degradation by endo- and exonucleases.

In the context of the present invention, an RNA molecule is considered to be 〈〈 stabilised 〉〉 when it has a half-life sufficiently long to allow accumulation of the RNA species in the nucleus or nucleolus as detected by, for example Northern analysis, RNase-A/T1 protection mapping or Reverse Transcriptase Polymerase Chain Reaction (RT-PCR). Accumulation is achieved by virtue of the capacity of the stabilised RNA molecule to resist degradation by endonucleases and by 3'- and 5'- exonucleases. RNA molecules which are not stabilised do not accumulate and hence the extremely low levels can only be detected by the most sensitive techniques e.g. RT-PCR.

More specifically, the stabilised RNA molecules of the invention comprise the following two components :
i) a non-translated RNA sequence which is capable of functionally interacting with a cellular component, and
ii) stabilising sequences comprising at least one protein binding site and, optionally, sequences capable of giving rise to secondary structure associated with said protein binding site, said stabilizing sequences being heterologous with respect to the non-translated RNA sequence, and said stabilized RNA molecule being devoid of 5' cap sequences, and of 3' polyadenylated sequences.

The composite RNA molecule comprising the functional RNA (i) in association with the stabilizing sequences (ii) will be referred to hereafter as the 〈〈 stabilized RNA unit 〉〉.

The functional RNA component (i) of the stabilized RNA unit is the molecule whose stabilization is required. This molecule is capable of interacting in some way with a cellular component, for example capable of hybridising and/or cleaving RNA within the cell. The cellular component with which the stabilized RNA interacts can be any cellular component, for example RNA such as mRNA, rRNA, tRNA, or RNA of viral origin. Protein components may also be contemplated. Normally, the interaction of the stabilized RNA with the cellular component leads to the quantitative or qualitative modification of the function of the cellular component, for example, inactivates it, reduces its activity, or enhances its activity. This component of the stabilized RNA unit will be referred to hereinafter as the 〈〈 functional RNA 〉〉, the term 〈〈 functional 〉〉 in this context indicating the capacity of the molecule to interact with cellular components. The functional RNA is not normally a naturally occurring snoRNA. More particularly, it is not normally a full-length small nucleolar RNA of the Box C / Box D or H/ACA type.

Examples of such functional RNAs are ribozymes, antisense molecules, decoy RNA's (aptamers) and co-suppressional sense RNAs. Ribozymes are particularly preferred, such as those of the hammer-head type described in European patent EP-B-321201. Other types of ribozymes which may be stabilized by the process of the invention are hairpin ribozymes, or ribozymes of the Group I intron type. Particularly, preferred examples are ribozymes or antisense molecules which inactivate viral or endogenous mRNA within the cell. Preferred ribozyme and antisense targets are genes whose inactivation has therapeutic effects such as oncogenes, (myc, ras, myb), genes of Human Immunodeficiency Virus (HIV), Human Papilloma Virus (HPV), Hepatitis B virus (HBV), Hepatitis C virus (HCV), genes involved in diseases of skin and in restenosis. In plants, preferred ribozyme or antisense targets are Potyvirus genes, for example WMVZ, ZYMV, CMV, tobamovirus, potexvirus, tombus virus and gemini virus (for example TYLCV) genes.

The stabilized RNA unit also comprises stabilising sequences comprising at least one protein binding site and, optionally, sequences capable of giving rise to secondary structure normally associated with said protein binding site. The stabilising sequences may be any sequence, naturally occurring or synthetic which, by protein-RNA binding interactions confer on the functional RNA the capacity to resist degradation by endonucleases and by 3'- and 5'-exonucleases, so that accumulation of the functional RNA is achieved. This in turn gives the functional RNA the opportunity to function efficiently in the cell.

The stabilizing sequences typically comprise protein binding sites of naturally occurring non-translated RNA, such as small nuclear RNA's, including both spliceosomal and small nucleolar RNAs, or of translated RNA. Functional equivalents of these naturally occurring sequences may also be employed, wherein 〈〈 functional equivalent 〉〉 means any sequence, which, through RNA-protein binding interactions, is capable of preventing or reducing digestion, by both endonucleases and by 5'- and 3'-exonucleases, of an RNA in a cell so that accumulation of the RNA is achieved.

The stabilizing sequences of the present invention are heterologous with respect to the functional RNA. By 〈〈 heterologous 〉〉 is meant that the stabilizing sequences do not naturally occur adjacent to the functional RNA sequence, for example in the genome of a source organism. In other words, the chimeric molecule comprising the stabilizing sequences and functional RNA is not a naturally occurring molecule.

As examples of stabilizing sequences, mention can be made of the stabilizing sequences of small nucleolar RNAs (snoRNAs). SnoRNA's of the two major classes - fibrillarin-binding snoRNA's (or 〈〈 Box C/Box D 〉〉 snoRNAs) and H/ACA snoRNAs are preferred sources of stabilizing sequences according to the invention.

In particular, the phylogenetically conserved protein binding sites of the fibrillarin-binding snoRNAs, i.e. the 〈〈 Box C 〉〉 and 〈〈 Box D 〉〉 sequences, are particularly preferred stabilizing sequences of the invention. They normally have the following sequences :
- **Box C :** XGANWP: wherein X = U, G or A, preferably U
N = any nucleotide preferably U
W = G, C or U, preferably G
P = any nucleotide preferably A.
- **Box D :** YXVA: wherein Y = C or U preferably C
X = U, G or A preferably U
V = G or U preferably G.

Examples of Box C sequences are sequences of the type UGANGS where S is A, U or C preferably A, for example UGAUGA and UGAUGU. Examples of Box D sequences are sequences of the CUGA type, for example UUCUGA and GUCUGA. Any of the above nucleotides may be substituted in sugar, phosphate or base.

The protein binding sites of snoRNA's of the H/ACA class are also preferred stabilizing sequences of the present invention. These are sequences known as the 〈〈 H 〉〉 Box, whose sequence is ANANNA, wherein N is any nucleotide, for example AUACAA, and the 〈〈 ACA 〉〉 Box having the sequence ACA. The H Box naturally occurs in the hinge region of the hairpin-hinge-hairpin structure of the H/ACA type snoRNA, and the ACA sequence is positioned 3' nucleotides from the 3' end of the molecule. These sequences are thought to be involved in the binding of the protein GAR1.

The capacity of the stabilizing sequences to stabilize the functional RNA molecule can be tested by performing the following stabilization test :
- transfecting a cell of the type in which expression is required, or a functional equivalent thereof, with a construct comprising the non-translated RNA to be stabilised and the stabilising sequences under test, optionally flanked by flanking sequences, all these sequences being under the transcriptional control of a functional promoter ;
- northern blotting the RNA transcribed in the cell and hybridising with a probe corresponding to the non-translated RNA to be stabilized. Alternatively, RNase A/T1 protection mapping or RT-PCR may be carried out. Details of these techniques are given below in the Examples ;
- verification by primer extension or RNase-A/T1 protection mapping that the 5' and 3' ends correspond to those of the non-translated RNA to be stabilized together with the stabilizing sequence, any flanking sequences originally present in the construct having been endo- and exo-nucleolytically degraded.

As further examples of protein binding sequences which are useful in the invention, mention can be made of spliceosomal snRNA (small nuclear RNA) binding sites e.g. U1A / U2B'' RNA protein binding sites (Simpson et al, 1995), and the histone-RNA hairpin binding protein site.

The protein binding site(s) chosen as stabiliser(s) should be capable of binding a protein endogenous to the cell in which expression is sought. The protein which binds to the domain is preferably a protein found in plant and/or yeast cells. For expression in animal cells, proteins endogenous to animal cells are used. Preferably, the protein is found in both plant and animal cells, and optionally also in yeast cells. Moreover, the protein binding site may be chosen to bind a protein present in a particular cellular compartment e.g. nucleus or nucleolus so that stabilization of the RNA will occur in that compartment.

The capacity of a particular protein to bind to the protein binding sites of the stabilized RNA can be detected by electrophoretic mobility shift analysis.

Normally, the stabilisation of the functional RNA will require two or more protein binding sites, which will be positioned with respect to the functional RNA so that the conformation adopted by the composite RNA molecule and its bound proteins will not hinder the function of the stabilized RNA. Generally speaking, the natural molecules used as source of binding sequences may be used as a model for the positioning of the protein bnding sequences with respect to the functional RNA. For example, for snoRNA stabilising sequences, one binding site may be positioned at each extremity of the functional RNA, as in Box C/Box D-type snoRNAs.

The number of sites required for stabilization and their relative positions can be tested using the stabilisation test indicated above. The conservation of function of the functional RNA can be tested using conventional tests for ribozyme or antisense activity, depending upon the precise nature of the functional RNA in question.

The stabilising sequences may also include sequences capable of giving rise to secondary structure, found normally associated with the protein binding site. These sequences are typically sequences which confer on the molecule a conformation which facilitates efficient protein binding, for example stem-loop structures in the vicinity of the protein binding site. Particularly preferred are protein binding site / secondary structure combinations wherein the secondary structure involves sequences of the 5' and 3' termini of the molecule. In this way, the secondary structure does not interfere with the functional RNA.

Sequences capable of forming at least one base-paired stem structure are particularly preferred, especially when the protein binding sites are derived from snoRNA. For example, the protein binding sites of many snoRNAs have a stem formed by base-pairing between inverted repeat sequences occurring at the 5' and 3' extremities of the molecule. The stem appears to prevent over-digestion of the snoRNA by 5' and 3' exonucleases. In accordance with the invention, the stem, if present, normally has at least two, and preferably at least three base pairs, and may extend up to about 30 base-pairs without affecting processing. A stem of between 3 and 15 base pairs is particularly preferred. Examples of suitable stem structures are inverted repeats from snoRNA's such as :
WTGCC wherein W = T or G, with its complement,
TATGGC and GCCATA,
TXYZTTGC
   - wherein: X = G or C, with its complement,
   Y = C or T,
   Z = A or T,
TTGGGGGATT and AATCCCCCAA,
GCCZTGTT wherein Z = A or T, with its complement.

To obtain a stabilising stem structure according to the invention, a specific helix sequence is not required, the only requirement is complementarity of the bases, provided by inverted repeat sequences within the non-translated RNA. The inverted repeats are normally positioned at the 5' and 3' ends of the molecule to be stabilised, and are positioned with respect to the protein binding sites in such a way as to not hinder protein binding or function of the RNA. Usually, the stem sequences are within one to three bases of the protein binding sites, and are external with respect to the said sites. Other positions may be found using the stabilisation test described above.

The stabilising sequences need not consist exclusively of the protein binding sites and stem structures described above, but may be included in larger structures, as long as the stabilizing influence is not adversely affected by the additional sequences. For example, it has been shown by the inventors that the functional RNA to be stabilized may be embedded in a full-length snoRNA sequence, e.g. U14. According to this variant, the stabilizing sequences are present along with the other sequences of the snoRNA.

A further feature of the stabilized RNA units of the invention is that they are devoid of 5' caps, for example 5' m7G or 5' m3G caps, and of 3'-polyadenylated sequences. This is due to the fact that the RNA molecules are produced in vivo by processing of a larger transcript. The 5' and 3' terminal features normally occurring on RNA transcripts as a result of the transcription of DNA by a particular RNA polymerase, or as a result of signals occurring within the DNA, are removed during processing by endo- and exonucleases.

The invention relates not only to the stabilized RNA molecules, but also to the process of producing such RNA molecules in a cell. This process involves introducing, into a plant or yeast cell, a DNA molecule (so-called 〈〈 precursor DNA 〉〉) coding for the stabilized RNA molecule(s) as described above.

More specifically, this aspect of the invention relates to a process for producing stabilized non-translated RNA molecules within a cell, comprising introducing into a plant or yeast cell a nucleic acid molecule comprising a DNA sequence which will be referred to hereinafter as a 〈〈 precursor DNA 〉〉, said precursor DNA containing a coding sequence, or a cluster of coding sequences, each coding sequence encoding :
- a non-translated RNA sequence to be stabilised, which is capable of functionally interacting with a cellular component, and
- stabilising sequences comprising at least one protein binding site and, optionally, sequences capable of giving rise to secondary structure associated with said protein binding site, said stabilizing sequences being heterologous with respect to the non-translated RNA sequence to be stabilized, the precursor DNA being under the transcriptional control of regulatory sequences functional in said cell,
   whereby a transcript is produced by transcription of the precursor DNA, and one or more stabilized RNA units comprising the non-translated RNA sequence in association with the stabilising sequences is, or are, then produced by processing of the transcript by the cell's endogenous processing machinery.

The production of functional stabilized RNA molecules according to the invention thus involves the introduction, into a eukaryotic cell, particularly a yeast or plant cell, of a nucleic acid molecule, containing the 〈〈 precursor DNA 〉〉, designed to give rise, on transcription, to a mono- or polycistronic RNA intermediate, which is then processed by the cell's natural processing machinery to single or multiple mature, stabilized RNA molecules as described above. Natural cellular processing of the RNA intermediate removes from the transcript, and degrades, all sequences, with the exception of the RNA stabilized by the stabilizing sequences.

The sequences within the precursor DNA form a single transcriptional unit, i.e. all the sequences within the DNA precursor (coding sequences, stabilizing sequences and possibly flanking sequences) are under the transcriptional control of the same promoter, and transcription of the precursor DNA from this promoter gives rise to a single RNA transcript. Transcription is always initiated upstream of said coding sequence or cluster, and terminated downstream of said coding sequence or cluster. The promoter directing transcription of the precursor DNA is normally part of the nucleic acid molecule containing the precursor DNA.

In the context of the invention, the terms 〈〈 coding sequence 〉〉 signify a DNA sequence, which codes for a stabilized functional non-translated RNA molecule. The stabilised non-translated functional RNA is the transcript of the coding sequence. The 〈〈 coding sequence 〉〉 is in fact the assembly of two types of coding sequence - namely, the coding sequence encoding the functional RNA capable of interacting with a cellular component and that encoding the stabilizing sequences. The 〈〈 coding sequence 〉〉 is devoid of protein-encoding sequences, although such protein encoding sequences may be present elsewhere in the precursor DNA or in the nucleic acid construct containing the precursor DNA.

The precursor DNA is either monocistronic or polycistronic, i.e. it comprises either a single coding sequence, or a plurality of coding sequences respectively, encoding the stabilized RNA unit(s) as defined above.

In cases where the precursor DNA contains a single coding sequence, the transcript is monocistronic and the processing of the monocistronic transcript gives rise to a single stabilized RNA unit. In the context of the invention, the term 〈〈 cistron 〉〉 indicates a DNA sequence which codes for a functional RNA, and may be used synonymously with the terms 〈〈 coding sequence 〉〉 as defined above.

According to a preferred embodiment of the invention, the precursor contains a cluster of coding sequences. The RNA transcript is thus polycistronic, and processing gives rise to multiple stabilized RNA units. This embodiment of the invention is particularly advantageous since it enables the production of multiple, stabilized functional RNAs, for example ribozymes, from a single promoter.

A 〈〈 cluster 〉〉 of coding sequences signifies, in the context of the invention, a plurality, i.e. at least two, for example two to ten, or particularly two to five, coding sequences as defined above, separated from each other by spacer sequences. The coding sequences may be the same or different, either in so far as the functional RNA or the stabilising sequences are concerned. For example, the different coding sequences may code for stabilised ribozymes having the same or different target sequences, or may code for a mixture of ribozymes and antisense sequences.

According to this 〈〈 polycistronic 〉〉 embodiment of the invention, the spacer sequences between adjacent coding sequences of any given cluster are devoid of exon sequences and associated functional intron splice sites, either normal GT...AG intron splice sites, or AT...AC type sites. Generally, the spacers do not code for protein or for functional RNA molecules or parts thereof. The spacers are also preferably devoid of known protein binding sites.

The spacers separating adjacent coding sequences in the polycistronic precursor DNA may be any nucleotide sequence. They normally have a length of between 5 to 200 nucleotides, for example 10 to 150, or more particularly 15 to 100 nucleotides. The spacers have no particular function in the polycistronic transcript. They are degraded in the cell after transcription, by endo- and exonucleases. They thus do not accumulate in the cell and are not readily detectable by Northern analysis, but may be detected by the more sensitive techniques of RT-PCR and possibly by RNase-A/T1 protection mapping. The spacers are normally non-protein encoding sequences. Even if protein- coding sequences were chosen as spacers, they would not give rise to a protein since they are degraded rapidly in the cell. Typical examples of suitable spacers of the invention are the intergenic regions of polycistronic plant or yeast snoRNAs, particularly those of plant U14s.

The precursor DNA may further comprise flanking sequences on one or both sides of the coding sequence or on one or both sides of the cluster of coding sequences. The terms 〈〈 flanking sequences 〉〉 signify, in the context of the invention, sequences occurring outside of the sequences encoding the stabilized RNA unit or cluster of units. The stabilizing influence of the snoRNA-derived stabilising sequences does not extend to the flanking sequences. Thus, in cases where the stabilised RNA unit is composed of the functional RNA linked at both the 5' and 3' ends to a protein binding site, the flanking sequences are 5' and 3' to the termini of the stabilized RNA unit.

The flanking sequences, if present, have a length of at least one nucleotide, and preferably at least two or five nucleotides. They may be as long as several hundred nucleotides, for example from between 100 to 1000 nucleotides. The flanking sequences may be any desired sequence. They may be heterologous with respect to the sequence to be stabilised, or the stabilising sequences, i.e. in cases where the sequence to be stabilised or the stabilizing sequences are naturally occurring sequences, the flanking sequences of the precursor DNA need not be those sequences which immediately flank those sequences in the genome of the source organism.

The flanking sequences may or may not contain exon sequences and associated functional intron splice sites, since processing is splicing-independent. In cases where the flanking sequences contain exon sequences and intron splice sites, the coding sequence or cluster encoding the stabilized RNA are intron-encoded, and are transcribed as part of the pre-mRNA of an intron-containing gene. The intron containing the stabilised RNA coding sequences is either spliced out of the pre-mRNA, followed by endonucleolytic cleavage and exonucleolytic trimming of the excised intron to release the stabilized RNA, or stabilized RNA is directly processed from pre-mRNA.

According to this 〈〈 intron-encoded 〉〉 embodiment of the invention, the stabilised RNA may be produced simultaneously with a protein. The flanking sequences, in this case, comprise a protein-encoding gene, and transcription is directed by the promoter of this gene. Any type of protein-encoding gene may be chosen to carry the stabilized RNA sequences. Usually, eukaryotic genes which naturally contain introns are used, but it is also possible to use prokaryotic carrier-genes if an intron is artificially inserted. As examples of protein-encoding carrier genes, mention can be made of selectable marker genes, such as antibiotic resistance genes, genes encoding resistance to viruses, such as coat protein genes etc., genes encoding herbicide and insect resistance, genes encoding endogenous plant or yeast proteins, such as seed storage proteins, genes encoding mammalian proteins such as hormones, blood proteins, genes required for colour production, for example anthacyanins, or GFP, or LUX, etc..

The 〈〈 intron-encoded 〉〉 variant of the invention described above can be used with both single coding sequences and clusters of coding sequences. Clusters are particularly preferred.

According to another variant of the invention, the flanking sequences are devoid of exon sequences and associated functional intron splice-sites. The coding sequences are thus non-intron encoded. In this case, the flanking sequences are degraded when the transcript of the precursor DNA is processed by endo- and exonucleases to release the stabilized RNA. Again, according to this variant, the flanking sequences can be any sequence of choice, for example, sequences which naturally flank snoRNA genes, vector sequences, sequences which facilitate cloning etc.

The 〈〈 non-intron encoded 〉〉 variant of the invention is particularly advantageous since its application is simple. For example, the construction of the precursor DNA is facilitated by the absence of exons and introns. The construct is therefore relatively small and easy to manipulate. Again, both single and multiple RNA coding sequences can be used with this embodiment of the invention.

A particularly preferred variant of the invention is the monocistronic RNA coding sequences in a non-intron-encoded construct. For the polycistronic coding sequences, both the intron-encoded and non-intron-encoded constructs are particularly advantageous.

Transcription of the precursor DNA is controlled by regulatory sequences which are functional in the cell under consideration. In particular, the precursor DNA is placed under the control of a functional promoter at the 5' end, and transcription termination signals are placed at the 3' end. The promoter may be any promoter, including RNA-polymerase II or III promoters. In plant cells, preferred examples are viral promoters such as the CaMV 35S promoter ; bacterial promoters such as the NOS promoter ; and plant promoters, for example tissue- or developmentally specific promoters such as the napin promoter. In yeast cells, the GAL1 promoter is a suitable example.

If the precursor DNA is within an intron of a protein-encoding gene, transcription of the precursor DNA is driven by the natural promoter directing transcription of the protein-encoding gene or a different promoter as above. Thus, the transcription of the precursor DNA can be co-ordinated with that of a protein-encoding gene whose promoter has desirable strength, or tissue or developmental specificity.

Alternatively, if the precursor DNA is not intron-encoded, the promoter will drive transcription of non-translated RNA. Again, any promoter functional in the cell under consideration may be used.

According to a particularly preferred aspect of the invention, the promoter employed for the transcription of the precursor DNA is an snoRNA promoter, especially from a plant polycistronic snoRNA gene cluster. Indeed, it has been demonstrated by the present inventors that plant snoRNA gene clusters are under the transcriptional control of novel promoters not previously isolated in plants.

Thus, the invention also relates to novel promoters, contained within the upstream region of a higher plant snoRNA gene cluster. The terms 〈〈 upstream region 〉〉 signify in the context of the invention, the non-translated region extending from the nucleotides immediately 5' of the snoRNA coding sequence, or immediately 5' of the 5'-most snoRNA coding sequence of a cluster, to approximately 1000 nucleotides upstream of the coding sequence. The upstream region is free of protein-encoding regions, intron splice sites and other snoRNA coding sequences.

The novel promoters contain a putative TATA box, normally around 200 bases upstream of the first snoRNA coding sequence, and are GC-rich, for example around 60% (62% in maize). Promoter activity is normally associated with a region of around 300-350 nt, for example 340 nt, in length lying within approximately 800 bp of the first snoRNA coding sequence.

The promoters of the invention may be identified by first identifying snoRNA genes in the genome of a cell, and then testing the upstream region of the snoRNA gene in an experimental system for demonstrating promoter activity. For example, the following techniques may be used to detect novel snoRNA promoters :
i) identification of snoRNA coding sequences, particularly clusters of such sequences, for example on the basis of the presence of Box C/Box D sequences approximately 100 - 200 bases apart, complementarity to rRNA, and presence of inverted repeats. Identification of Box H/ACA type snoRNAs may be made on the basis of the presence of the conserved sequences ANANNA and ACA, and 5' and 3' stem-loop structures. This identification may be carried out by scanning of sequences listed in computer-bases, or by PCR or probe-hybridisation, on the basis of the above conserved sequences followed by :
ii) cloning of a fragment of DNA corresponding to the region upstream of the 5'-most snoRNA coding sequence in the cluster, and testing of the capacity of the fragment to drive transcription of, for example a reporter gene. This capacity can be tested using conventional analyses.
iii) additionally, upstream regions of a number of snoRNA gene clusters from the same or different species may be compared. Regions showing at least 75%, and possibly at least 85% identity with each other are indicative of promoter regions.

The promoters of the present invention are particularly advantageous because they naturally have the function of directing transcription of polycistronic RNA, which occurs only infrequently in eukaryotes and usually indicates high promoter strength, wherein 〈〈 strength 〉〉 signifies the number of transcripts initiated per unit time. Furthermore, it has been demonstrated by the inventors that the promoters are functional in both monocotyledonous and dicotyledonous species, regardless of the source of the promoter.

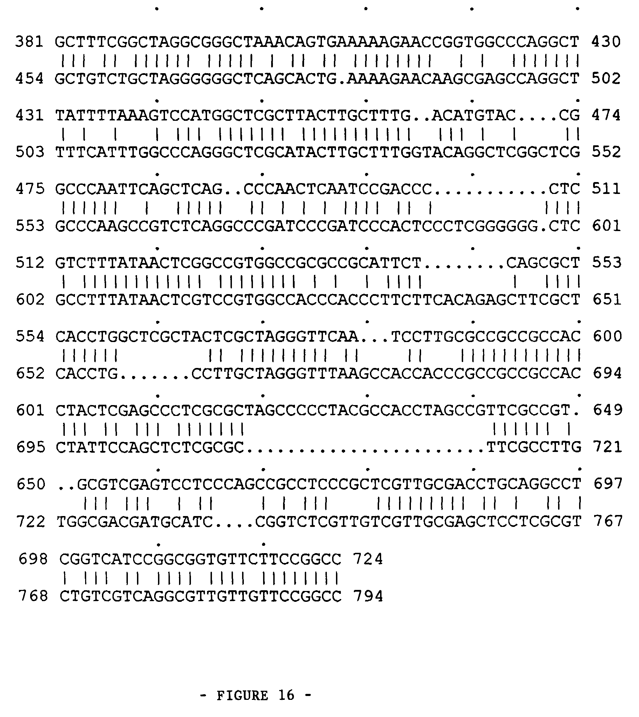

In particular, the DNA molecules having promoter activity according to the invention comprise
- the conserved sequence illustrated in figure 16, or
- a fragment of said conserved sequence, capable of directing transcription of polycistronic RNA, or
- a homologous sequence presenting at least 70%, preferably 75%, and most preferably 85% identity with the conserved sequence or with the fragment, said homologous sequence being capable of directing transcription of polycistronic RNA.

The terms 〈〈 conserved sequence 〉〉 in relation to the sequences of Figure 16, signify a sequence which is at least 70% identical to one of the two sequences shown in Figure 16, particularly where the identical bases correspond to the conserved ones, indicated by a vertical line in Figure 16.

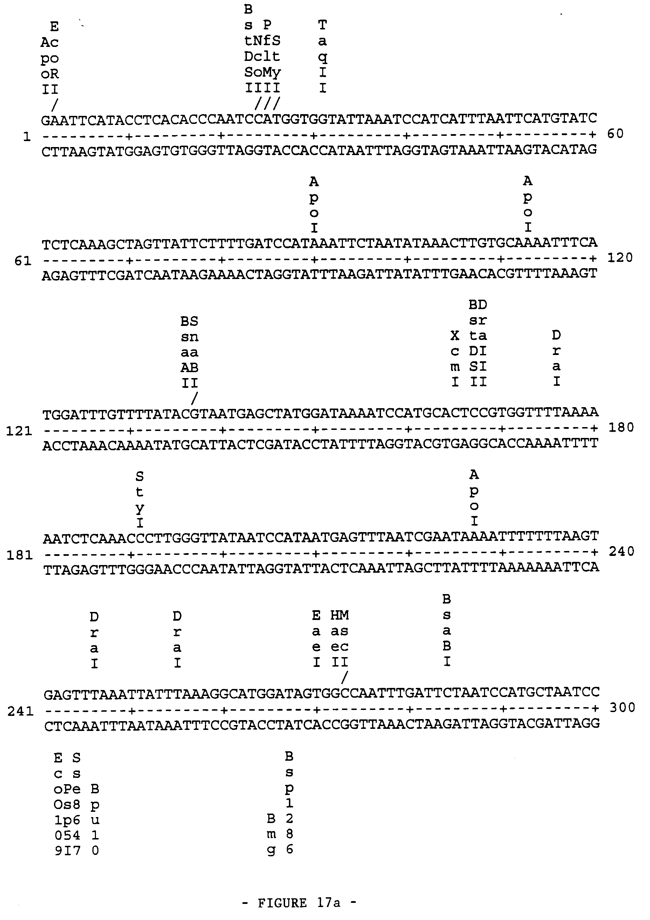

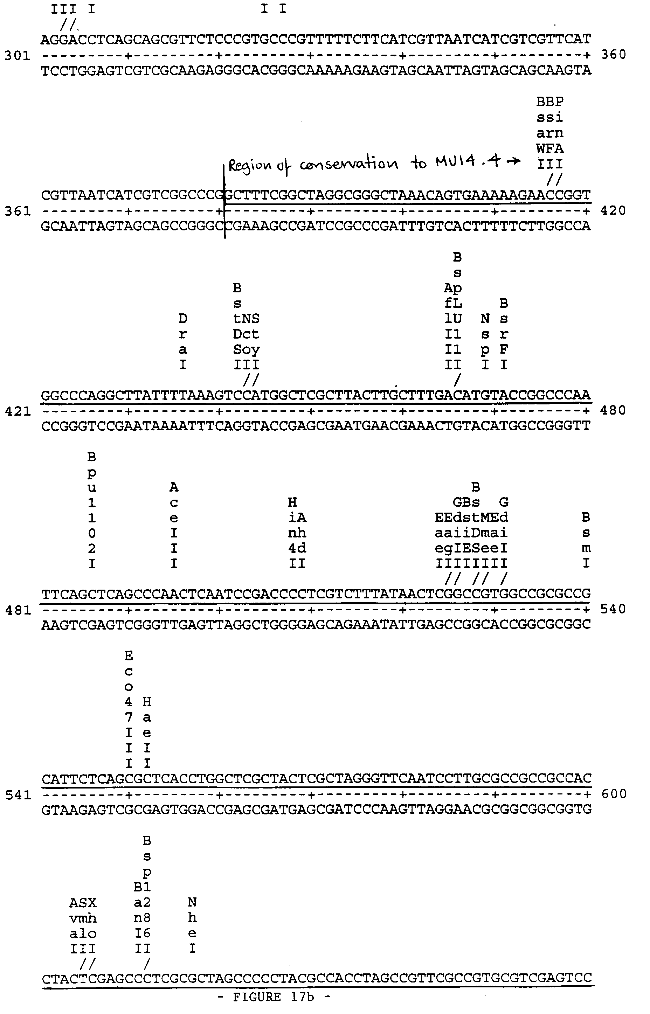

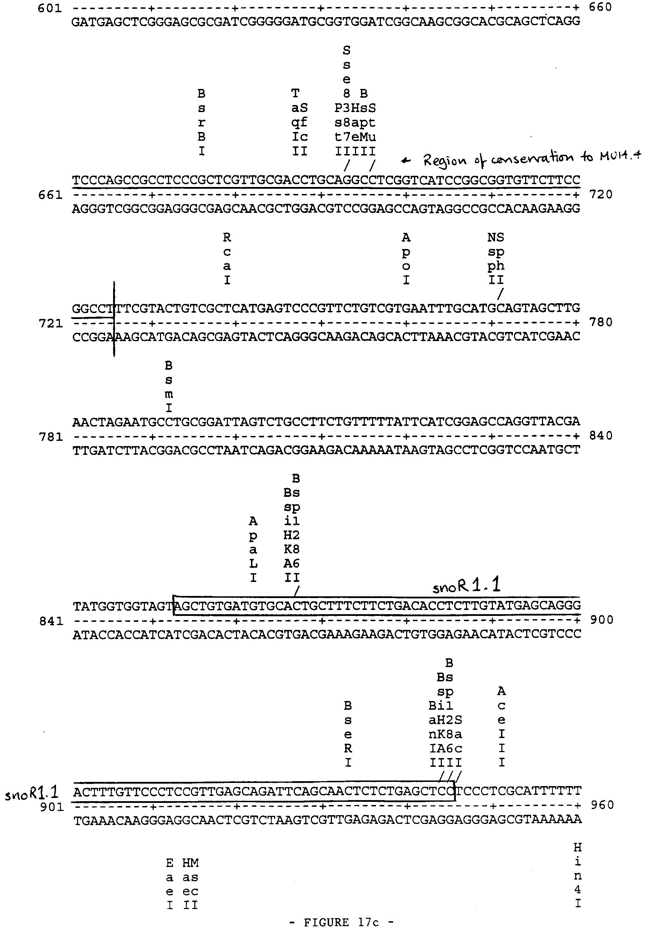

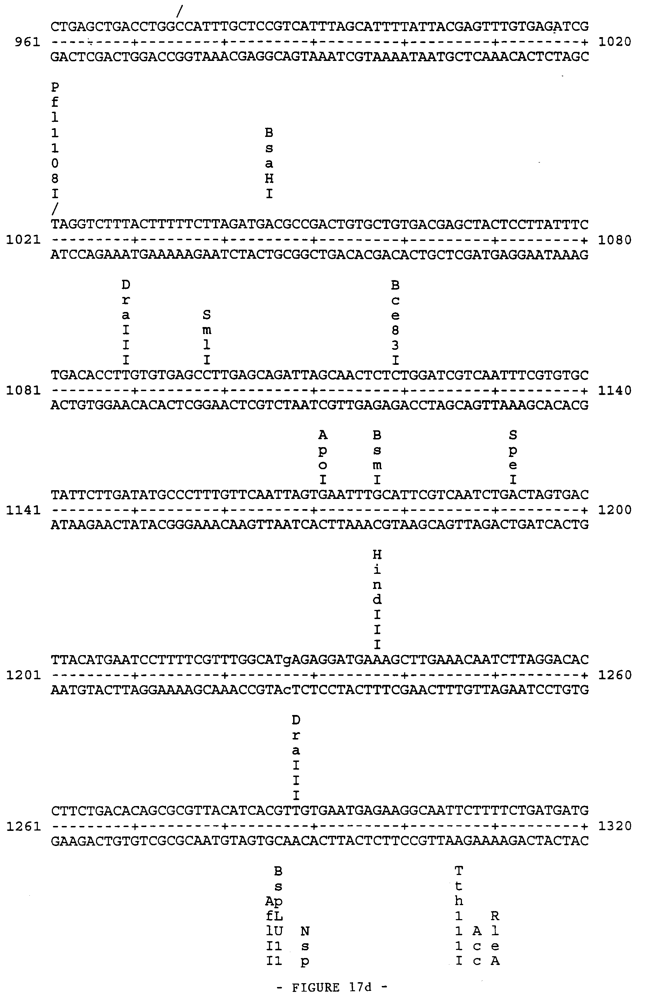

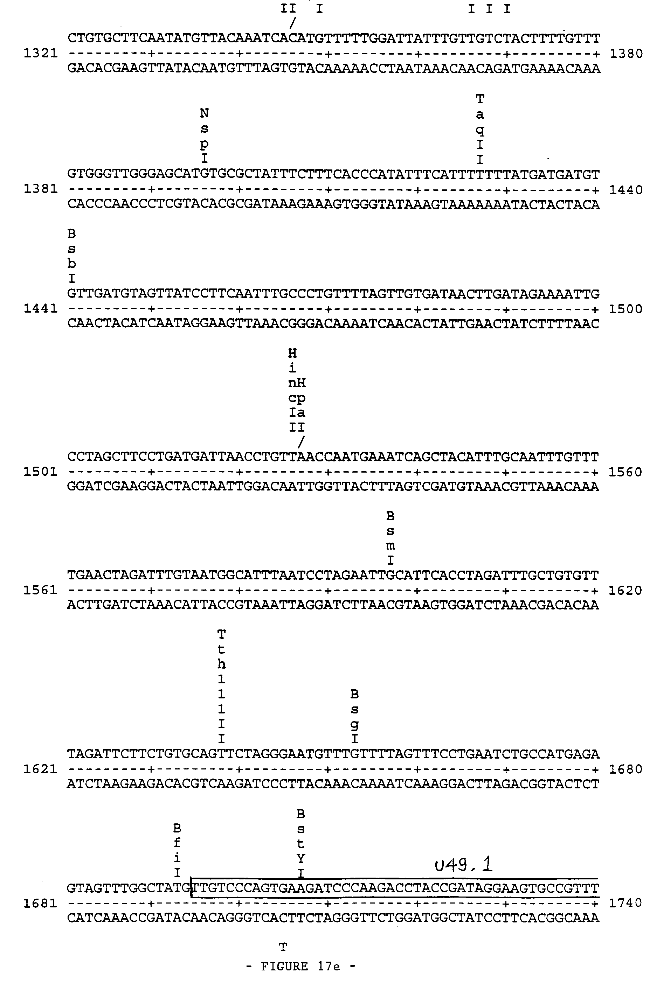

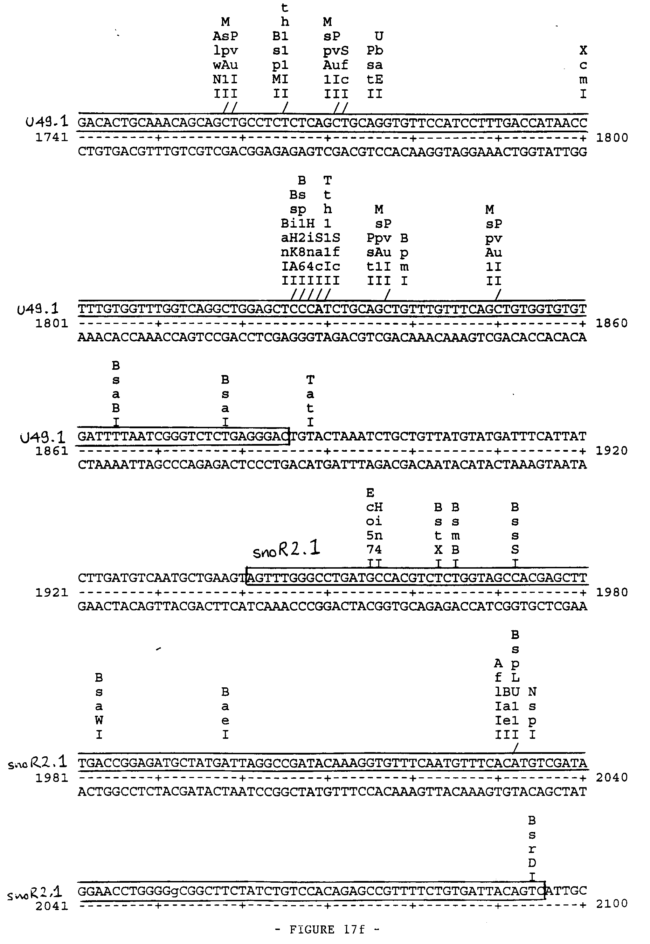

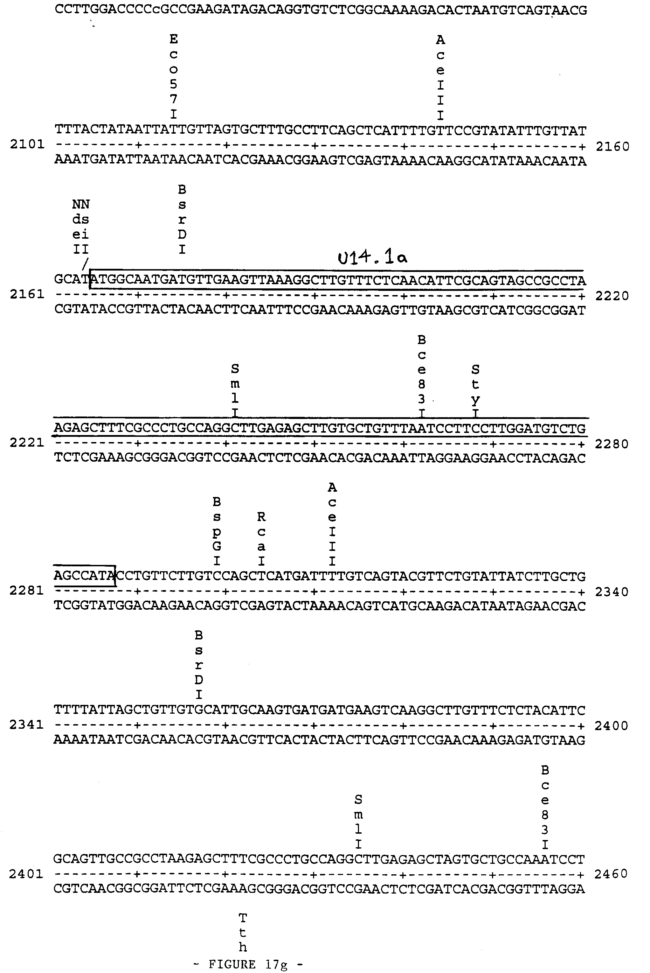

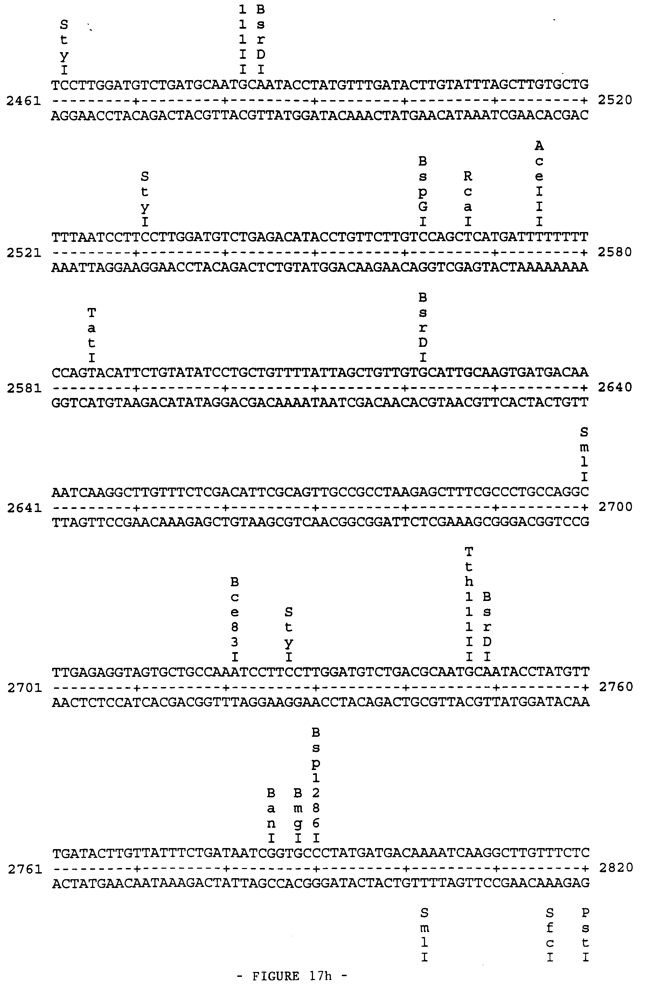

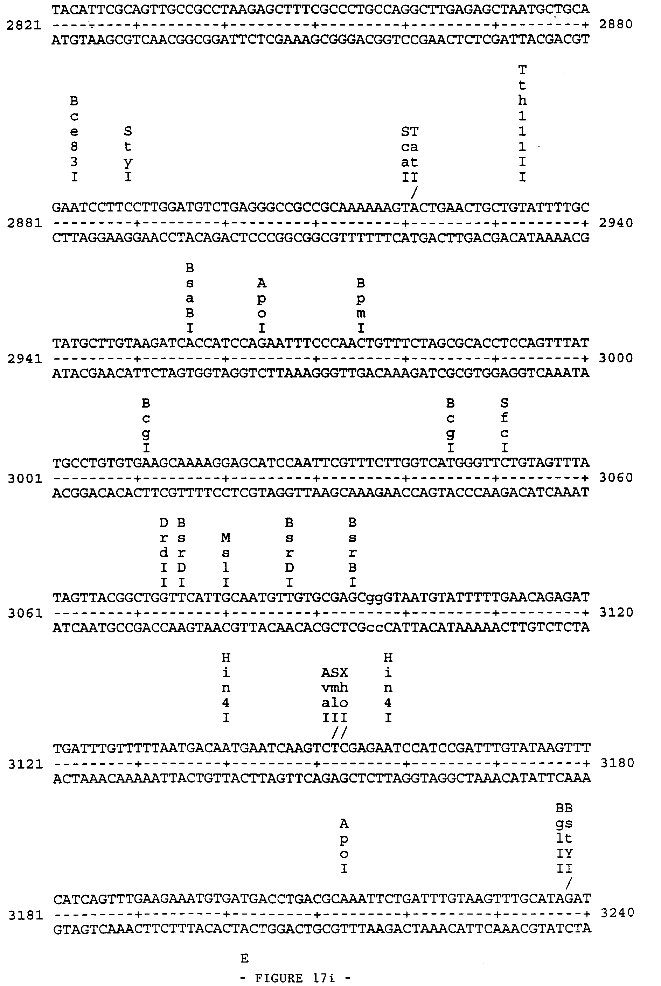

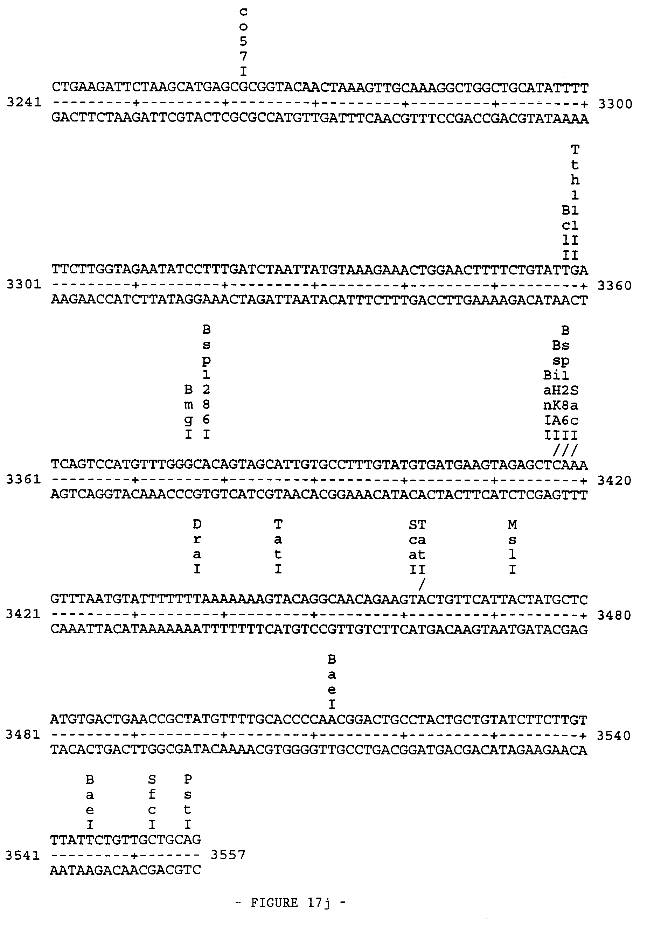

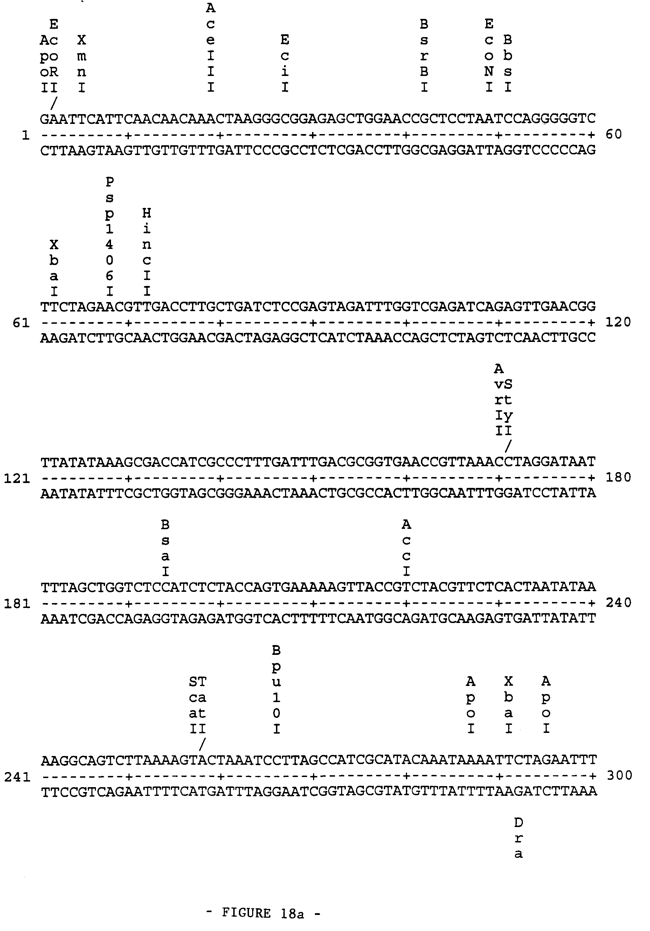

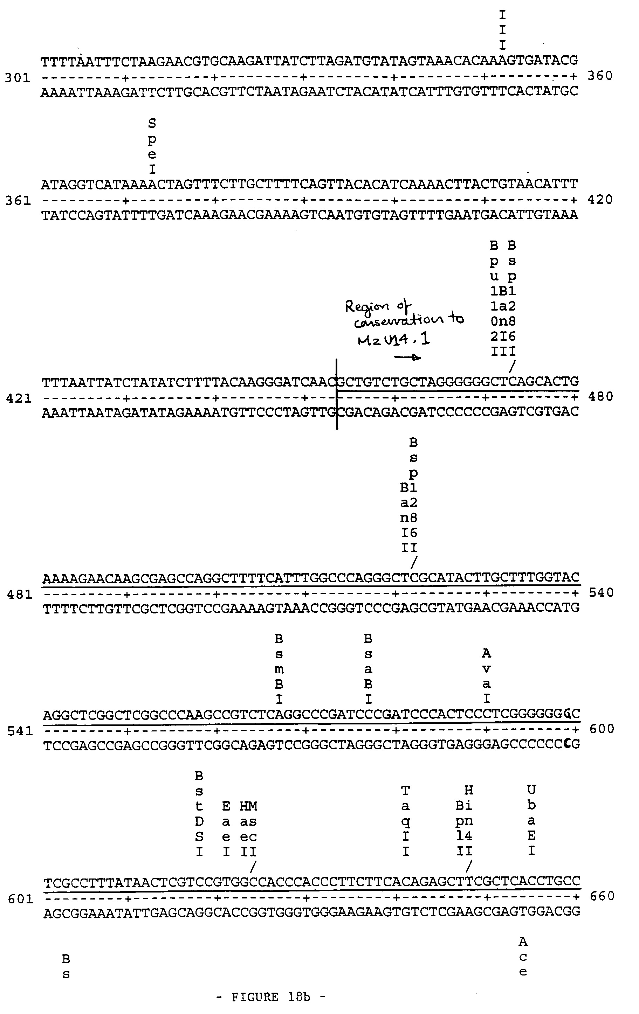

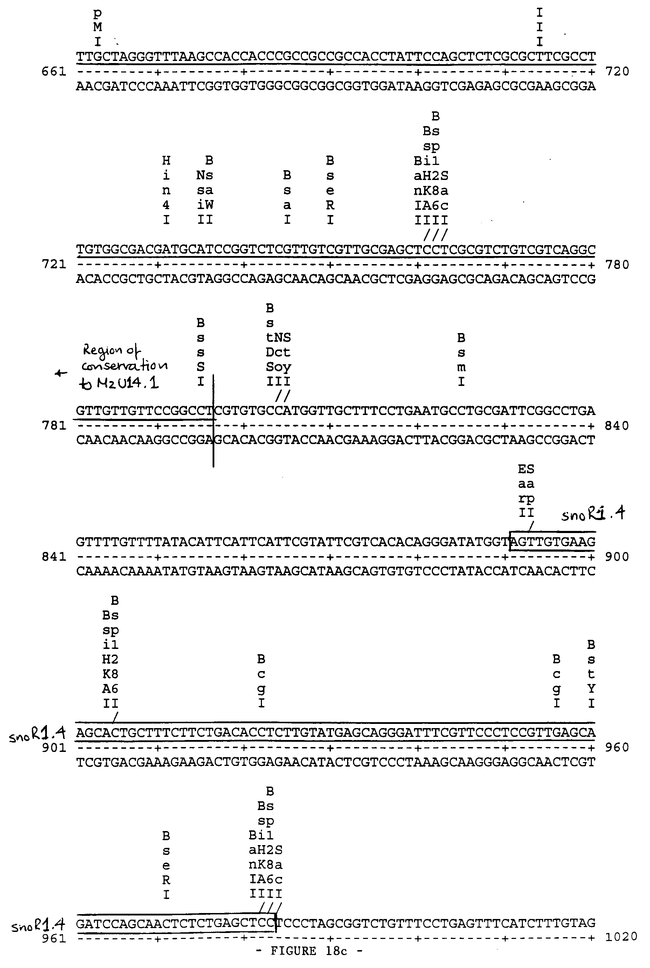

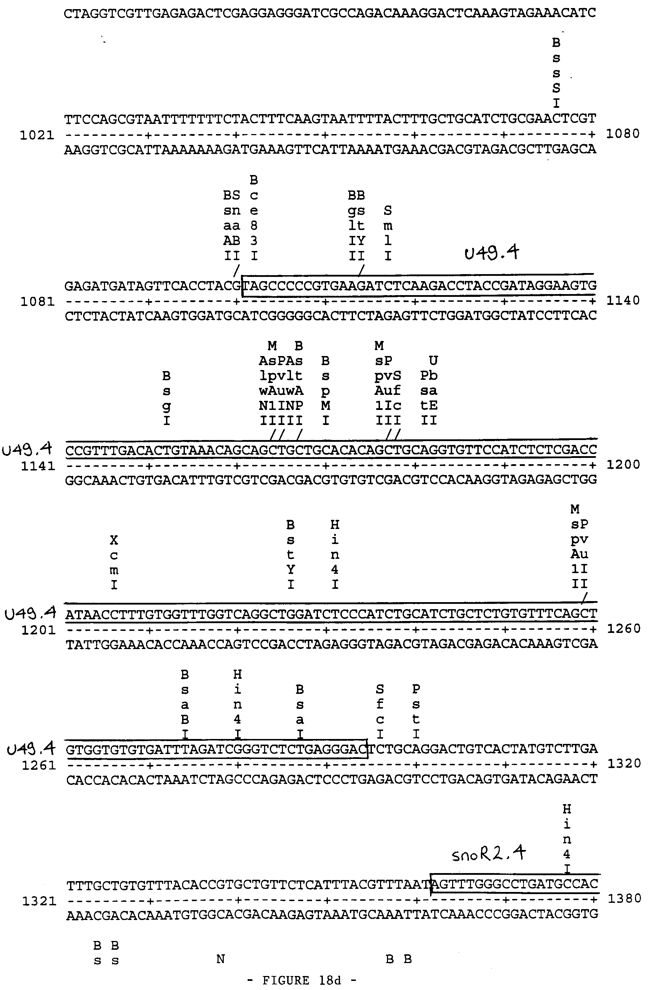

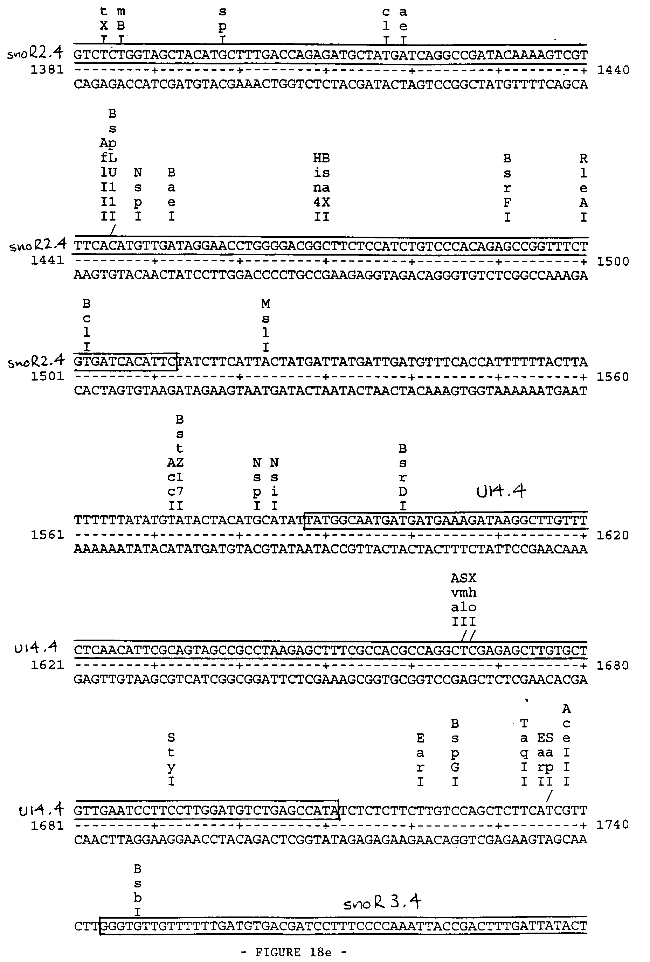

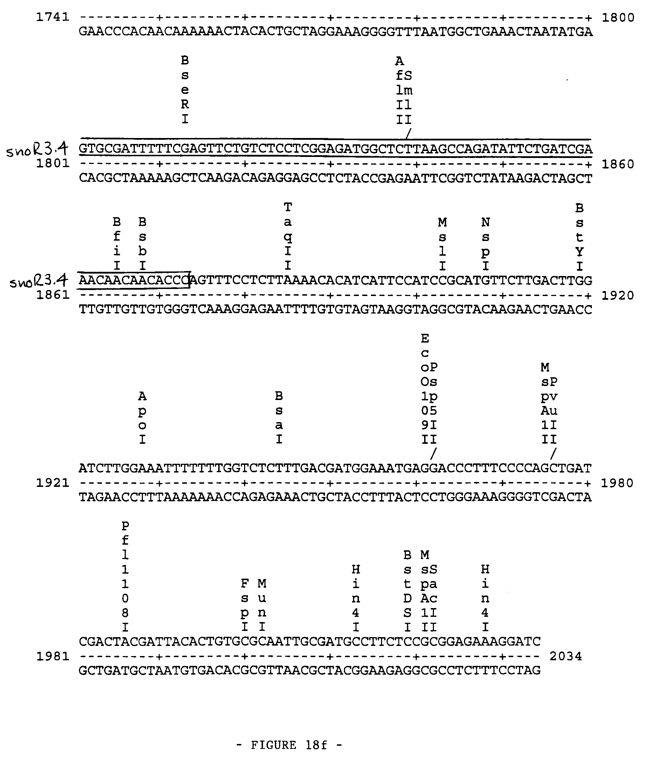

Preferred examples of the promoter regions of the invention are the maize U14.1 and U14.4 cluster promoters corresponding to nucleotides 1-850 and 1-880 of Figures 17 and 18 respectively.

The promoter regions of the invention may be used in chimeric genes for the expression of heterologous coding sequences in eukaryotic, particularly plant, cells. The promoter region is operably linked to a coding sequence, either translated or non-translated which is other than a full, uninterupted snoRNA coding sequence, and which is normally stripped of its endogenous promoter. As examples of non-translated coding sequences, mention may be made of ribozymes, antisense sequences, decoy RNAs (aptamers) and sense RNA (co-suppression). As examples of translated coding sequences, mention may be made of coding sequences of selectable marker genes, virus resistance genes, herbicide resistance genes, insecticidal genes, genes involved in male sterility, ripening, genes involved in metabolic pathways, oil production, protein quality and flower colour.

The invention further relates to transformation vectors containing any of the sequences of the invention, suitable for stably transforming eukaryotic cells, and to the transformed cells thus obtained. Plant and yeast cells are particularly preferred although animal cells, especially mammalian cells may also be included in the invention. All necessary signals for transcription initiation and termination etc..., must be present. Any of the conventional transformation techniques are applied.

Transgenic plants, obtained by regeneration of the transformed plant cells, and the progeny of such plants also fall within the scope of the invention, particularly plants capable of producing the stabilized antisense and ribozyme sequences discussed above, as well as plants or plant cell comprising the chimeric gene of the invention. As examples of plants capable of being transformed by the sequence of the invention, mention can be made of monocotyledons and dicotyledons such as the cereals e.g. wheat, maize, barley, rice, etc... ; Brassicae e.g. oilseed rape, cabbage, broccoli, etc... ; Cucurbitacea e.g. melon, cucumber and also sunflower, soya, etc...

The invention also relates to snoRNA sequences and intergenic sequences identified in the framework of the invention.

More particularly, this aspect of the invention relates to a nucleic acid molecule corresponding to, or derived from, a higher plant snoRNA gene cluster, wherein said molecule comprises or consists of a sequence having at least 20 consecutive bases of the sequence from nucleotide 1 to nucleotide 2165 illustrated in Figure 17, or of a sequence from nucleotide 1 to nucleotide 1587 in illustrated Figure 18, or of a sequence from nucleotide 1712 to 2034 illustrated in Figure 18, said molecule being devoid of U14 sequences characterized in this context by the presence of two sequences complementary to 18-S rRNA, called 18S-A and 18S-B (as described in International patent application WO 95/30748).

Preferably, the snoRNA sequences of the invention consist or comprise the following sequences, or fragments of the following sequences having at least 20 nucleotides, and preferably at least 25 nucleotides, for example 25 - 100 nucleotides :
nucleotides 1-850 of Figure 17
nucleotides 381-724 of Figure 17
nucleotides 853-945 of Figure 17
nucleotides 1695-1885 of Figure 17
nucleotides 1941-2095 of Figure 17
nucleotides 2165-2288 of Figure 17
nucleotides 2356-2479 of Figure 17
nucleotides 2621-2749 of Figure 17
nucleotides 2782-2911 of Figure 17
nucleotides 946-1694 of Figure 17
nucleotides 1886-1940 of Figure 17
nucleotides 2096-2164 of Figure 17
nucleotides 2288-2355 of Figure 17
nucleotides 2480-2620 of Figure 17
nucleotides 2750-2781 of Figure 17
nucleotides 2913-3557 of Figure 17
nucleotides 1-890 of Figure 18
nucleotides 891-983 of Figure 18
nucleotides 1101-1294 of Figure 18
nucleotides 1362-1512 of Figure 18
nucleotides 1588-1711 of Figure 18
nucleotides 1744-1873 of Figure 18
nucleotides 984-1100 of Figure 18
nucleotides 1295-1361 of Figure 18
nucleotides 1513-1587 of Figure 18
nucleotides 1712-1743 of Figure 18
nucleotides 1874-2034 of Figure 18

The invention also relates to sequences which are complementary to the above sequences, to sequences having at least 75 % and preferably at least 85 % identity with the above sequences, and to RNA equivalents of the above sequences. The sequences of the invention may be labelled for use as probes, or alternatively, may be used as primers in reactions, for identification of snoRNA sequences and intergenic regions. The sequences may be species-specific and therefore can be used to identify a species.

Different aspects of the invention are illustrated in the figures :

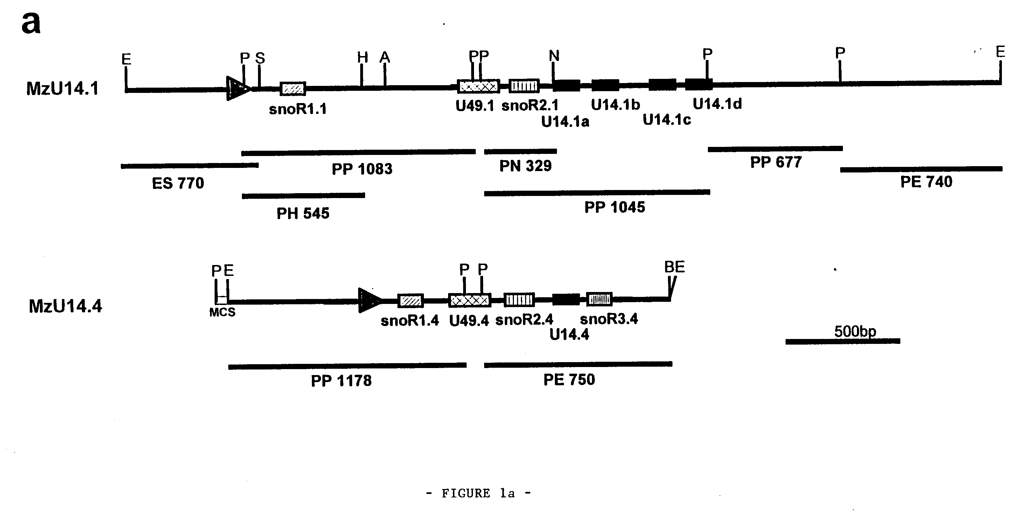

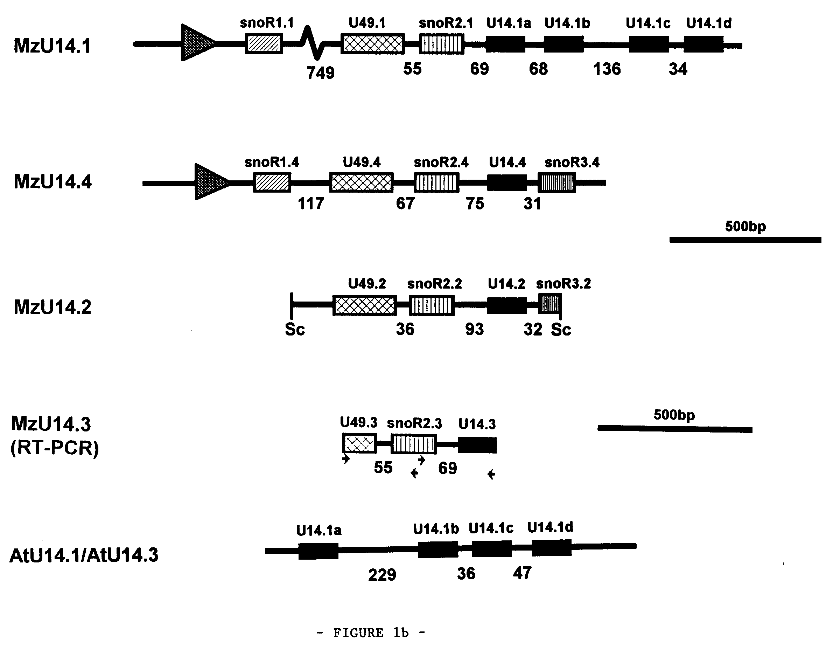

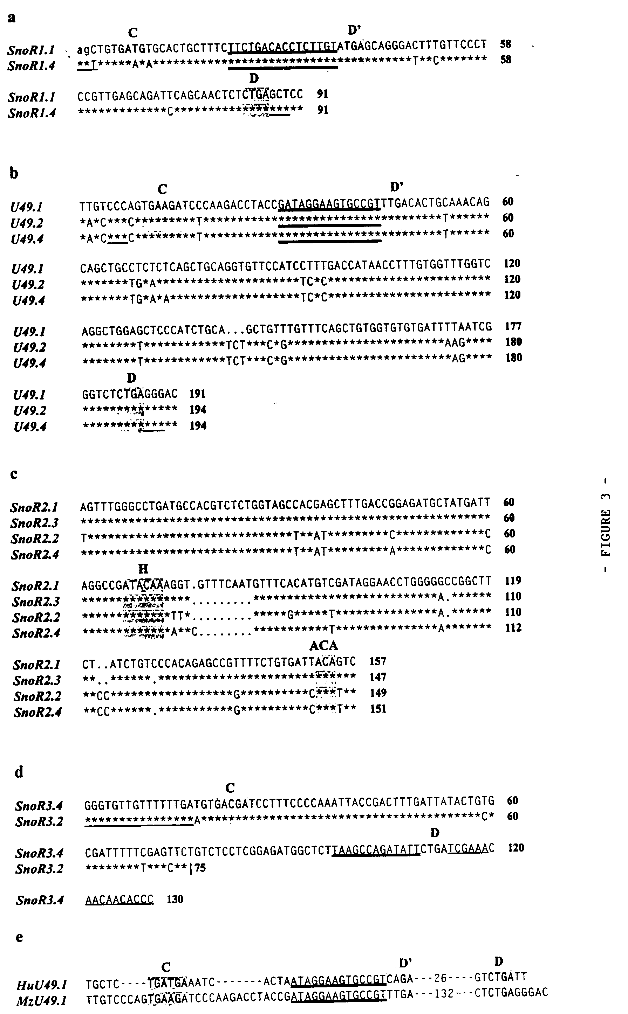

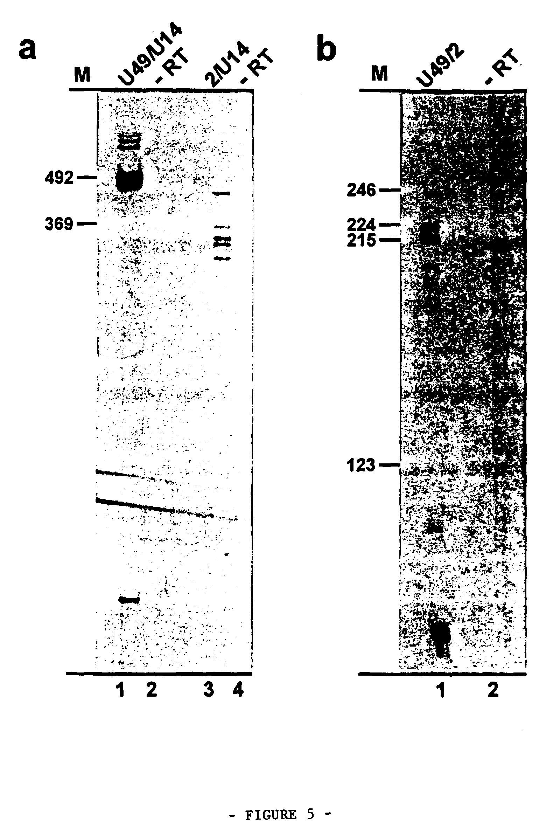

**Figure 1**. Organization of maize snoRNA genes. a) The position of snoRNA genes on the 4.3 kbp EcoRI fragment of MzU14.1 and the 2.2 kbp EcoRI-BamHI fragment of MzU14.4 are shown with fragments used in Northern analyses. b) Detailed gene organisation of the genomic clones MzU14.1, MzU14.2 and MzU14.4 and the cloned RT-PCR fragment (MzU14.3). Intergenic distances are given, and the gene sizes are presented in Figure 3. The approximate positions of gene-specific primers used in intergenic RT-PCR (Figure 5) are indicated by arrowheads on MzU14.3. U14 clusters of Arabidopsis thaliana are also shown (AtU14.1 / AtU14.3). Genes are represented by boxes, and flanking and intergenic regions by solid lines. Conserved upstream regions representing putative promoter elements are indicated as large arrowheads. B - BamHI; E - EcoRI; H - HindIII; N - NsiI; P - PstI; S - SphI; Sc - SacI and MCS - multiple cloning site.

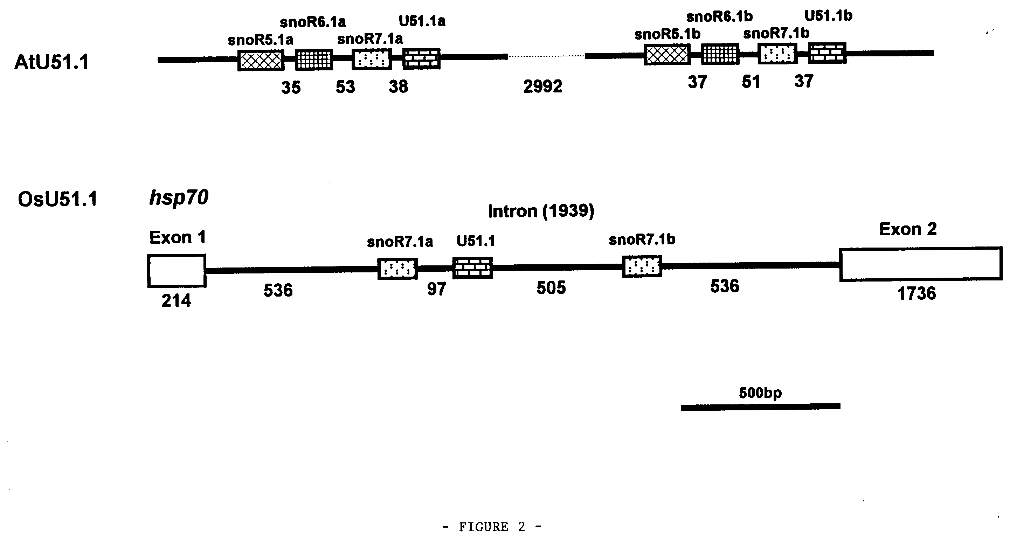

**Figure 2.** Organization of snoRNA genes in Arabidopsis thaliana (At) and Oryzae sativa (Os). Arabidopsis - 2 clusters of 4 different genes including U51 approximately 3Kb apart. Os : intron-encoded U51 cluster. Intergenic distances are given.
**Figure 3.** Sequence comparison of different alleles of the maize snoRNAs. (a) snoR1, (b) U49, (c) snoR2, (d) snoR3 and (e) alignment of human and maize U49. The 5' and 3' ends of the snoR1, snoR2 and snoR3 gene products were mapped using a combination of primer extension analysis and RNase A/T1 protection with probes specific to the 5' and 3' ends of the molecules (not shown). The ends of the snoR4 products have not been determined experimentally and the sequences presented are defined by the box C/D sequences and extensive inverted repeats. Nucleotide identities are shown by asterisks, inverted repeat sequences are underlined and sizes of coding regions are given. Conserved snoRNA motifs box C, box D, box D' , box H and ACA are shaded. Complementarities between snoRNAs and ribosomal RNAs are doubly underlined. Nucleotides which are not present in the mature snoRNA but form potential inverted repeats are shown in lower case.

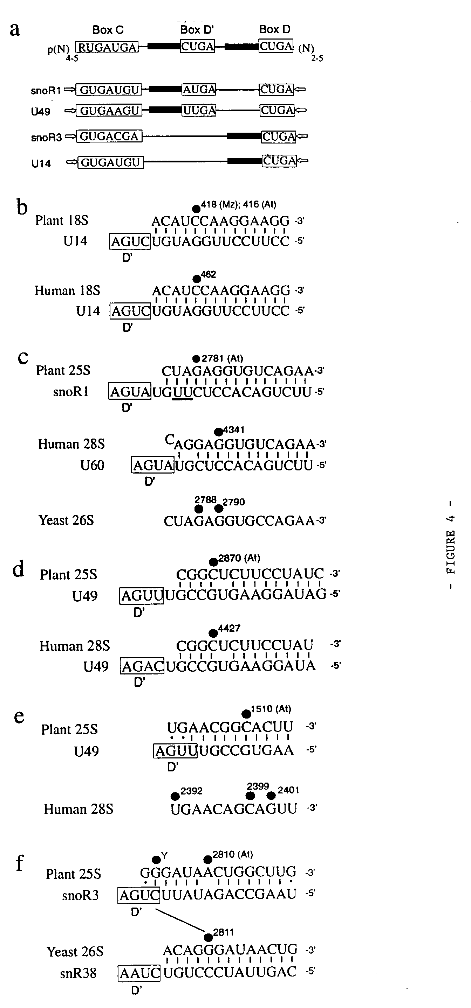

**Figure 4.** Structure of U14, snoR1, U49 and snoR3 RNAs and complementarity to plant 18S and 25S rRNAs. a) The structure of the maize snoRNAs are shown in comparison to the consensus structure for box C/D snoRNAs (Kiss-László et al., 1996). Box C, D and D' sequences are given, inverted repeats are represented by arrows and the positions of regions of complementarity to 18S and 25S rRNAs, adjacent to box D or D' sequences, are indicated by thick black lines. b-f). Potential base-pairing interactions between complementary regions of maize snoRNAs and plant 18S and 25S rRNAs (Mz - maize; At - Arabidopsis thaliana). These interactions are compared to the equivalent or overlapping interactions between human or yeast snoRNAs and rRNAs (Kiss-László et al., 1996; Nicoloso et al., 1996), or to the equivalent rRNA sequence from human or yeast. b) U14/18S; c) snoR1/25S; d and e) U49/25S - two potential regions of complementarity to 25S exist although the complementary region in U49 overlaps; f) snoR3/25S. The putative sites of plant rRNA ribose methylation and known sites of human or yeast rRNA methylation (Maden, 1990a) are indicated by black dots.
**Figure 5.** Detection of polycistronic snoRNA transcripts by RT-PCR. RT-PCR amplification (a) between U14 and U49 (lane 1), and U14 and snoR2 (lane 3), and (b) between U49 and snoR2 (lane 1). Lanes 2, 4 (a) and lane 2(b) (-RT) - control RT-PCR reactions carried out without reverse transcriptase.

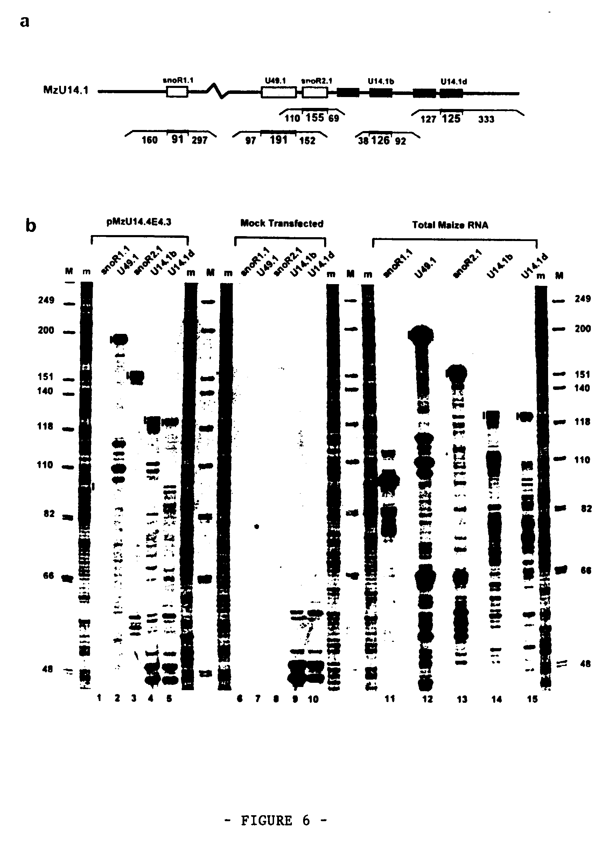

**Figure 6.** Expression of multiple snoRNAs from the maize U14.1 genomic clone in transfected tobacco protoplasts. (a). Schematic representation of RNaseA/T1 probes specific to each of the snoRNA genes encoded by MzU14.1 showing expected full-length protected products (thicker bars) and flanking sequences complementary to cloned sequences (thin lines). Angled lines represent transcribed vector-derived sequences. (b) RNase A/T1 analysis of RNA isolated from tobacco protoplasts transfected with pMU14.1E4.3 (lanes 1-5), mock-transfected tobacco protoplasts (lanes 6-10) or maize leaf total RNA (lanes 11-15) with probes specific to snoR1.1 (lanes 1, 6 and 11), U49.1 (lanes 2, 7 and 12), snoR2.1 (lanes 3, 8 and 13), U14.1b (lane 4, 9 and 14) or U14.1d (lanes 5, 10 and 15). Vertical bars show full-length protected products. M - [³²P]-end-labelled HindIII-digested φX174 DNA markers; m - DNA sequence markers.

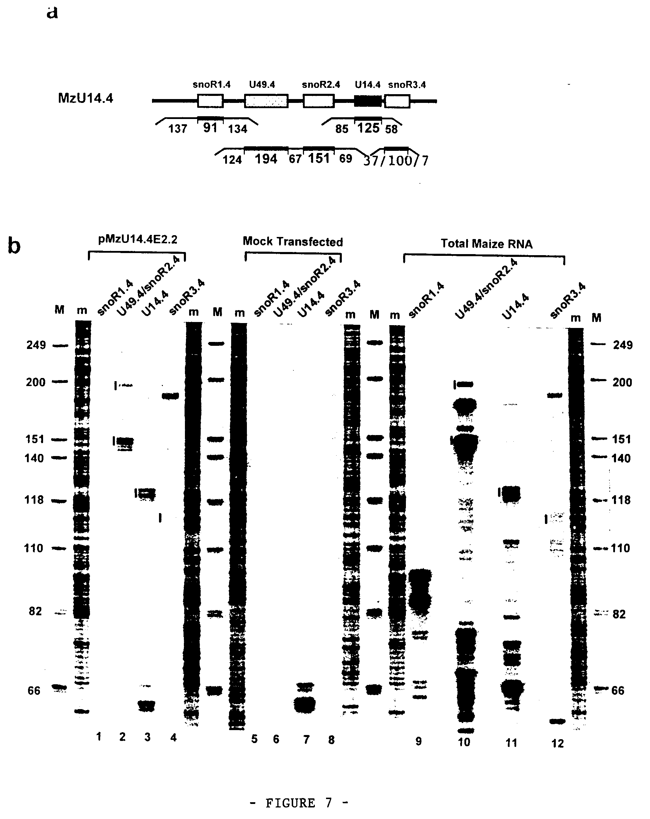

**Figure 7.** Expression of multiple snoRNAs from the maize U14.4 genomic clone in transfected tobacco protoplasts. (a). Schematic representation of RNaseA/T1 probes specific to each of the snoRNA genes encoded by MzU14.4 (see legend to Figure 6). (b) RNase A/T1 protection analysis of RNA isolated from tobacco protoplasts transfected with pMU14.4E2.2 (lanes 1-4), mock-transfected tobacco protoplasts (lanes 5-8) or maize leaves (lanes 9-12) with probes specific to snoR1.4 (lanes 1, 5 and 9), U49.4 and snoR2.4 (lanes 2, 6 and 10), U14.4 (lane 3, 7 and 11) or snoR3.4 (lanes 4, 8 and 12). Vertical bars show full-length protected products. M - [³²P]-end-labelled HindIII-digested φX174 DNA markers; m - DNA sequence markers.

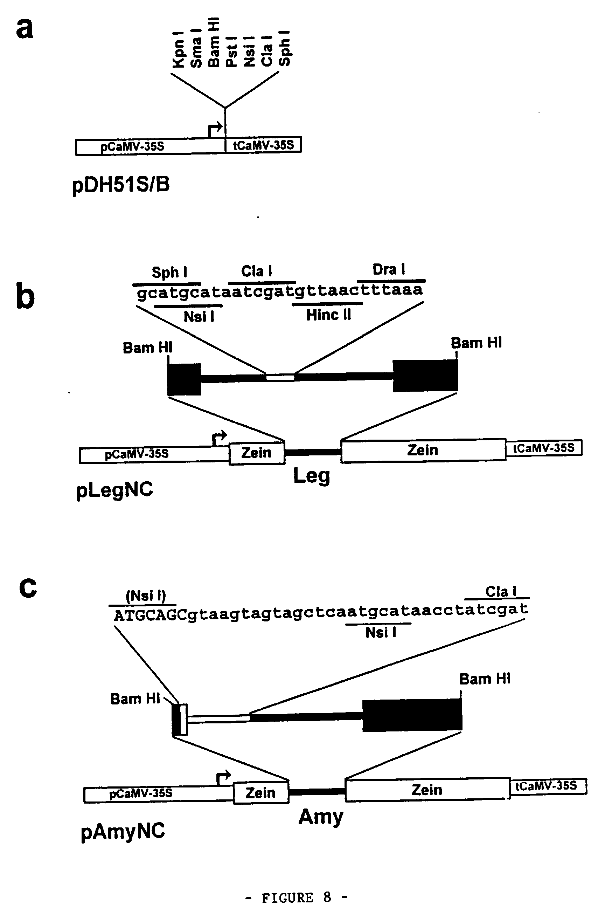

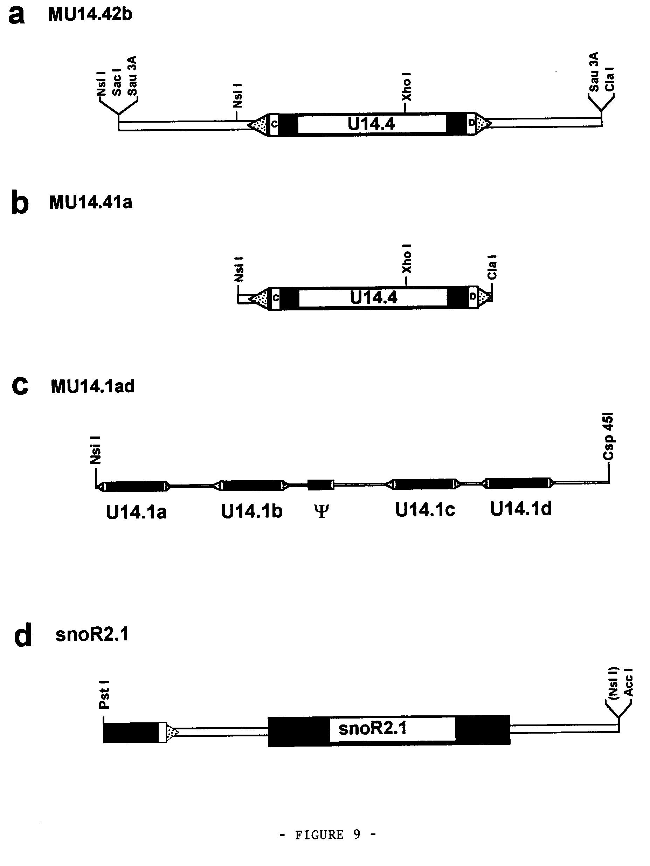

**Figure 8.** Vectors for expression of snoRNAs in different transcriptional contexts. a) pDH515S/B for non-intronic transcription, b) pLegNC and c) pAmyNC for intronic transcription. The CaMV 35S promoter and terminator regions are shown as narrow open boxes, and the transcription start as an arrow. The zein protein coding sequence is shown as a larger open box and the legumin and amylase intron insertions as large filled boxes (authentic exon sequence) and thick black lines (introns). The sequences of polylinkers introduced into the introns are given. SnoRNA constructs (Figure 9) are introduced as NsiI-ClaI fragments.
**Figure 9.** SnoRNA gene fragments for analysis of processing in vivo in protoplasts. a) MU14.42b - the U14.4 coding region plus flanking sequences. b) MU14.41a - the U14.4 coding region with minimal flanking sequences. c) MU14.1a-d - the MU14.1 gene cluster containing the four complete U14 genes (U14.1a-d) and a gene fragment (Ψ). d) snoR2.1 - the box H/ACA snoR2.1 gene plus flanking sequences. Coding regions are shown as filled boxes ; boxes C and D as small open boxes and shaded triangles represent inverted repeats.

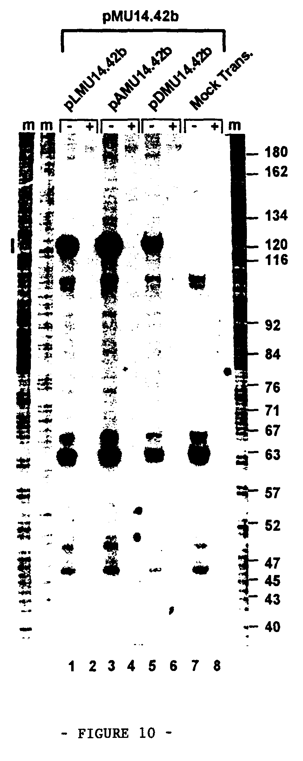

**Figure 10.** Processing of U14.4 with authentic flanking sequences from intronic and non-intronic transcripts. RNase A/T1 protection mapping of RNA from protoplasts transfected with pLMU14.42b (lanes 1 and 2), pAMU14.42b (lanes 3 and 4), pDMU14.42b (lanes 5 and 6), and mock-transfected protoplasts (lanes 7 and 8). RNA was probed with antisense (-) or sense (+) MU14.42b probes. m-DNA sequence markers. Full length products are indicated by the bar on the left hand side.

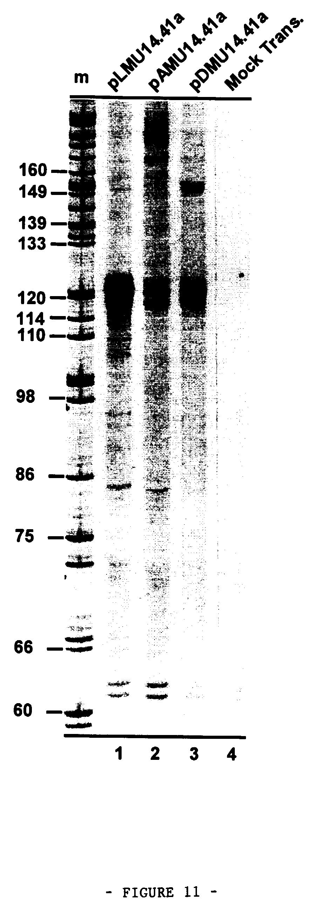

**Figure 11.** Processing of U14.4 with minimal authentic flanking sequences from intronic and non-intronic transcripts. RNase A/T1 protection mapping of RNA from protoplasts transfected with pLMU14.41a (lanes 1), pAMU14.41a (lanes 2), pDMU14.41a (lanes 3) and mock-transfected protoplasts (lanes 4). RNA was probed with antisense probes specific to the U14.4 coding region and flanking regions of each of the three constructs. m - DNA sequence markers.

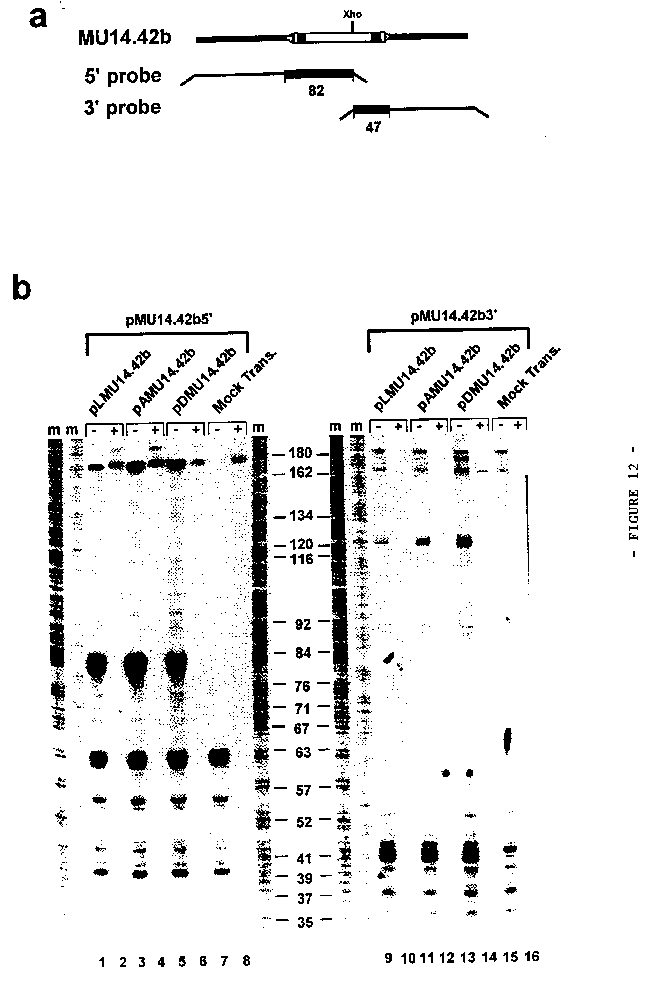

**Figure 12.** Processing of 5' and 3' ends of U14.4 from intronic and non-intronic transcripts. a) Schematic representation of the MU14.42b gene fragment and the positions of the 5'- and 3'-specific probes. The regions complementary to the coding region are shown as thick black lines and the expected product size is given. b) RNase A/T1 protection mapping of RNA from protoplasts transfected with pLMU14.42b (lanes 1, 2, 9 and 10), pAMU14.42b (lanes 3, 4, 11 and 12), pDMU14.42b (lanes 5, 6, 13 and 14) and mock-transfected protoplasts (lanes 7, 8, 15 and 16). RNA was probed with antisense (-) or sense (+) 5'-specific (lanes 1-8) or 3'-specific (lanes 9-16) probes. m - DNA sequence markers.

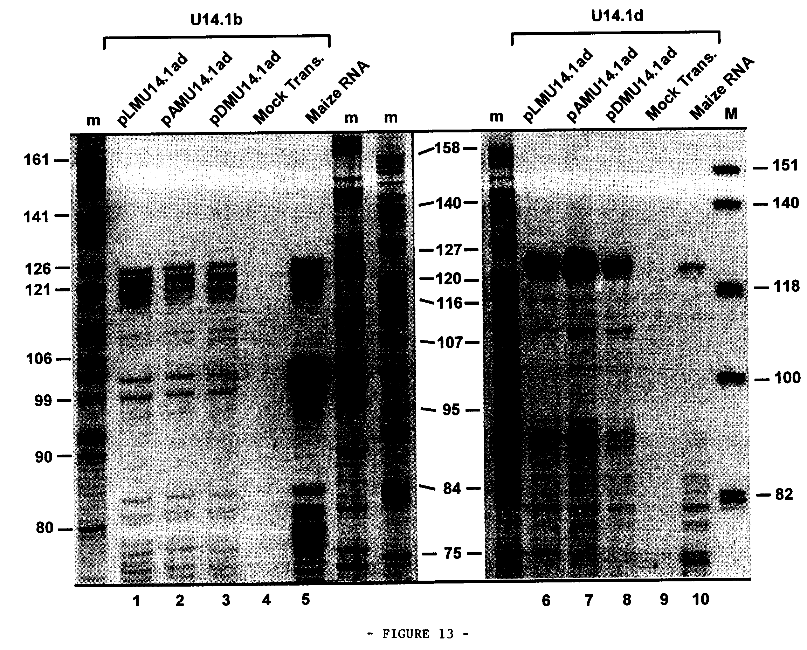

**Figure 13.** Processing of multiple U14 genes from intronic and non-intronic transcription constructs. RNASE A/T1 protection mapping of RNA from tobacco protoplasts transfected with pLMU14.1ad (lanes 1 and 6), pAMU14.1ad (lanes 3 and 7), pDMU14.1ad (lanes 5 and 8), mock-transfected protoplasts (lanes 7 and 9) and RNA from total maize leaf (lanes 5 and 10). RNA was probed with probes specific to U14.1b (lanes 1-5) or U14.1d (lanes 6-10). M - [32P]-end-labelled HindIII-digested φX174 DNA markers ; m - DNA sequence markers.

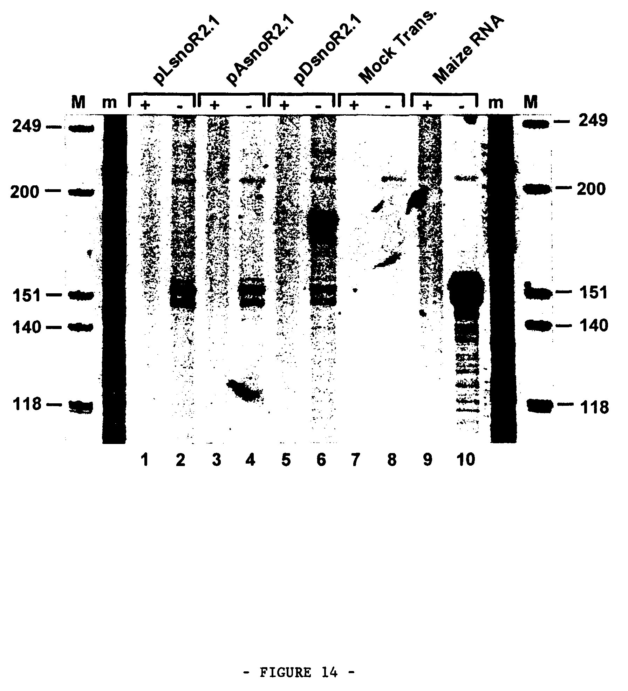

**Figure 14.** Processing of snoR2.1 from intronic and non-intronic transcripts. RNase A/T1 protection mapping of RNA from protoplasts transfected with pLsnoR2.1 (lanes 1 and 2), pAsnoR2.1 (lanes 3 and 4), pDsnoR2.1 (lanes 5 and 6), mock-transfected protoplasts (lanes 7 and 8) and RNA from maize leaf. RNA was probed with antisense (-) or sense (+) snoR2.1 probes. M - [32P]-end-labelled HindIII-digested φX174 DNA markers ; m - DNA sequence markers.

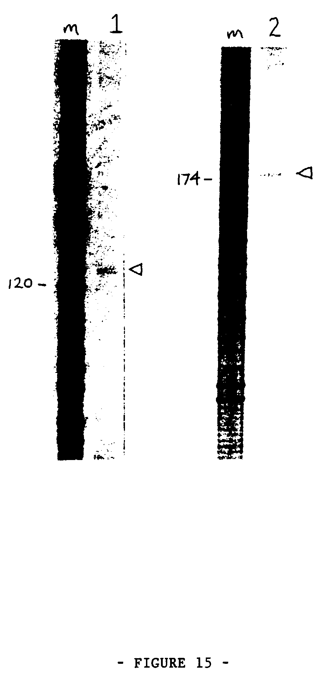

**Figure 15.** RNase A/T1 protection mapping of potato U14.1 and U14.1/ribozyme construct. RNase A/T1 mapping was carried out on total potato leaf RNA with an antisense probe to potato U14.1 (lane 1) and on RNA isolated from tobacco protoplasts transfected with the potato U14.1/ribozyme in pLegNC (lane 2). Expected products of 125 and 175 nt were observed. M - DNA sequencing markers.
**Figure 16.** Comparison of regions upstream of the snoR1 genes of MzU14.1 and MzU14.4. Identification of a conserved region. The upstream sequences (MzU14.1 : nucleotides 1-850 ; MzU14.4 : nucleotides 1-880) of MzU14.1 and MzU14.4 were compared using the BESTFIT programme. Top sequence = MzU14.1 ; lower sequence = MzU14.4. Identity = 76%. These sequences are contained within the approximately 800 base pair upstream regions shown to have promoter activity. Numbering applied in this figure is consistent with that of figures 17 and 18. Dots in the sequences have been introduced to facilitate alignment.
**Figure 17.** Full sequence of MzU14.1 cluster (shown schematically in Figures 1a and 1b). MzU14.1 contains :
   - Upstream region -: nucleotides 1-850 approximately, having promoter activity and including conserved sequence between nucleotides 381-724.
   - Flanking sequences- 5' :: nucleotides 1-850 approximately ;
   3' : nucleotides 2912-3557 approximately.
   - SnoRNA coding sequences :: SnoR1 - nucleotides 853-945
   U49.1 - nucleotides 1695-1885
   SnoR2.1 - nucleotides 1941-2095
   U14.1a - nucleotides 2165-2287
   U14.1b - nucleotides 2356-2479
   U14.1c - nucleotides 2621-2749
   U14.1d - nucleotides 2782-2911
   (sequences U14.1a - U14.1d have been described in WO 95/30748).
   - Intergenic regions :: nucleotides 946-1694
   nucleotides 1886-1940
   nucleotides 2096-2164
   nucleotides 2288-2355
   nucleotides 2480-2620
   nucleotides 2750-2781
**Figure 18.** Full sequence of MzU14.4 cluster (shown schematically in Figures 1a and 1b). MzU14.4 contains :
   - Upstream region -: nucleotides 1-890 approximately, having promoter activity and including conserved sequence between nucleotides 454 and 794.
   - Flanking sequences - 5' :: nucleotides 1-890 approximately ;
   3' : nucleotides 1874-2034 approximately.
   - SnoRNA coding sequences :: SnoR1.4 - nucleotides 891-983
   U49.1 - nucleotides 1101-1294
   SnoR2.4 - nucleotides 1362-1512
   U14.4 - nucleotides 1588-1711
   SnoR3.4 - nucleotides 1744-1873
   - Intergenic regions :: nucleotides 984-1100
   nucleotides 1295-1361
   nucleotides 1513-1587
   nucleotides 1712-1743

### EXAMPLES

The inventors have shown previously that certain plant U14snoRNA genes are tightly clustered and transcribed polycistronically (Leader et al., 1994b and International patent application WO 95/30748). This novel genomic organization of snoRNA genes has led the inventors to investigate whether processing of the pre-U14snoRNA transcript requires endonucleolytic cleavage between individual U14s. In the following examples, sequences flanking the U14 genes in isolated maize genomic clones have been characterized. Instead of the expected exonic sequences, it was found that four novel plant snoRNA genes (both box C/D and the first plant box H/ACA snoRNA) were tightly linked to the U14 genes. The multiple snoRNA genes are non-intronic and are expressed from an upstream promoter as a polycistronic transcript, from which they are processed. It has also been demonstrated that processing of a U14 cluster is splicing-independent. Therefore, the evolution of a novel snoRNA gene organization in plants is complemented by a processing mechanism which allows release of individual snoRNAs from polycistronic transcripts. The organization of these multiple snoRNA genes is so far unique to plants.

The materials and methods used in these examples are set out in section II.

### I. RESULTS

### Example 1. 5' regions flanking maize U14snoRNAs contain other small RNA genes :

Isolation of two maize genomic clones ( MzU14.1 and MzU14.4) containing U14 genes has been described previously (Leader et al., 1994b).

A total of 1.96 kb and 0.65 kb of 5' and 3' sequence flanking the maize U14.1 gene cluster and 1.57 kb and 0.32 kb flanking the maize U14.4 gene have been generated. These sequence data have been submitted to the EMBL database under Accession Numbers Y11048 and Y11049. A number of short open reading frames were observed in the flanking sequences but none matched sequences in the database, or contained suitable combinations of splice site sequences to suggest that the U14 genes were contained within introns. Four regions of nucleotide homology (90-340 bp) (Figure 1 and 2A) were observed between the 5' flanking regions of the two genomic clones but did not correspond to potential open reading frames nor were putative amino acid sequences conserved.

To investigate whether sequences flanking the maize U14snoRNA genes corresponded to protein coding exons, subfragments of the genomic clones (Figure 1) were hybridised to total maize seedling RNA in Northern analyses. Initial formaldehyde-agarose Northern blots failed to detect higher molecular weight RNAs expected for host gene mRNAs but instead clearly showed hybridisation to small RNA species.

Further analysis was therefore carried out using denaturing 6% polyacrylamide gels to resolve the small RNAs. The 1045 bp PstI-PstI fragment containing the majority of the U14.1 gene cluster hybridised to a major band of around 120 nt (U14), to three bands in the 135-150 nt range, a band of 195 nt and a faint band of 250 nt in maize leaf total RNA. The 1083 bp PstI-PstI fragment of U14.1 lying upstream of the U14 gene cluster hybridised to two bands of approximately 90 and 195 nt. The 329 bp PstI-NsiI fragment ( U14.1) hybridised to the 195 nt RNA and to the group of bands of 135-150 nt (not shown). The 545 bp PstI-HindIII fragment ( U14.1) hybridised to the 90 nt RNA only. The 770 bp EcoRI-SphI fragment at the 5' end of the U14.1 clone and the PstI-PstI 677 bp and PstI-EcoRI 740 bp fragments at the 3' end (Figure 1a) did not hybridise to maize RNA (not shown). Finally, the 1178 bp EcoRI-PstI fragment of the U14.4 genomic clone hybridised to only the 90 nt and 195 nt RNAs. Taken together, these results indicated that other small RNA genes were located upstream of the U14 genes in both genomic clones. Furthermore, the patterns of hybridisation were consistent with three of the four regions of homology between the two genomic clones representing genes encoding the various small RNAs.

Fragments corresponding to these regions were either subcloned directly or isolated by PCR and subcloned into pGEM3Zf+ or pGEM7Zf- and used in Northern analysis with total maize seedling RNA. In addition to the conserved regions 5' to the U14 genes, the 3' regions were searched for potential snoRNA sequences on the basis of identifying box C/D sequences and adjacent inverted repeats. This analysis identified a putative small RNA gene 3' to the U14.4 gene. This region was also isolated by PCR and used as a probe in Northern analysis. The three upstream conserved regions which lay closest to the U14 genes hybridised to RNAs of 195 nt, 150 nt and 90 nt demonstrating that these regions indeed represented genes encoding small RNAs.

The furthest upstream of the three encoded the 90 nt RNA and the gene was called snoR1. The next gene encoded a 150 nt RNA, which when sequenced, contained the same region of complementarity to 28S rRNA as human U49 (Figures 5 and 6; Kiss-László et al., 1996) and was therefore called U49. The gene immediately 5' to U14 was called snoR2 and the small RNA gene downstream of U14.4 which hybridised to an RNA species of 103 nt was called snoR3.

In addition, clusters of snoRNA genes have been found in other species, for example Arabidonsis thaliana and Oryzae sativa (Figure 1B and Figure 2). In Oryzae sativa, the U51 cluster is intron-encoded in contrast to the maize and A. thaliana clusters in the above figures.

### Example 2. Nucleolar localisation of the small RNAs :

To demonstrate that the above genes encoded small nucleolar RNAs, gene-specific antisense probes were used in in situ hybridisations on maize root tip tissue sections. Nuclei were counterstained with the DNA-specific dye DAPI, resulting in nucleoli appearing as dark, unstained holes. An antisense U14 probe clearly labelled the nucleoli. The four novel snoRNAs were all localized to the nucleolus although the detailed patterns of hybridisation within the nucleolar region varied among the different genes. SnoR1 was detected at relatively low levels throughout the nucleolus and particularly in a region at the centre of the nucleolus. The labelling patterns seen with U49 and snoR2 probes were similar to that seen with U14 which was previously shown to label the dense fibrillar component (Beven et al., 1996). SnoR3 was present throughout the nucleolus. As previously observed with U3, U14 and 7-2/MRP in plant nuclei (Beven et al., 1996), each of the snoRNAs often showed a concentration of labelling in the central nucleolar cavity.

### Example 3. The novel snoRNA genes encode box C/D and box H/ACA-type snoRNAs :

The order of snoRNA genes was conserved between the MzU14.1 and MzU14.4 genomic clones (Figure 1b). Southern analysis suggested that there are 8-10 genomic locations of U14 genes in maize (results not shown). Isolation of a third genomic clone (MzU14.2) and an RT-PCR product (MzU14.3) (see below) confirmed conservation of gene order at these different loci (Figure 1b). The 5' and 3' ends of snoR1, U49 and snoR2 were determined by a combination of primer extension and RNase A/T1 mapping using probes spanning the whole gene and 5'-specific probes (results not shown).

The sequences of the gene variants from the three genomic clones and the RT-PCR product are aligned in Figure 3.

SnoR1, U49 and snoR3 genes, like U14, encode box C/D-type snoRNAs, containing conserved box C and D sequences and inverted repeats at their 5' and 3' ends (Figures 3 and 4a). As with vertebrate box C/D snoRNAs, regions of complementarity to rRNAs which specify sites of ribose methylation (Kiss-László et al., 1996; Nicoloso et al., 1996; Cavaillé et al., 1996) are present in these plant snoRNAs (Figures 3 and 4). The regions of complementarity of U14, snoR1 and U49 RNAs coincided with methylation sites conserved in yeast and human rRNAs (Figure 4b-e). The U14 sequence is highly conserved in all eukaryotes and the base-pairing interaction is identical to that of human (Figure 4b) (Maden, 1990a; Kiss-László et al., 1996; Nicoloso et al., 1996). This sequence would specify ribose methylation at position Cm418 in maize (Messing et al., 1984) and Cm416 in Arabidopsis 18S rRNA (Unfried et al., 1989).

The sequence of complementarity in snoR1 is similar to that of human U60 but contains two extra nucleotides which would specify ribose methylation of maize 25S rRNA two nucleotides upstream of the equivalent position in human 28S (Figure 4c). This position is not methylated in human 28S rRNA, but is methylated in yeast 26S (Maden,1990a). Thus, snoR1 would specify methylation at position Gm2781 in Arabidopsis 25S rRNA (Unfried and Gruendler, 1990) which corresponds to Gm2788 in yeast (Maden, 1990a).

A sequence in the maize U49 is complementary to two regions of 25S rRNA. The first, specifying methylation at position Cm2870 in Arabidopsis 25S rRNA, is virtually identical to that of human U49 (Kiss-László et al., 1996), even containing the same U-U mismatch in the base-paired region (Figure 4d). The equivalent position is methylated in both yeast and human. Interestingly, although the maize and human U49s contained the same complementary sequence, besides boxes C and D, the RNAS were otherwise unrelated (Figure 3e). The maize U49 (195 nt) is more than twice the length of its human counterpart (71 nt - Kiss-László et al., 1996). The second region of potential complementarity in U49, although only 10 bp long, would specify methylation at position Cm1510 in Arabidopsis 25S rRNA (Figure 4e) and the eqivalent position in human 28S (Cm2399) is methylated but is unmethylated in yeast (Maden, 1990a).

The region of complementarity found in snoR3 would specify methylation at position Am2810 in Arabidopsis 25S rRNA (Figure 4f). This position is not known to be methylated in other eukaryotes, although it lies close to a known site of methylation in yeast 25S rRNA (position Gm2811) (Maden,1990a) such that the complementary sequence overlaps that of yeast snR38 (Kiss-László et al., 1996; Nicoloso et al., 1996).

Thus, for the most part, the plant box C/D snoRNAs contain complementary regions to rRNAs at known sites of ribose methylation in other eukaryotes. This strongly suggests that these snoRNAs are also involved in this post-transcriptional modification and that the mechanism of determination of the position of methylation is conserved in plants.

SnoR2 contained the sequence, ACA, 3 nt from the predicted 3' end of the molecule. Secondary structure models predict stem-loop structures in the 5' and 3' halves of the molecule and box H (consensus ANANNA) in the single-stranded hinge region as found in other box H/ACA-snoRNAs (Balakin et al., 1996; Ganot et al., 1997). Thus, snoR2 appears to belong to the box H/ACA class and represents the first plant box H/ACA snoRNA gene to be isolated.

### Example 4. Polycistronic expression of multiple snoRNAs :

The original cluster of U14 genes isolated on genomic clone MzU14.1 were shown to be expressed on the same polycistron by RT-PCR (Leader et al., 1994). The discovery of multiple, closely linked snoRNAs suggests that these genes may also be polycistronically expressed. This was supported by the lack of known plant promoter elements, particularly those of UsnRNA genes (TATA box; Upstream Sequence Element and Monocot Specific Element - Vankan and Filipowicz, 1992; Connelly et al., 1994) and RNA polymerase III elements, between adjacent genes. In addition, the small intergenic distances which ranged in size from 31-136 bp (with the exception of snoR1.1 and U49.1 which lie 749 bp apart) (Figure 1) further suggested that individual genes do not contain their own promoter elements.

RT-PCR reactions were therefore carried out using primers designed to amplify between different combinations of snoRNA genes: U49/snoR2, U49/U14 and snoR2/U14 (Figure 1b; Figure 5a and b). RT-PCR using labelled primers produced a number of bands which corresponded largely to the sizes of products expected from the sequences of U14.1 and U14.4. For the U49/U14 and snoR2/U14 primer combinations, bands of 489/502 bp and 344/348 bp were expected and bands of similar size were observed (Figure 5a, lanes 1 and 3 respectively). RT-PCR products of around 200-225 bp were obtained with the U49/snoR2 primer combination (Figure 5b, lane 1) corresponding to the sizes expected from the genomic clones of 213/224 bp. In all cases, a number of bands of varying sizes were obtained, reflecting the amplification from different genetic loci. No products were observed in any of the RT-PCR controls lacking reverse transcriptase (Figure 5a, lanes 2 and 4; Figure 5b, lane 2). An RT-PCR product from the U49/U14 reaction was cloned and sequenced, and contained the 3' half of an U49 allele (U49.3), a complete snoR2 allele (snoR2.3) and most of a U14 allele (U14.3) (Figures 1b and 3).

Thus, the novel snoRNAs are transcribed on the same polycistronic transcript as the U14 genes.

### Example 5. Expression, in protoplasts, of clustered plant snoRNAs from novel upstream promoters :

The tight linkage of the multiple snoRNAs and their polycistronic transcription suggests that the snoRNAs are transcribed from an upstream promoter. While it is feasible that the snoRNA clusters lie within an intron of a protein coding gene, no open reading frame (exon) sequence could be identified in either U14.1 or U14.4. In addition, a highly conserved region of 340 bp (75% identical) was present upstream of the snoR1 genes, which did not hybridise to small RNAs on Northern analyses (data not shown) were present (Figure 1) and could represent promoter regions.

In order to investigate whether the cloned plant snoRNA gene loci contain all of the sequences necessary for expression of the snoRNA polycistrons, tobacco protoplasts were transfected with plasmids containing each of the snoRNA gene clusters. The plasmid, pMU14.1E4.3 (containing the full MzU14.1 sequence), which encodes snoR1.1, U49.1, snoR2.1 and the maize U14.1 gene cluster (Figure 6a), contained 830 bp of sequence upstream of the snoR1.1 gene, including the 340 bp region of sequence homology, and 1.52 kbp downstream of the U14 gene cluster.

Protoplasts from three transfections were pooled and RNA isolated. In addition, RNA was isolated from three sets of mock-transfected protoplasts from the same batches used for the transfections. Finally, total maize leaf seedling RNA was included as positive controls.

RNA from approximately 2.5 x 10⁵ transfected (Figure 6b, lanes 1-5) or untransfected (Figure 6b, lanes 6-10) protoplasts or 5µg of total maize RNA (Figure 6b, lanes 11-15) was used in RNase A/T1 analysis with gene-specific probes to snoR1.1 (Figure 6b, lanes 1, 6 and 11), U49.1 (Figure 6b, lanes 2, 7 and 12), snoR2.1 (Figure 6b, lanes 3, 8 and 13), U14.1b (Figure 6b, lanes 4, 9 and 14) and U14.1d (Figure 6b, lanes 5, 10, and 15).

Full-length protected products were detected for four of the snoRNAs encoded by pMU14.1E4.3. The U49.1 probe protected products of approximately 195 nt (lane 2), the snoR2.1 probe detected bands of approximately 150-155 nt (lane 3) and the two U14 probes each detected bands of approximately 117-124 nt (lanes 4 and 5). The full-length products protected for each probe corresponded to products protected by total maize RNA (lanes 12-15). The similarity in banding patterns, where some probes produce multiple bands in both tobacco and maize (for example, snoR2.1 and U14.1b - lanes 3 and 13, and 4 and 14 respectively) represent transcripts which differ slightly in length due to the extent of processing as observed previously for other snoRNAs (Balakin et al., 1994; Kiss and Filipowicz, 1995).

In addition to products corresponding to transcripts from the genes under study, maize total RNA also protected a number of shorter fragments due to the presence of other sequence variants.

No full-length protected products were detected with RNA from mock-transfected protoplasts, but shorter fragments representing partial protection of endogenous tobacco snoRNAs were observed with the U14 probes. The probe specific to snoR1.1 protected a faint product of approximately 103 nt and a much stronger band of 85-90 nt with total maize RNA (Figure 6b, lane 11). The latter is the expected size of full-length protected product for snoR1.1. A similar product is visible with RNA from transfected protoplasts (Figure 6b, lane 1) but is present at a level approximately 100-fold less than the other snoRNAs.

These results indicate that the 4.3kb EcoRI fragment encoded by pMU14.1E4.3 contains sufficient sequences for the efficient expression and processing of at least four of the snoRNAs of the gene cluster.

In addition, the results demonstrate that the maize (monocot.) promoter functions in tobacco (dicot.) protoplasts. The absence of strong protected products corresponding to snoR1.1 may indicate that this snoRNA is unstable in tobacco protoplasts (although this appears unlikely from the presence of strong products in total maize RNA). Alternatively, the transcription start site of the maize promoters may be inaccurate in tobacco protoplasts, interfering with expression of snoR1.1 by, for example, truncating the 5' end of the snoRNA.

RNaseA/T1 protection mapping of RNA from tobacco protoplasts transfected with the plasmid, pMzU14.4E2.2 (containing the full MzU14.4 sequence), mock-transfected protoplasts and total maize leaf RNA are shown in Figure 7. This plasmid contains the snoR1.4, U49.4, snoR2.4, U14.4 and snoR3.4 genes with 880 bp upstream of snoR1.4 and 150 bp downstream of snoR3.4 (Figure 7a). RNAs from transfected protoplasts were probed with gene-specific probes to snoR1.4 (Figure 7b, lane 1), both U49.1/snoR2.4 on the same probe (lane 2), U14.4 (lane 3) and snoR3.4 (lane 4). No full-length products were observed with RNA from mock-transfected protoplasts (Figure 7b, lanes 5-8) but were obtained with total maize RNA (Figure 7b, lanes 9-12).

In the plasmid transfected protoplasts, as with pMzU14.1E4.3 (Figure 6), protected products for snoR1.4 were extremely faint (Figure 7b, lane 1). The probe specific to both U49.4 and snoR2.4 protected full-length products of expected size (195 nt and 152-155 nt respectively - Figure 7b, lane 2). The U14.4 full-length products were 121-124 nt (lane 3). Finally, the probe to snoR3.4, the gene downstream of U14.4 (Figure 7a), showed only a faint band of expected size (118 nt) (Figure 7b, lane 4) - the corresponding products protected by maize total RNA Figure 7b, (lane 12) are also faint. Thus, this snoRNA variant may be inherently unstable, but the levels of product may also reflect the fact that the snoRNA gene and therefore probe contains extended terminal inverted repeats which can potentially form an intermolecular stem of 16 bp. This, in addition to any internal secondary structure may preclude the probe from efficient hybridisation to target RNAs. Nevertheless, the protection of full-length products corresponding to U49.4, snoR2.4, U14.4 and snoR3.4 shows that the transfected plasmid contains the regulatory sequences required for expression of the snoRNAs in the cluster and that the promoter is active in tobacco. Given the fairly limited sequences upstream of the snoRNAs on the two plasmids, it seems likely that the conserved sequences may form at least part of the promoter region directing polycistronic transcription of the snoRNA gene clusters.

### Example 6. snoRNA with authentic flanking sequences (U14.4) is processed from both non-intronic and intronic transcripts :

Vertebrate snoRNAs are each contained within a single intron and, with the exception of some Xenopus snoRNAs, are processed in a largely splicing-dependent manner involving exonuclease activity. The small intergenic distances and the lack of suitable intron (either normal or AT-AC type) splice sites between the clustered maize snoRNA genes suggest that the individual snoRNAs are not separated by mini-exons and must, therefore, be processed from the polycistronic transcript without the requirement for splicing.

To demonstrate that processing was splicing-independent, a 308bp NsiI-ClaI fragment containing the maize U14.4 gene with 83bp upstream of box C and 64bp downstream of box D (Figure 9a - MU14.42b) was introduced into three different plant expression vectors: pDH51S/B (a modified version of pDH51 - Pietrzak et al., 1986), and pLegNC and pAmyNC (modified versions of pL and pA (Simpson et al., 1993, 1996; see Materials and Methods; Figure 8a-c). The U14.4 construct would thus be transcribed as part of an mRNA transcript from the CaMV 35S promoter either as :
- a non-intronic transcript in pDH51S/B (pDMU14.42b) or,
- within the efficiently spliced legumin intron in pLegNc (pLMU14.42b) or,
- within the poorly spliced amylase intron in pAmyNC (pAMU14.42b).

RNA was extracted and analysed by RNase A/T1 protection mapping (Figure 10) with a labelled antisense probe complimentary to the U14.4 insert. Accumulation of U14.4 was observed in protoplasts transfected with pLMU14.42b (Figure 10, lane 1), pAMU14.42b (lane 3) and pDMU14.42b) (lane 5). All three expression constructs produced three major protected products of 121-125 nt. These corresponded to protection products detected with the same probe using total RNA isolated from maize leaf RNA (Figure 7b, lane 11). These results demonstrate that plant snoRNA processing is splicing-independent.

### Example 7. snoRNA (U14.4) with minimal flanking sequences is processed from both non-intronic and intronic transcipts :

In Xenopus U14, the signals required for processing are contained within the coding region of the U14 genes (Watkins et al., 1996; Xia et al., 1997). As the genomic organization of the plant snoRNAs in this study (clustering and polycistronic transcription) implies that endonucleolytic cleavage occurs between individual snoRNAs, it was possible that sequences outside the U14 coding regions might be required for recognition by an endonuclease. To investigate this, a U14.4 fragment with minimal flanking sequences (Mu14.41a - Figure 9b) was generated by PCR and inserted into the three expression vectors : pDH51S/B, pLegNC and pAmyNC. The fragment contained the entire coding sequence of the U14.4 gene including the flanking inverted repeats with 8nt of upstream sequence and 2nt of downstream sequence.

These plasmids were transfected into tobacco protoplasts and extracted RNA was probed with a set of antisense probes corresponding to the three transfected constructs. That is, the probes consisted of the MU14.41a sequence with flanking sequences corresponding to either the legumin or amylase introns, or the CaMV 35S 5' and 3' transcribed sequences. RNase A/T1 protection mapping gave full length protected products with a similar pattern of fully processed U14.4 products from all three constructs (Figure 11, lanes 1-3). Thus, U14.4 lacking its authentic flanking sequences is correctly processed from both intronic and non-intronic transcripts suggesting that the sequences required for U14 processing are contained within the minimal U14 sequence used, and probably within the U14 sequence itself.

### Example 8. Demonstration that endonucleolytic cleavage of the authentic flanking sequences of monocistronic snoRNA does not occur at a conserved site :

To examine whether endonucleolytic cleavage of the authentic flanking sequences occurred at a preferred or conserved site, processing of U14.4 (MU14.42b - Figure 9a) was investigated using 5' and 3' specific probes (Figure 12a). RNase A/T1 analysis of RNA isolated from protoplasts transfected with the three expression vectors containing MU14.42b is shown in Figure 12b. Again processed U14.4 is observed from all three constructs confirming the splicing-independence of processing (Figure 12b, lanes 1, 3 and 5). The 5'-specific probe protected multiple products of 80-83 nt. In addition, larger protected fragments corresponding to protection of unprocessed U14.4 are also observed. Faint protected products of >180 nt in lanes probed with sense probe are indicative of residual plasmid DNA in the RNA preparations and the product of around 178 nt in these lanes appears to be a tobacco-specific product as it is found in untransfected protoplasts probed with the MU14.42b sense probe (Figure 12b, lane 8). The bands of 39-63 nt in lanes 1, 3, 5 and 7 are protected fragments of tobacco U14s as they are found in untransfected protoplast RNA (Figure 12b, lane 7). The 3'-specific probe generated similar results in that the only strongly protected products represented unprocessed U14.4 (products of around 120 nt) and fully processed U14.4 (products of 41-45 nt). Multiple 3' ends are again observed indicative of variable processing at the 3' end. The full-length protected products overlap protected fragments of tobacco U14 (Figure 12b, lane 15) and are not observed with sense probes (Figure 12b, lanes 10, 12, 14 and 16).

The sizes of the fully processed products are consistent with processing being halted at or within the terminal inverted repeats. No strongly protected bands representing specific cleavage intermediates are observed with either the 5' or 3'-specific probes. Instead a faint smear with a number of bands is observed. Thus, procesing of U14.4 does not appear to occur via well-defined intermediates as seen in processing of Xenopus U16/U18 (Caffarelli et al., 1996) or in in vitro processing of some human snoRNAs (Tykowski et al., 1993; Kiss and Filipowicz, 1993, 1995).

### Example 9. Polycistronic plant U14s can be efficiently processed from introns and non-intronic transcripts :

To demonstrate that multiple U14snoRNAs can be processed from single intronic and non-intronic transcripts, a fragment containing the entire U14.1 gene cluster (Figure 9c) was cloned into the three expression vectors described above (pDH51S/B, pLegNC and pAmyNC). The fragment was generated by PCR and contained 13 bp upstream of the inverted repeat sequence of the U14-1a gene located at the 5' end of the cluster. The 3' end of the fragment contained a PCR-generated Csp 45I site located 115 bp downstream of the box D sequence of the U14-1d gene located at the 3' end of the cluster (Figure 9c).

Each of the three constructs were transfected into tobacco protoplasts and the isolated RNA aliquoted and probed with antisense RNA probes to U14.1b (Figure 13, lanes 1-3) and U14-1d (Figure 13, lanes 6-8). Full length protection products of approximately 120-125nt were detected with both probes for all three constructs. Thus, individual U14snoRNA variants were fully processed from both intronic and non-intronic sequences in expression plasmids in tobacco protoplasts. This confirms processing to be splicing-independent and suggesting a requirement for endonuclease activity. From the general features of the multiple snoRNA gene clusters in plants and the likely association of proteins with each snoRNA sequence, it seems unlikely that only a single snoRNA is produced from each transcript. In control experiments an aliquot of protoplasts was mock-transfected with sterile water. None of the U14 protected products were observed (Figure 13, lanes 4 and 9), while maize seedling total RNA protected full length products (Figure 13, lanes 5 and 10).

### Example 10. Processing of snoR2, a box H/ACA-type snoRNA, is also splicing-independent :

The maize snoRNA gene clusters contain snoR2 alleles which are the only box H/ACA-type snoRNA genes to have been isolated from plants. To demonstrate that processing of this class of snoRNAs in plants is also splicing-independent, a 334bp Pst I/Acc I fragment containing the snoR2.1 gene (Figure 9d) was subcloned into each of the expression vectors to produce pLsnoR2.1, pAsnoR2.1 and pDsnoR2.1.

RNA was isolated from protoplasts transfected with each of the snoR2.1 constructs and used in RNase A/T1 protection analysis (Figure 14). The RNA was incubated with either an antisense (Figure 14, lanes 2, 4, 6, 8 and 10) or sense probe (Figure 14, lanes 1, 3, 5, 7 and 9). Several products of between 148 and 156 nt, of which the products of 152 nt and 153 nt were the most prominent, were observed in all cases (Figure 14, lanes 2, 4 and 6). These products were equivalent to full-length protected bands produced by protection with total RNA isolated from maize leaf tissue (Figure 14, lane 10). No products were detected with the sense probe (Figure 14, lanes 1, 3 and 5). Mock transfected protoplasts did not contain full length protected products (Figure 14, lane 8). Thus, the box H/ACA-type snoR2.1 can be correctly processed from either the legumin intron (lane 2), the amylase intron (lane 4) or from a non-intronic transcript (lane 6).

In addition to the full-length snoR2.1 products produced from pDsnoR2.1, a group of products was detected between 184 and 191nt. These products are approximately 30-40 nt longer than the expected snoRNA products. The identity of these bands is unknown at present but seem unlikely to represent processing intermediates produced by specific cleavage because their size would place any potential cleavage sites within authentic snoR2 flanking sequences which are also present in the accompanying intronic clones and transcripts. That the same products are not observed when snoR2.1 is processed from an intronic transcript nor from its native polycistronic transcript, suggests that they are an artefact of transcription/processing from the pDH51S/B vector possibly resulting from altered secondary structure of this particular transcript.

### Example 11. Construction of clone containing potato U14.4 gene with anti-GUS ribozyme :

A fragment of the potato U14.1 clone was generated by PCR using the primers, PU14Ns2 (5'-GGGATGCATAAAGGGACTACACTGGAATTTG-3') and PU14Nar (5'-CCATAGTGAGGCGCCTGTTATTTCATTCC-3'). The amplification product consists of the complete potato U14.1 gene with 100 bp of 5' and 3' flanking sequences, with NsiI and NarI restriction fragment sites at the 5' and 3' ends respectively. The insert was cloned into the NsiI and ClaI sites of pGEM7Zf(-), and of the three expression vectors pDH51S/B, pLegNC and pAmyNC.

A hammerhead ribozyme directed against the marker gene GUS was synthesised and contained two 12-mers complementary to GUS and a catalytic region to form the hammerhead structure as described in EP-B-321201. This oligonucleotide was annealed to its complement such that the DNA fragment had -GATC overhanging ends for introduction into the BglII site (see below).

The anti-GUS ribozyme has a total length of 50nt. The anti-GUS ribozyme was introduced in to the unique BgLII site in the potato U14 gene of each of these constructs. Following transfection of the plasmids into tobacco protoplasts, RNase A/T1 analysis of isolated RNA detected a full-length protected product of 175 nt. This product was of the expected size (U14 - 125 nt ; anti-GUS ribozyme - 50 nt) (Figure 15).

This construct can be used to inactivate GUS transcribed in a transformed plant cell.

### Example 12. Use of novel snoRNA promoter in a chimeric gene - Construction of maize U14 cluster promoter/GUS fusions :

Fusion constructs of the regions 5' upstream of the U14.1 and U14.4 gene clusters to the GUS coding sequence were made to examine expression of a protein coding sequence from the snoRNA promoters. The initial stage in the cloning procedure was the introduction of a novel polylinker sequence into pGEM3Zf(+). This was achieved by ligation of annealed complementary primers polySH3 (5'-CGTCGACAAGGATCCAAGAGCTCTGCAGGTACCA-3') and polySH4 (5'-AGCTTGGTACCTGCAGAGCTCTTGGTACCTTGTCGACGCATG-3') into the HindIII and SphI sites of pGEM3Zf(+). The 5' flanking region of MzU14.1 was isolated as an approximately 800 bp EcoRI-SphI fragment (SphI cuts 25 bp 5' to the start of the snoR1.1 gene - see Figure 17). This fragment was cloned into the modified pGEM3Zf(+). To remove the ATG codon contained in the SphI site, the resulting plasmid was lincarised with SphI, treated with Pfu Taq polymerase which removed the overhanging ends of the digested DNA and religated.

To create the GUS fusion, the GUS coding region and Cauliflower Mosaic Virus (CaMV) 35S terminator sequence were isolated from the plasmid, pJIT68, by a partial BamHI/BglII digest. This fragment was cloned into the BamHI site of the 5' region clone. Production of the MzU14.4 5' region/GUS fusion was carried out in exactly the same way except that the initial 5' region fragment was generated by PCR using a primer M4USphI (5'-TCTTGCATGCTACCATATCCCTGTGTGACG-3') in combination with a plasmid vector primer. The SphI site generated by PCR was adjacent to the start of the snoR1.4 gene. This SphI site was removed and the GUS coding sequence and CaMV 35S terminator was introduced as described above.

These constructs may be used to express GUS in both monocot. and dicot. plant cells and plants.

It has been shown in the above examples that U14snoRNA genes in plants are found in gene clusters alongside other different snoRNA genes. The occurrence of multiple, different snoRNA genes in tightly linked groups is presently unique to plants. The detection of polycistronic transcripts suggests that the snoRNA gene clusters are transcribed together as pre-snoRNA transcripts. Moreover this implies that release of individual snoRNAs will require endonuclease activity. It has been demonstrated that sequences upstream of the gene clusters are sufficient for production of a series of different fully processed snoRNAs. Thus, some plant snoRNAs have a mode of snoRNA expression distinct from the expression strategies employed by animals and yeast: transcription from classical promoter elements or intron-encoded (Maxwell and Fournier, 1995).

The snoRNA genes found linked to U14 encode either box C/D snoRNAs or box H/ACA-snoRNAs. The box C/D snoRNAs contain regions of complementarity to rRNAs at sites of ribose methylation in human and yeast. These snoRNAs are likely, therefore, to function in the determination of sites of rRNA ribose methylation as recently demonstrated for a number of animal snoRNAs (Kiss-László et al., 1996; Nicoloso et al., 1996; Cavaillé et al., 1996). Very little information exists on plant rRNA ribose methylation (Maden, 1990b) and to date no methylation sites have been mapped. However, detailed studies on yeast, human and Xenopus, have shown many methylation sites to be conserved although each organism can contain unique sites (Maden, 1990a). The location of the complementary regions of some of the plant box C/D snoRNAs at known sites of methylation in yeast and human strongly suggests that the mechanism of determination of sites of ribose methylation is conserved in plants. The snoR2 alleles are the first box H/ACA-type snoRNA genes to have been isolated from plants. A number of examples have been identified in vertebrates and yeast (Balakin et al., 1996; Ganot et al., 1997) and they appear to act as guide RNAs determining sites of pseudouridine modifications in rRNAs (T. Kiss, unpublished). The likely conservation of these snoRNAs in plants again suggests conservation of function in rRNA modification.

The small distances between adjacent snoRNA genes and the lack of known promoter elements suggest that they are not transcribed individually from classical snRNA promoters. The absence of normal and AT-AC intron splice site consensus sequences in the intergenic regions further suggests that unlike the majority of vertebrate and many yeast snoRNAs, the plant snoRNAs are not intron-encoded. The detection of polycistronic RNAs by RT-PCR and of fully processed snoRNAs from along the gene clusters following transfection of the maize genomic fragments into tobacco protoplasts showed that the gene clusters were transcribed from upstream promoters.

The activity of the maize promoter regions in tobacco is of interest because of the clear distinction which exists between the genes encoding spliceosomal snRNAs and nucleolar U3 and MRP snoRNAs in the two angiosperm classes (monocots and dicots) (Connelly et al., 1994). Expression of these genes in monocots requires a unique element - the Monocot-Specific Promoter (MSP) element - which is absent in dicot genes and is not needed for expression in dicots (Connelly et al., 1994). Transcription from both genomic fragments, each containing approximately 800 bp of sequence upstream of the snoR1 genes, suggests that the 340 bp conserved regions in the two promoters may represent or contain elements essential to expression. These regions did not contain classical snRNA promoter elements, but contained a putative TATA box at around -200 in both genomic clones, and were GC-rich (62%), reminiscent of housekeeping genes (Dynan, 1986).

The occurrence of multiple, different snoRNA genes in tightly linked groups is as yet unique to plants. In the above examples, it has been demonstrated that splicing-independent release of both single and multiple snoRNAs (both box C/D and box H/ACA) occurs, in contrast to the intronic snoRNAs of vertebrates and yeast which are, for the most part, released in a splicing-dependent manner via exonucleolytic digestion of debranched intron lariats (Kiss and Filipowicz, 1995; Kiss et al., 1996; D. Tollervey, personal communication). The requirement for splicing to generate a linear snoRNA-containing transcripts for exonucleolytic processing further explains the fact that only a single snoRNA gene is found in any one intron in vertebrates (Kiss and Filipowicz, 1995). On the other hand, splicing-independent processing of U16 and U18 in Xenopus has been shown to involve endonucleolytic activity which cleaves sequences flanking the snoRNA prior to exonucleolytic trimming (Caffarelli et al., 1996). This situation appears to be peculiar to the Xenopus oocyte system because the U16 and U18 genes still follow the "one snoRNA, one intron" rule, and possibly reflects low levels of debranching enzyme in these cells. The organisation of the yeast snoR190 and U14 genes as a dicistron is currently the example of snoRNA gene orgnisation which is most reminiscent of the non-intronic clustering of plant snoRNA genes. SnoR190 and U14 lie 67 bp apart and transcription as a dicistron would again imply that endonucleolytic cleavage is needed to generate the two snoRNAs (D. Tollervey, personal communication).

All of the snoRNAs examined here accumulate when expressed from a non-intronic construct transcribed from the CaMV 35S promoter. The analogous non-intronic construct containing vertebrate U17 without exons and transcribed from the cytomegalovirus promoter, produced U17 at greatly reduced levels than when in an intronic context (Kiss and Filipowicz, 1995). Thus, consistent with their gene organisation and mode of expression, plant snoRNAs are processed in a splicing-independent manner. The intronic constructs also accumulated snoRNAs in plant cells. However, it is not possible to distinguish between direct processing from the pre-mRNA transcript or processing from the linearised intron, and presumably both can occur.

Although endonucleolytic activity is implied from the gene organisation of the snoRNA clusters, little evidence of distinct intermediate products representing specific endonuclease cleavage sites was obtained in the various RNase A/T1 analyses. This is in contrast to the processing of U16 and U18 in Xenopus oocytes (Caffarelli et al., 1994) . In vitro analyses of vertebrate intronic snoRNA substrates has shown processing of the 5' and 3' flanking sequences to be independent of one another and to produce a longer lived intermediate with a short extension at the 3' end. Thus, either cleavage between the plant snoRNAs occurs at a specific site or randomly in the intergenic regions, and the termini are rapidly degraded by exonucleases, or cleavage occurs in the vicinity of the snoRNA (perhaps by direct recruitment of the endonuclease by components of the snoRNP), leaving only short sequences to be trimmed by exonucleases. An anlaysis of the intergenic sequences between the various snoRNA genes failed to identify a conserved sequence which might act as a recognition site for a specific endonuclease, and the smear observed in many RNase A/T1 analyses argues against the latter possibility and for cleavage at random sites.

The presence of pre-snoRNA transcripts in the nucleolar cavity, shown by in situ hybridisation using probes to intergenic regions, may suggest that at least some pre-snoRNA transcripts localise to this region such that the nucleolar cavity may represent a site of processing. More detailed analyses with other probes may help to enlighten this. Thus, transcripts may be transported to the nucleolus by virtue of their association with abundant nucleolar proteins such as fibrillarin, SSB1 or Gar1p and processed in the nucleolus or nucleolar cavity. Interestingly, plant snoRNAs, and especially the more abundant snoRNAs - U3, U14 and MRP, have all been shown to accumulate in the nucleolar cavity (Beven et al., 1996; Leader et al., 1997). It is not known whether this region represents a site of processing or assembly. The presence of U3 and MRP which are not found in gene clusters and which undergo only minor processing of their 3' ends, may suggest that assembly occurs here.

### II. MATERIALS AND METHODS

### Construction of snoRNA gene plasmids

For initial Northern analyses various fragments of the genomic clones were isolated and subcloned (Figure 1a) by standard techniques. Plasmids for production of antisense probes specific to each of the snoRNA genes for use in in situ hybridisation were prepared by PCR from appropriate subclones and contained the coding sequence of the gene with minimal flanking sequences. The snoR1.1 specific plasmid pgSnoR1.1 contained a 125bp PCR product generated with primers snS1F (5'-AAGGATCCGATAGCCTCCTAGTAGCTGTGATGTGC-3') and snS1R (5'-AAGAATTCGAGGGAGGAGCTCAGAGAGTTGC-3') (incorporated restriction sites underlined) and subcloned in pGEM3Zf+. Plasmid p7U49.1 contained a 191 bp EcoRI/BamHI fragment generated by PCR with primers snL1F (5'-AAGAATTCTTGTTTTAGTTTCCTGAATCTGCC-3') and snL1R (5'-AAGAATTCTACAGTCCCTCAGAGACC-3') subcloned in pGEM7Zf-. pgSnoR2.1 contained a 163bp Eco RI fragment generated by PCR with primers snM1F (5'-AACTGCAGTAGTTTGGGCCTGATGC-3') and snM1R (5'-AAGAATTCATGTAATCACAGAAAACGGC-3'). An antisense probe to the snoR3.4 gene was transcribed from plasmid pgSnoR3.4 which contained a Hind III site immediately upstream of the snoR3.4 5' inverted repeat sequence as a result of PCR with primer snoR3Hd (5'-GGGAAGCTTGTCCAGCTCTTCATCGTTC-3') and universal sequencing primer. In addition to the coding sequence of snoR3.4, pgSnoR3.4 also contains approximately 170bp downstream of the gene to an EcoRI site derived from the lambda vector. The plasmid pgMU14.1d has been described previously (Leader et al, 1994) and encodes the U14.1d gene from the maize U14.1 gene cluster.

Additional transcription plasmids for the preparation of RNaseA/T1 probes contained more extensive 3' and 5' flanking sequences. Plasmid pgS1PH contained a 548 bp Hind III/ Pst I fragment containing the complete snoR1.1 coding sequence and 160 and 297 bp of 5' and 3' flanking sequence. Plasmid pgU49.1 was made using PCR with the primer L1Ns1 (5'-GGATGCATTTGCCTAGCTTCCTGATG-3') which introduced an Nsi I site 104bp upstream of the U49.1 gene. Digestion with Nsi I and Taq I produced a 443bp fragment which was subcloned in pGEM3Zf(+). Probes specific to snoR2.1 were transcribed from pgM1PN and were complementary to snoR2.1, a 55 bp fragment of U49.1, 55bp of intergenic sequence and 69bp of sequence downstream of snoR2.1. Plasmid psnoR1.4 contained a 357bp Sac I/Bgl II fragment cloned in pGEM3Zf(+). Plasmid pgU49/2.4 contained a 623bp Sac I/Nsi I fragment including the complete coding sequences of both U49.4 and snoR2.4 as well as flanking and intergenic sequences, cloned in pGEM3Zf(+). Probes specific for the snoR3.4 gene were transcribed from the plasmid pgsnoR3.4 which contained a 145bp fragment generated with primers snoR3Hd (5'-GGGAAGCTTGTCCAGCTCTTCATCGTTC-3') and snoR3Sp (5'-CCCACTAGTTTCGATCAGAATATCTGGC-3') and cloned in pGEM3Zf(+).

### Construction of expression vectors expressing different snoRNA gene fragments

The plasmid pDH51S/B was a derivative of pDH51 (Pietrzak et al., 1986) in which part of the polylinker between the Sph I and BamH I was replaced with a novel polylinker produced by annealing the oligonucleotides DH51Sph (5'-CATCGATGATGCATCTGCAG-3') and DH51Bam (5'-GATCCTGCAGATGCATCATCGATGCATG-3'). The new polylinker contained BamH I, Pst I, Nsi I, Cla I and Sph I sites downstream of the cauliflower mosaic virus 35S (CaMV 35S ) promoter (Figure 10a). Plasmids pAmyNC and pLegNC were derivatives of the previously described plasmids pA and pL respectively (Simpson et al., 1996). Both of these plasmids were based on pDH51, into which an intron-less zein gene was introduced between the CaMV 35S promoter and terminator regions. This gene contained a unique BamH I site into which either an amylase intron (pA) or a legumin intron (pL) were introduced. Plasmid pLegNC was generated by replacing a 27nt Dra I/Sph I fragment in the legumin intron with an artificial linker made by annealing oligonucleotides LegDra (5'-CATAATCGATGTTAACTTT-3') and LegSph (5'-AAAGTTAACATCGATTATGCATG-3'). This generated Nsi I and Cla I sites 52 nt and 59 nt downstream of the legumin 5' splice-site respectively (Figure 10b) and resulted in the substitution of 5 nt within the intron with no change in AU content. Suitable restriction sites were inserted into the amylase intron by PCR with pA, using primers AMYNC (5'-ACCCTGCAGCGTAAGTAGTAGCTCAATGCATAACCTTATCGATAACATTTC-3') and O9 (Simpson et al., 1996) which is complementary to part of the downstream zein sequence. Following digestion with Pst I and Bam HI, the 189 bp PCR fragment was used to replace the equivalent Nsi I/Bam HI fragment of pA. This destroyed the Nsi I site located 7 nt upstream of the amylase intron 5' splice-site and generated novel Nsi I and Cla I sites 16 nt and 27 nt downstream of the 5' splice-site respectively (Figure 10c). These changes resulted in the substitution of 7 nt within the intron although the overall AU content was only increased by 1% and length of the intron was maintained.

The maize U14.4 gene was subcloned into the above vectors as the MU14.42b fragment (Figure 9A). This was initially isolated as a 263bp Sau3A I fragment, containing 93bp of sequence upstream of box C and 64bp of sequence downstream of box D, and subcloned into the Bam HI site of pGEM7Zf(-) to give plasmids pMU14.42b(+) and pMU14.42b(-) differing in the orientation of the cloned fragment. For cloning into the expression vectors a 302 bp Nsi I/Cla I fragment was isolated from pMU14.42b(+). This fragment contained an additional 39bp of sequence derived from the pGEM polylinker.

The MU14.41a fragment contained the U14.4 coding sequence and adjacent 6bp inverted repeats with a total of 16bp of wild-type sequence upstream of box C and 8bp of wild-type sequence downstream of box D. A Cla I site was introduced 6nt downstream of box D by PCR with primer MU144Cla (5'-GCCGATCGATATGGCTCAGACATCCAAGG-3'). This site was used with the Nsi I site located 16bp upstream of box C for cloning into the expression vectors.

The entire maize U14.1 gene cluster was isolated as a single fragment, MU141ad, by PCR with primers MU14L14 (5'-CATAACCTTTGTGGTTTGGTC-3') and MU141Cs (5'-CCTTTCGAATCACACAGGCAATAAACTGGAGGTGC-3') on plasmid pMU14-1E4.3. Following digestion with Nsi I and Csp45 I the 854bp product contained each of the four maize U14-1 coding sequences with 13 bp of sequence upstream of the box C sequence of U14-1a, including the Nsi I site, and 115bp of sequence downstream of the box D sequence of U14-1d including the artificial Csp45 I site introduced by the primer.

The snoR2.1 gene was subcloned into pGEM3Zf(+) as a 334bp Pst I/Nsi I to give pgsnoR2.1. This fragment contained the 155bp coding sequence of the snoR2.1 gene with 110bp and 69bp of 5' and 3' flanking sequence respectively. The snoR2.1 fragment was subsequently reisolated following digestion with Pst I and Acc I and subcloned into the expression vectors. This fragment contained 6nt of additional sequence derived from the pGEM polylinker.

### RNA and Northern analysis

RNA was isolated from leaf material of maize and potato as described previously (Chomczynski and Sacchi, 1987). 10ug of total maize seedling RNA was separated on a denaturing 6% polyacrylamide gel and electroblotted onto Hybond N+. The blot was cut and individual strips were hybridised to [³²P]-labelled probes. The probe for snoR1 was a 475bp NsiI-HindIII fragment generated by PCR with the primer MU14.1NsSp (5'-CCGCATGCATTAGCTTGAACTAGAATGCCTGCG-3') and universal reverse sequencing primer, cloned into pGEM7Zf+ to give plasmid p7Sno1NsiH. The gene specific probes for U49, snoR2 and snoR3 were the inserts of plasmids p7U49.1, pgSnoR2.1 and pgSnoR3.4 respectively (described above). Probes were labelled with [³²P]-dCTP using random priming kit (Boehringer-Mannheim).

### In situ hybridisation

Root tips were excised from 3 day old maize seedlings, fixed in 4% formaldehyde in a buffer containing 50 mM PIPES-KOH , pH 6.9, 5 mM EGTA, 5 mM MgSO4. After thorough wasking with TBS (25 mM Tris-HC1, pH 7.4, 140 mM NaC1 and 30 mM KC1), 50 µm sections were cut with a vibratome and dried onto microscope slides. In situ hybridisation was carried out as described previously (Beven et al., 1995 ; 1996) with digoxygenin-labelled antisense RNA probes. The hybridised probe was detected by a primary anti-digoxygenin FAB fragment and secondary fluorescein- or Cy3-coupled antibody. Confocal data sets were collected using a BioRad MRC 600 or MRC 1000 UV confocal microscope.

### Protoplast transfections

Protoplasts were isolated from fully expanded leaves of six to eight week old N. tabacum cv. Xanthii as described by Chupeau et al. (1994). Approximately 2.5 x 10⁵ protoplasts were transfected with 15µg of doubly caesium chloride purified plasmid DNA and incubated for 24h prior to RNA extraction. Protoplasts were also co-transfected with the plasmid pMzU5.3 which contains the maize U5.3 gene (Connelly et al.,1994). RNA was isolated from protoplasts or from plant tissues by the guanidinium iso-thiocyanate method according to Goodall et al. (1990). RNA was treated twice with RNase-free DNase, prior to use in RNase A/T1 and RT-PCR analysis.

### RT-PCR and RNase A/T1 protection analysis

Probes for the detection of expression from vectors containing the MU14.42b fragment were transcribed from the plasmid pMU14.42b(-). This probe was complementary to the entire MU14.42b fragment with additional nucleotides derived from the pGEM vector.

Specific transcription constructs were made to produce probes specific to each of the MU14.41a constructs. Bam HI fragments of 315 bp and 320 bp were isolated from pAMU14.41a and pLMU14.41a respectively and subcloned into pGEM7Zf(-) to give p7AMU14.41a and p7LMU14.41a respectively. Probes transcribed from pAMU14.41a were complementary to the MU14.41a sequence as well as 37 nt and 152nt of 5' and 3' flanking amylase sequences respectively. Probes transcribed from pLMU14.41a were complementary to the MU14.41a sequence as well as 76 nt and 118 nt of 5' and 3' flanking legumin sequence respectively. The plasmid pgDMU14.41a contained a 360bp fragment from pDMU14.41a cloned into pGEM3Zf(+) was complementary to 20 nt and 207 nt of 5' and 3' flanking sequence derived from pDH51S/B. Radiolabelled [³²P]-antisense and sense probes were generated by in vitro transcription with either SP6 or T7 RNA polymerase from appropriately lineasrised plasmids.

RT-PCR was carried out with 5'-end labelled primers as described previously (Simpson et al., 1996).

RNase A/T1 protection analysis was performed as described (Goodall et al., 1990) using 5µg of total RNA isolated from maize seedlings (cultivar Kelvedon Glory). RNA was isolated from leaf material of maize and potato as described previously (Chomczynski and Sacchi, 1987). [³²P]-labelled antisense probes for RNaseA/T1 protection analysis were transcribed from plasmids with either T7 or SP6 RNA polymerase following appropriate linearisation of the plasmids. Probes were treated with RQ1-RNase-free, DNase (Promega), gel purified and protected products were separated on an 8% polyacrylamide denaturing gel with sequencing reactions or a [³²P]-end-labelled HinfI-digested φX174 DNA as size markers.

### References :

- Bachellerie,J.-P., Michot,B., Nicoloso,M., Balakin,A., Ni,J. and Fournier MJ. (1995a) Antisense snoRNAs: A family of nucleolar RNAs with long complementarities to rRNA. Trends Biochem. Sci. **20,** 261-264.
- Bachellerie,J.-P., Nicoloso,M., Qu,L.-H., Michot,B., Caizergues-Ferrer,M., Cavaillé,J. and Renalier,M.-H. (1995b) Novel intron-encoded small nucleolar RNAs with long sequence complementarities to mature rRNAs involved in ribosome biogenesis. Biochem. Cell. Biol. **73**, 835-843.
- Baier G., Coggeshall K.M., Baier-Bitterlich G., Giampa L., Telford D., Herbert E., Shih W. & Altman A. (1994). Construction and characterisation of Ick- and fyn- specific tRNA : ribozyme chimeras. Mol. Immun., **31** : 923-932.
- Balakin,A.G., Lempicki,R.A., Huang,G.M. and Fournier,M.J. (1994) Saccharomyces cerevisiae U14 small nuclear RNA has little secondary structure and appears to be produced by post-transcriptional processing. J. Cell Biol. **269**, 739-746.
- Balakin,A.G., Smith,L. and Fournier,M.J. (1996) The RNA world of the nucleolus: two major families of small RNAs defined by different box elements with related functions. Cell **86**, 823-834.
- Beven,A.F., Simpson,G.G., Brown,J.W.S. and Shaw,P.J. (1995) The organisation of spliceosomal components in nuclei of higher plants. J. Cell Sci. **108**, 509-518.
- Beven,A.F., Lee,R., Razaz,M., Leader,D.J., Brown,J.W.S. and Shaw,P.J. (1996) The organisation of ribosomal RNA processing correlates with the distribution of nucleolar snRNAs. J. Cell Sci. **109**, 1241-1251.
- Bouvet P., Dimitrov S. & Wolffe A.P. (1994). Specific regulation of Xenopus chromosomal 5S rRNA gene transcription in vivo by histone H1. Genes and Develop., **8** : 1147-1159.
- Caffarelli,E., Arese,M., Santoro,B., Fragapame,P. and Bozzoni,I. (1994) In vitro study of processing of the intron-encoded U16 snoRNA in X. Laevis. Mol. Cell Biol. **14**, 2966-2974.
- Caffarelli,E., Fatica,A., Prislei,S., De Gregorio,E., Fragapane,P. and Bozzoni,I. (1996) Processing of the intron-encoded U16 and U18 snoRNAs: the conserved C and D boxes control both the processing reaction and the stability of the mature snoRNA. EMBO J. **15**, 1121-1131.
- Cavaillé,J., Nicoloso,M., Bachellerie,J.-P. (1996) Targeted ribose methylation of RNA in vivo directed by tailored antisense RNA guides. Nature **383**, 732-735.
- Cecconi,F., Mariottini,P. And Amaldi, F. (1995) The Xenopus intron-encoded U17 snoRNA is produced by exonucleolytic processing of its precursor in oocytes. Nucleic Acids Res. **23**, 4670-4676.
- Chen C.J., Banerjea A.C., Harmison G.G., Haglund K. & Schubert M. (1992). Multitarget ribozyme directed to cleave at up to nine highly conserved HIV-1 env RNA regions inhibits HIV-1 replicatio-potential effectiveness against most presently sequenced HIV-1 isolates. Nuc. Acid Res., **20** : 4581-4589.
- Chomczynski,P. and Sacchi,N. (1987) Single-step method of RNA isolation by acid guanidinium thiocyanate-phenol-chloroform extraction. Anal. Biochem. **162**, 156-159.
- Chupeau,Y., Bourgin,J.P., Missonier,C., Dorion,N. and Morel,G. (1974) Préparation et culture de protoplastes de divers Nicotiana. CR Acand Sci Paris **278D:** 1565-1568.
- Connelly,S. Marshallsay,C., Leader,D.J., Brown,J.W.S. and Filipowicz,W. (1994). Small nuclear RNAs genes transcribed by either RNA polymerase II or RNA polymerase III in monocot plants share three promoter elements and use a strategy to regulate gene expression different from that used by their dicot plant counterparts. Mol. Cell. Biol. **14**, 5910-5919.
- Cotten M. & Birnstiel M.L. (1989). Ribozyme mediated destruction of RNA in vivo. EMBO J., **8** : 3861-3866.
- DeYoung M.B., Kincade-Decker J., Boehm C.A., Riek R.P., Mamone J.A., McSwiggen J.A. & Graham R.M. (1994). Functional characterization of ribozymes expressed using U1 and T7 vectors for the intracellular cleavage of ANF mRNA. Biochemistry, **33** : 12127-12138.
- Dynan (1986), Trends Biochem., **11** : 196-197.
- Eichler,D.C. and Craig,N. (1995) Processing of eukaryotic ribosomal RNA. Prog. Nucleic Acid Res. Mol. Biol. **49**, 197-239.
- Ganot, P., Caizergues-Ferrer, M. and Kiss, T. (1997) The family of box ACA small nucleolar RNAs is defined by an evolutionarily defined secondary structure and ubiquitous sequence elements essential for RNA accumulation. Genes Dev (in press).
- Goodall,G.J., Wiebauer,K. and Filipowicz,W. (1990). Analysis of pre-mRNA processing in transfected plant protoplasts. Meth. Enzymol. **181,** 148-161.
- Huang,G.M., Jarmolowski,A., Struck,J.C. and Fournier,M.J.. (1992) Accumulation of U14 small nuclear RNA in Saccharomyces cerevisiae requires box C, box D and a 5',3' terminal stem. Mol. Cell Biol. **12**, 4456-4463.
- Kiss,T., Bortolin,M.-L. and Filipowicz,W. (1996) Characterisation of the intron-encoded U19 RNA, a new mammalian small nucleolar RNA that is not associated with fibrillarin. Mol. Cell. Biol. **16**, 1391-1400.
- Kiss,T. and Filipowicz,W. (1995) Exonucleolytic processing of small nucleolar RNAs from pre-mRNA introns. Genes Dev. **9**, 1411-1424.
- Kiss,T., Marshallsay,C. and Filipowicz,W. (1991) Alteration of the RNA polymerase specificity of U3 snRNAs during evolution and in vitro. Cell **65**, 517-526.
- Kiss T, Filipowicz W. 1993 Small nucleolar RNAs encoded by introns of the human cell cycle regulatory gene RCC1. EMBO J. 12:2913-2920.
- Kiss-László,Z., Henry,Y., Bachellerie,J.-P., Caizergues-Ferrer,M. and Kiss,T. (1996) Site-specific ribose methylation of preribosomal RNA: a novel function for small nucleolar RNAs. Cell **85**, 1077-1088.
- Lafontaine and Tollervey, (1995) Biochem. Cell Biol., **73** : 803-812.
- Leader,D.J., Connelly,S., Filipowicz,W, Waugh,R. and Brown,J.W.S. (1993) Differential expression of U5snRNA gene variants in maize (Zea mays) protoplasts. Plant Mol. Biol. **21**, 133-143.
- Leader,D.J., Connelly,S., Filipowicz,W and Brown,J.W.S. (1994a) Characterisation and expression of a maize U3snRNA gene. Biochim. Biophys. Acta. **1219**, 145-147.
- Leader,D.J., Sanders,J.F., Waugh,R., Shaw,P.J. and Brown,J.W.S. (1994b) Molecular characterisation of plant U14 small nucleolar RNA genes: closely linked genes are transcribed as a polycistronic U14 transcript. Nucl. Acid. Res. **22,** 5196-5200.
- Lygerou,Z., Allmang,C., Tollervey,D. and Seraphin,B. (1996). Accurate processing of a eukaryotic precursor ribosomal RNA by ribonuclease MRP in vitro. Science **272**, 268-270.
- Maden,B.E.H. (1990a) The numerous modified nucleotides in eukaruotic ribosomal RNA Prog. in Nucleic Acid Res. and Mol. Biol. **39**, 241-303.
- Maden, B. E. H. (1990b) The modified nucleotides in ribosomal RNA of man and other eukaryotes. In Gehrke,C.W. and Kuo,K.C.T. (eds.) Chromatogrphy and modification of nucleosides. Elsevier, Amsterdam, pp.B265-301.
- Maxwell,E.S. and Fournier,M.J. (1995). The small nucleolar RNAs. Annu. Rev. Biochem. **35,** 897-934.
- Messing,J., Carlson,J., Hagen,G., Rubenstein,I. and Oleson A. (1984) Cloning and sequencing of the ribosomal RNA genes in maize: the 17S region. DNA **3,** 31-40.
- Nicoloso,M., Qu,L.-H., Michot,B. and Bachellerie,J.-P. (1996) Intron-encoded, antisense small nucleolar RNAs: the characterisation of nine novel species points to their direct role as guides for the 2'-O-ribose methylation of rRNAs. J. Mol. Biol. **260**, 178-195.
- Peculis,B.A. and Steitz,J.A. (1994) Sequence and structural elements critical for U8 snRNP function in Xenopus oocytes are evolutionarily conserved. Genes and Devel. **8**, 2241-2255.
- Periman, R. et al, (1995) P.N.A.S., **92** : 6175-6179.
- Pietrzak,M., Shillito,R., Hohn,T. and Potrykus,I. (1986) Expression in plants of two bacterial antibiotic resistance genes after transformation with a newplant expression vector. Nucleic Acids Res. **14**, 5857-5868.
- Scanlon K.J., Jiao L., Funato T., Wang W., Tone T., Rossi J.J. & Kashani-Sabet M. (1991). Ribozyme mediated cleavage of c-fos mRNA reduces gene expression of DNA synthesis enzymes and metallothionein. Proc. Natl. Acad. Sci. U.S.A., **88** : 10591-10595.
- Simpson, G.G. et al, (1995) EMBO J., **14** : 4540-4550.
- Simpson,C.G., Clark,G., Davidson,D., Smith, P. And Brown, J.W.S. (1996) Mutation of putative branchpoint consensus sequences in plant introns reduces splicing efficiency. Plant J. **9**, 369-380.
- Sioud M., Natvig J.B., Forre O. (1992). Preformed ribozyme destroys tumor necrosis factor mRNA in human cells. J. Mol. Biol., **223** : 831-835.
- Sollner-Webb,B., Tycowski,K.T. and Steitz,J.A . (1995) Ribosomal RNA processing in eukaryotes. In Zimmerman RA and Dahlberg AE (Eds.) Ribosomal RNA. CRC Press, Boca Raton, pp 469-490.
- Terns,M.P., Grimm, C., Lund,E. and Dahlberg,J.E. (1995) A common maturation pathway for small nucleolar RNAs EMBO J. **14**, 4860-4871.
- Tycowski KT, Steitz JA. 1989 U3, U8 and U& comprise a new class of mammalian snRNPs localised in the cell nucleolus. EMBO J. 8, 3113-3119.
- Tycowski,K.T., Shu,M.D. and Steitz,J.A. (1993) A small nucleolar RNA is processed from an intron of the human gene encoding ribosomal protein S3. Genes and Devel. **6**, 1120-1130.
- Tycowski,K.T., Shu,M.D. and Steitz,J.A. (1996) A mammalian gene with introns instead of exons generating stable RNA products. Nature **379,** 464-466.
- Unfried,I. and Gruendler,P. (1990) Nucleotide - sequence of the 5.8S and 25S rRNA genes and of the internal transcribed spacers from Arabidopsis thaliana. Nucleic Acids Res. **18**, 4011.
- Unfried,I., Stocker,U. and Greundler,P. (1989) Nucleotide sequence of the 18S rRNA gene from Arabidopsis thaliana Col0. Nucleic Acids Res. **17**, 7513.
- Van der Ven WTG, Powell W, Ramsay G, Waugh R. 1990 Restriction fragment length polymorphisms as genetic markers in Vicia. Heredity 65:329-342.
- Vankan,P. and Filipowicz,W. (1992) A UsnRNA gene-specific upstream element and a -30 TATA box are required for transcritpion of the U2snRNA gene of Arabidopsis thaliana. EMBO J. **8**, 3875-3882.
- Venema,J. and Tollervey,D. (1995) Processing of pre-ribosomal RNA in Saccharomyces cerevisiae. Yeast **11**, 1629-1650.
- Watkins,N.J., Leverette,R.D., Xia,L., Andrews,M.T. and Maxwell,E.S. (1996) Elements essential for processing intronic U14 snoRNA are located at the termini of the mature snoRNA sequence and include conserved nucleotide boxes C and D. RNA **2,** 118-133.
- Xia L, Liu J, Sage C, Trexler EB, Andrews MT, Maxwell ES. 1995 Intronic U14 snoRNAs of Xenopus laevis are located in two different parent genes and can be processed from their introns during early oogenesis. Nucleic Acids Res. 23:4844-4849.
- Xia,L., Watkins,N.J. and Maxwell,E.S. (1997) Identification of specific nucleotide sequences and structural elements required for intronic U14snoRNA processing. RNA **3,** 17-26.
- Zagorski,J., Tollervey,D. and Fournier M.J. (1988). Characterisation of an SNR gene locus in Saccharomyces cerevisiae that specifies both dispensible and essential small nuclear RNAs. Mol. Cell Biol. **8,** 3282-3290

## Claims

1. Process for producing stabilised non-translated RNA molecules within a cell, comprising introducing into a plant or yeast cell a nucleic acid molecule comprising a 〈〈 precursor DNA 〉〉, said precursor DNA containing a coding sequence, or a cluster of coding sequences, each coding sequence encoding :
- a non-translated RNA sequence to be stabilized, which is capable of functionally interacting with a cellular component, and
- stabilising sequences comprising at least one protein binding site and, optionally, sequences capable of giving rise to secondary structure associated with said protein binding site, said stabilising sequences being heterologous with respect to the non-translated RNA sequence to be stabilized, the precursor DNA being under the transcriptional control of regulatory sequences functional in said cell,
whereby a transcript is produced by transcription of the precursor DNA, and one or more stabilized RNA units comprising the non-translated RNA sequence in association with the stabilising sequences is, or are, then produced by processing of the transcript by the cell's endogenous processing machinery.

2. Process according to claim 1 wherein the stabilizing sequences comprise at least one protein binding site and sequences capable of forming at least one stem/loop structure.

3. Process according to claim 2, wherein the stem structure is formed by base-pairing between the 5' and 3' extremities of the stabilized RNA unit.

4. Process according to claim 3 wherein the stabilizing sequences comprise at least one protein binding site and stem/loop structure derived from snoRNA.

5. Process according to claim 4 wherein the protein binding sites comprise at least one Box C element and at least one Box D element of a fibrillarin-binding snoRNA.

6. Process according to claim 5 wherein Box C element has the sequence XGANWP and Box D element has the sequence YXVA, wherein X represents U, G or A, preferably U, N represents any nucleotide preferably U, W represents G, C or U preferably G, P represents any nucleotide preferably A, Y represents C or U preferably C, and V represents G or U preferably G.

7. Process according to claim 4 wherein the protein binding sites comprise at least one H Box element and at least one ACA Box element of an H/ACA-type snoRNA.

8. Process according to claim 5 wherein the H Box element has the sequence ANANNA, wherein N represents any nucleotide, and the ACA Box element has the sequence ACA.

9. Process according to claim 1 wherein the non-translated functional RNA molecule comprises one or more antisense sequences or one or more ribozymes.

10. Process according to claim 9 wherein the precursor DNA contains a single coding sequence, and a monocistronic transcript is produced on transcription of the precursor DNA, and a single stabilized RNA unit is produced by processing of the transcript by the cell's endogenous processing machinery.

11. Process according to claim 10 wherein the precursor DNA contains a plurality, or so-called 〈〈 cluster 〉〉, of coding sequences, the coding sequences in the cluster being separated from each other by spacer sequences,
wherein said spacer sequences are devoid of exon sequences and of associated functional intron splice sites,
whereby a polycistronic transcript is produced on transcription of the precursor DNA, and a plurality of stabilised RNA units are produced by processing of the polycistronic transcript by the cell's endogenous processing machinery.

12. Process according to any one of claims 10 or 11 wherein the precursor DNA further comprises flanking sequences, on one or both sides of the coding sequence or cluster of coding sequences.

13. Process according to claim 12 wherein the flanking sequences are devoid of exon sequences and of associated functional intron splice sites.

14. Process according to claim 12 wherein the flanking sequences comprise exon sequences and intron splice sites.

15. Process according to claim 1 wherein the regulatory sequence is a promoter derived from a plant or yeast snoRNA gene cluster.

16. Process according to claim 1 wherein the regulatory sequence is an RNA polymerase II promoter.

17. Nucleic acid molecule, called precursor DNA, capable of giving rise, on transcription, to one or more stabilised non-translated RNA molecules, said precursor DNA, comprising:
- a regulatory sequence (I) capable of directing transcription in a plant or yeast cell, and
- a sequence to be transcribed (II), operably linked to the regulatory sequence (I), wherein said sequence (II) contains a coding sequence or a cluster of coding sequences, each coding sequence encoding :
a non-translated RNA sequence to be stabilised, which is capable of functionally interacting with a cellular component, and
stabilising sequences comprising at least one protein binding site and, optionally, sequences capable of giving rise to secondary structure associated with said protein binding site, said stabilizing sequences being heterologous with respect to the non-translated RNA sequence.

18. Nucleic acid molecule according to claim 17 wherein the stabilizing sequences comprise at least one protein binding site and sequences capable of forming at least one stem/loop structure.

19. Nucleic acid molecule according to claim 18, wherein the stem structure is formed by base-pairing between the 5' and 3' extremities of the stabilized RNA molecule.

20. Nucleic acid molecule according to claim 17 wherein the stabilizing sequences comprise at least one protein binding site and stem/loop structure derived from snoRNA.

21. Nucleic acid molecule according to claim 18 wherein the protein binding sites comprise at least one Box C element and at least one Box D element of a fibrillarin-binding snoRNA.

22. Nucleic acid molecule according to claim 19 wherein Box C element has the sequence XGANWP and Box D element has the sequence YXVA, wherein X represents U, G or A preferably U, N represents any nucleotide preferably U, W represents G, C or U preferably G, P represents any nucleotide preferably A, Y represents C or U preferably C, and V represents G or U preferably G.

23. Nucleic acid molecule according to claim 20 wherein the protein binding sites comprise at least one H Box element and at least one ACA Box element of an H/ACA-type snoRNA.

24. Nucleic acid molecule according to claim 23 wherein the H Box element has the sequence ANANNA, wherein N represents any nucleotide, and the ACA Box element has the sequence ACA.

25. Nucleic acid molecule according to claim 17, wherein the non-translated functional RNA molecule comprises one or more antisense sequences or one or more ribozymes.

26. Nucleic acid molecule according to claim 17, wherein the precursor DNA contains a single coding sequence.

27. Nucleic acid molecule according to claim 17, wherein the precursor DNA contains a plurality of coding sequences, called a 〈〈 cluster 〉〉 of coding sequences, the coding sequences in the cluster being separated from each other by spacer sequences, wherein said spacer sequences are devoid of exon sequences and of associated functional intron splice sites.

28. Nucleic acid molecule according to claim 17, wherein the coding sequence or cluster of coding sequences is flanked by flanking sequences.

29. Nucleic acid molecule according to claim 26 or 27, wherein the flanking sequences are devoid of exon sequences and of associated functional intron splice sites.

30. Nucleic acid molecule according to claim 26 or 27 wherein the flanking sequences comprise exon sequences and intron splice sites.

31. Nucleic acid molecule according to claim 17 wherein the regulatory sequence is a promoter derived from a plant or yeast snoRNA gene cluster.

32. Nucleic acid molecule according to claim 17 wherein the regulatory sequence is an RNA polymerase II promoter.

33. Vector comprising the nucleic acid molecule according to any one of claims 15 to 32.

34. Stabilized RNA molecule comprising :
- a non-translated RNA sequence which is capable of functionally interacting with a cellular component, and
- stabilising sequences comprising at least one protein binding site and, optionally, sequences capable of giving rise to secondary structure associated with said protein binding site, said stabilising sequences being heterologous with respect to the non-translated RNA sequence,
and wherein said stabilised RNA molecule when produced in vivo is devoid of 5' cap sequences, and of 3' polyadenylated sequences.

35. Stabilised RNA molecule according to claim 34, wherein the non-translated functional RNA sequence comprises at least one antisense molecule or at least one ribozyme.

36. Stabilised RNA molecule according to claim 34 wherein the stabilising sequences comprise at least one protein binding site and sequences capable of forming at least one stem/loop structure.

37. Stabilised RNA molecule according to claim 35 wherein the protein binding sites comprise at least one Box C element and at least one Box D element of a fibrillarin-binding snoRNA.

38. Stabilised RNA molecule according to claim 37, wherein Box C element has the sequence XGANWP and Box D element has the sequence YXVA, wherein X represents U, G or A, preferably U, N represents any nucleotide preferably U, W represents G, C or U preferably G, P represents any nucleotide preferably A, Y represents C or U preferably C, and V represents G or U preferably G.

39. Stabilised RNA molecule according to claim 35 wherein the protein binding sites comprise at least one H Box element and at least one ACA Box element of an H/ACA-type snoRNA.

40. Stabilised RNA molecule according to claim 39, wherein the H Box element has the sequence ANANNA, wherein N represents any nucleotide, and the ACA Box element has the sequence ACA.

41. Stabilised RNA molecule according to claim 36 wherein a first protein binding site is positioned at the 5' end of the non-translated functional RNA, and a second protein binding site is positioned at the 3' end of the non-translated functional RNA.

42. Stabilised RNA molecule according to claim 36 wherein a stem structure is present and is formed by base-pairing between inverted repeat sequences at the the 5' and 3' extremities of the stabilised RNA molecule.

43. DNA molecule, or 〈〈 promoter 〉〉, capable of directing transcription of mono- and polycistronic RNA, comprising the upstream region of a higher plant snoRNA gene cluster.

44. DNA molecule according to claim 43, comprising :
- the conserved sequence illustrated in figure 16, or
- a fragment of said conserved sequence, capable of directing transcription of mono- and polycistronic RNA, or
- a homologous sequence presenting at least 70%, preferably 75%, and most preferably at least 85%, identity with the conserved sequence or with the fragment, said homologous sequence being capable of directing transcription of polycistronic RNA.

45. Chimeric gene comprising the DNA molecule according to claim 43 or 44, operably linked to a coding sequence which is other than a complete uninterrupted snoRNA coding sequence.

46. Nucleic acid molecule corresponding to or derived from a higher plant snoRNA gene cluster, wherein said molecule comprises or consists of a sequence having at least 20 bases, preferably of the sequence from nucleotide 1 to nucleotide 2165 illustrated in Figure 17, or of a sequence from nucleotide 1 to nucleotide 1587 in illustrated Figure 18, or of a sequence from nucleotide 1712 to 2034 illustrated in Figure 18, said molecule being devoid of U14 sequences.

47. Nucleic acid molecule according to claim 46 comprising or consisting of one or more of the following sequences :
nucleotides 1-850 of Figure 17
nucleotides 381-724 of Figure 17
nucleotides 853-945 of Figure 17
nucleotides 1695-1885 of Figure 17
nucleotides 1941-2095 of Figure 17
nucleotides 2165-2288 of Figure 17
nucleotides 2356-2479 of Figure 17
nucleotides 2621-2749 of Figure 17
nucleotides 2782-2911 of Figure 17
nucleotides 946-1694 of Figure 17
nucleotides 1886-1940 of Figure 17
nucleotides 2096-2164 of Figure 17
nucleotides 2288-2355 of Figure 17
nucleotides 2480-2620 of Figure 17
nucleotides 2750-2781 of Figure 17
nucleotides 2913-3557 of Figure 17
nucleotides 1-890 of Figure 18
nucleotides 891-983 of Figure 18
nucleotides 1101-1294 of Figure 18
nucleotides 1362-1512 of Figure 18
nucleotides 1588-1711 of Figure 18
nucleotides 1744-1873 of Figure 18
nucleotides 984-1100 of Figure 18
nucleotides 1295-1361 of Figure 18
nucleotides 1513-1587 of Figure 18
nucleotides 1712-1743 of Figure 18
nucleotides 1874-2034 of Figure 18

48. Process for producing stabilised non-translated RNA molecules within a cell, comprising introducing into a plant or yeast cell a nucleic acid molecule, called 〈〈 precursor DNA 〉〉, said precursor DNA containing a cluster of coding sequences, separated from each other by spacer sequences, each of said coding sequences encoding :
- a non-translated RNA sequence to be stabilised, and
- stabilising sequences derived from snoRNAs or functional equivalents of the said stabilising sequences,
wherein said spacer sequences are devoid of exon sequences and of associated functional intron splice sites,
said cluster being flanked by flanking sequences, which are heterologous with respect to the non-translated RNA sequence to be stabilised,
the precursor DNA being under the transcriptional control of regulatory sequences functional in said cell,
whereby transcription of the precursor DNA gives rise to a polycistronic transcript, and discrete stabilized RNA units consisting of the non-translated RNA sequence in association with the stabilising sequences are then produced by processing of the polycistronic transcript by the cell's endogenous processing machinery.

49. Process for producing a stabilised non-translated RNA molecule within a cell, comprising introducing into a plant or yeast cell, a nucleic acid molecule, called 〈〈 precursor DNA 〉〉, said precursor DNA containing a single coding sequence encoding :
- a non-translated RNA sequence to be stabilized, and
- stabilizing sequences derived from snoRNAs or functional equivalents thereof,
and flanking sequences on one or both sides of the coding sequence, wherein said flanking sequences are devoid of exon sequences and of associated functional intron splice sites, and are heterologous with respect to the non-translated RNA sequence to be stabilised,
the introduced precursor DNA being under the transcriptional control of regulatory sequences functional in said cell,
whereby a transcript of the precursor DNA is produced and a stabilised RNA unit comprising the non-translated RNA in association with the stabilising sequences, is produced by processing of the transcript by the cell's endogenous processing machinery.

50. Yeast or plant cell containing stabilised RNA according to any one of claims 34 to 42.

51. Yeast or plant cell containing the nucleic acid molecule according to any one of claims 17 to 32.

52. Plant comprising cells according to claims 49 or 50.

53. Yeast or plant cell containing the chimeric gene of claim 45.

54. Plant comprising cells according to claim 53.


SEQUENCE LISTING

(1) GENERAL INFORMATION:

(i) APPLICANT:
(A) NAME: GENE SHEARS PTY LTD
(B) STREET: Suite 1, Building 5 - 105 Delhi Road
(C) CITY: NORTH RYDE
(D) STATE: NSW
(E) COUNTRY: AUSTRALIA
(F) POSTAL CODE (ZIP): 2113

(ii) TITLE OF INVENTION: PROCESS FOR PRODUCING STABILISED RNA
MOLECULES WITHIN CELLS, RNA MOLECULES THUS PRODUCED AND
NUCLEIC ACID PRECURSORS OF SUCH MOLECULES

(iii) NUMBER OF SEQUENCES: 64

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97401480.5

(2) INFORMATION FOR SEQ ID NO: 1:

(i) SEQUENCE CHARACTERISTICS:
(A) LENGTH: 10 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..10
(D) OTHER INFORMATION:/note= "Inverted repeat sequence 1
page 16"

(xi) SEQUENCE DESCRIPTION: SEQ ID NO: 1:

TTGGGGGATT 10

(2) INFORMATION FOR SEQ ID NO: 2:

(i) SEQUENCE CHARACTERISTICS:
(A) LENGTH: 10 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..10
(D) OTHER INFORMATION:/note= "inverted repeat sequence n
2 page 16"

(xi) SEQUENCE DESCRIPTION: SEQ ID NO: 2:

AATCCCCCAA 10

(2) INFORMATION FOR SEQ ID NO: 3:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..31
(D) OTHER INFORMATION:/note= "primer PU14Ns2 page 54"

(xi) SEQUENCE DESCRIPTION: SEQ ID NO: 3:

GGGATGCATA AAGGGACTAC ACTGGAATTT G 31

(2) INFORMATION FOR SEQ ID NO: 4:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..29
(D) OTHER INFORMATION:/note= "primer PU14Nar page 54"

(xi) SEQUENCE DESCRIPTION: SEQ ID NO: 4:

CCATAGTGAG GCGCCTGTTA TTTCATTCC 29

(2) INFORMATION FOR SEQ ID NO: 5:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(ix) FEATURE:
(A) NAME/KEY: misc_feature
(B) LOCATION:1..34
(D) OTHER INFORMATION:/note= "primer polySH3 page 55"

(xi) SEQUENCE DESCRIPTION: SEQ ID NO: 5:

CGTCGACAAG GATCCAAGAG CTCTGCAGGT ACCA 34

(2) INFORMATION FOR SEQ ID NO: 6:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(ix) FEATURE:
(A) NAME/KEY: misc_feature
(B) LOCATION:1..42
(D) OTHER INFORMATION:/note= "primer polySH4 page 55"

(xi) SEQUENCE DESCRIPTION: SEQ ID NO: 6:

AGCTTGGTAC CTGCAGAGCT CTTGGTACCT TGTCGACGCA TG 42

(2) INFORMATION FOR SEQ ID NO: 7:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..30
(D) OTHER INFORMATION:/note= "primer M4USphI page 55"

(xi) SEQUENCE DESCRIPTION: SEQ ID NO: 7:

TCTTGCATGC TACCATATCC CTGTGTGACG 30

(2) INFORMATION FOR SEQ ID NO: 8:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..35
(D) OTHER INFORMATION:/note= "Primer snS1F page 61"

(xi) SEQUENCE DESCRIPTION: SEQ ID NO: 8:

AAGGATCCGA TAGCCTCCTA GTAGCTGTGA TGTGC 35

(2) INFORMATION FOR SEQ ID NO: 9:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(ix) FEATURE:
(A) NAME/KEY: misc_feature
(B) LOCATION:1..31
(D) OTHER INFORMATION:/note= "primer snS1R page 61"

(xi) SEQUENCE DESCRIPTION: SEQ ID NO: 9:

AAGAATTCGA GGGAGGAGCT CAGAGAGTTG C 31

(2) INFORMATION FOR SEQ ID NO: 10:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(ix) FEATURE:
(A) NAME/KEY: misc_feature
(B) LOCATION:1..32
(D) OTHER INFORMATION:/note= "primer snL1F page 61"

(xi) SEQUENCE DESCRIPTION: SEQ ID NO: 10:

AAGAATTCTT GTTTTAGTTT CCTGAATCTG CC 32

(2) INFORMATION FOR SEQ ID NO: 11:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(ix) FEATURE:
(A) NAME/KEY: misc_feature
(B) LOCATION:1..26
(D) OTHER INFORMATION:/note= "primer snL1R page 61"

(xi) SEQUENCE DESCRIPTION: SEQ ID NO: 11:

AAGAATTCTA CAGTCCCTCA GAGACC 26

(2) INFORMATION FOR SEQ ID NO: 12:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..25
(D) OTHER INFORMATION:/note= "primer snM1F page 61"

(xi) SEQUENCE DESCRIPTION: SEQ ID NO: 12:

AACTGCAGTA GTTTGGGCCT GATGC 25

(2) INFORMATION FOR SEQ ID NO: 13:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..28
(D) OTHER INFORMATION:/note= "primer snM1R page 61"

(xi) SEQUENCE DESCRIPTION: SEQ ID NO: 13:

AAGAATTCAT GTAATCACAG AAAACGGC 28

(2) INFORMATION FOR SEQ ID NO: 14:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..28
(D) OTHER INFORMATION:/note= "primer snoR3Hd page 61"

(xi) SEQUENCE DESCRIPTION: SEQ ID NO: 14:

GGGAAGCTTG TCCAGCTCTT CATCGTTC 28

(2) INFORMATION FOR SEQ ID NO: 15:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..26
(D) OTHER INFORMATION:/note= "primer L1Ns1 page 61"

(xi) SEQUENCE DESCRIPTION: SEQ ID NO: 15:

GGATGCATTT GCCTAGCTTC CTGATG 26

(2) INFORMATION FOR SEQ ID NO: 16:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..28
(D) OTHER INFORMATION:/note= "primer snoR3Hd page 62"

(xi) SEQUENCE DESCRIPTION: SEQ ID NO: 16:

GGGAAGCTTG TCCAGCTCTT CATCGTTC 28

(2) INFORMATION FOR SEQ ID NO: 17:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..28
(D) OTHER INFORMATION:/note= "primer snoR3Sp page 62"

(xi) SEQUENCE DESCRIPTION: SEQ ID NO: 17:

CCCACTAGTT TCGATCAGAA TATCTGGC 28

(2) INFORMATION FOR SEQ ID NO: 18:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..20
(D) OTHER INFORMATION:/note= "oligo DH51sph page 62"

(xi) SEQUENCE DESCRIPTION: SEQ ID NO: 18:

CATCGATGAT GCATCTGCAG 20

(2) INFORMATION FOR SEQ ID NO: 19:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..28
(D) OTHER INFORMATION:/note= "OLIGO DH51Bam page 62"

(xi) SEQUENCE DESCRIPTION: SEQ ID NO: 19:

GATCCTGCAG ATGCATCATC GATGCATG 28

(2) INFORMATION FOR SEQ ID NO: 20:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..19
(D) OTHER INFORMATION:/note= "oligo LegDRA page 62"

(xi) SEQUENCE DESCRIPTION: SEQ ID NO: 20:

CATAATCGAT GTTAACTTT 19

(2) INFORMATION FOR SEQ ID NO: 21:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..23
(D) OTHER INFORMATION:/note= "oligo LegSph page 62"

(xi) SEQUENCE DESCRIPTION: SEQ ID NO: 21:

AAAGTTAACA TCGATTATGC ATG 23

(2) INFORMATION FOR SEQ ID NO: 22:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..51
(D) OTHER INFORMATION:/note= "primer AMYNC page 63"

(xi) SEQUENCE DESCRIPTION: SEQ ID NO: 22:

ACCCTGCAGC GTAAGTAGTA GCTCAATGCA TAACCTTATC GATAACATTT C 51

(2) INFORMATION FOR SEQ ID NO: 23:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..29
(D) OTHER INFORMATION:/note= "primer MU144Cla page 63"

(xi) SEQUENCE DESCRIPTION: SEQ ID NO: 23:

GCCGATCGAT ATGGCTCAGA CATCCAAGG 29

(2) INFORMATION FOR SEQ ID NO: 24:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..21
(D) OTHER INFORMATION:/note= "primer MU14L14 page 64"

(xi) SEQUENCE DESCRIPTION: SEQ ID NO: 24:

CATAACCTTT GTGGTTTGGT C 21

(2) INFORMATION FOR SEQ ID NO: 25:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..35
(D) OTHER INFORMATION:/note= "primer MU141Cs page 64"

(xi) SEQUENCE DESCRIPTION: SEQ ID NO: 25:

CCTTTCGAAT CACACAGGCA ATAAACTGGA GGTGC 35

(2) INFORMATION FOR SEQ ID NO: 26:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..33
(D) OTHER INFORMATION:/note= "primer MU14.1NsSp page 64"

(xi) SEQUENCE DESCRIPTION: SEQ ID NO: 26:

CCGCATGCAT TAGCTTGAAC TAGAATGCCT GCG 33

(2) INFORMATION FOR SEQ ID NO: 27:

(i) SEQUENCE CHARACTERISTICS:
(A) LENGTH: 93 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..93
(D) OTHER INFORMATION:/note= "SNOR1.1 of drawing 3A"

(xi) SEQUENCE DESCRIPTION: SEQ ID NO: 27:

AGCTGTGATG TGCACTGCTT TCTTCTGACA CCTCTTGTAT GAGCAGGGAC TTTGTTCCCT 60

CCGTTGAGCA GATTCAGCAA CTCTCTGAGC TCC 93

(2) INFORMATION FOR SEQ ID NO: 28:

(i) SEQUENCE CHARACTERISTICS:
(A) LENGTH: 93 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..93
(D) OTHER INFORMATION:/note= "SNOR1.4 of drawing 3A"

(xi) SEQUENCE DESCRIPTION: SEQ ID NO: 28:

AGTTGTGAAG AGCACTGCTT TCTTCTGACA CCTCTTGTAT GAGCAGGGAT TTCGTTCCCT 60

CCGTTGAGCA GATCCAGCAA CTCTCTGAGC TCC 93

(2) INFORMATION FOR SEQ ID NO: 29:

(i) SEQUENCE CHARACTERISTICS:
(A) LENGTH: 191 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..191
(D) OTHER INFORMATION:/note= "U49.1 of drawing 3B"

(xi) SEQUENCE DESCRIPTION: SEQ ID NO: 29:

TTGTCCCAGT GAAGATCCCA AGACCTACCG ATAGGAAGTG CCGTTTGACA CTGCAAACAG 60

CAGCTGCCTC TCTCAGCTGC AGGTGTTCCA TCCTTTGACC ATAACCTTTG TGGTTTGGTC 120

AGGCTGGAGC TCCCATCTGC AGCTGTTTGT TTCAGCTGTG GTGTGTGATT TTAATCGGGT 180

CTCTGAGGGA C 191

(2) INFORMATION FOR SEQ ID NO: 30:

(i) SEQUENCE CHARACTERISTICS:
(A) LENGTH: 194 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..194
(D) OTHER INFORMATION:/note= "U49.2 of drawing 3B"

(xi) SEQUENCE DESCRIPTION: SEQ ID NO: 30:

TAGCCCCCGT GAAGATCTCA AGACCTACCG ATAGGAAGTG CCGTTTGACA CTGTAAACAG 60

CAGCTGCTGC ACTCAGCTGC AGGTGTTCCA TCTCTCGACC ATAACCTTTG TGGTTTGGTC 120

AGGCTGGATC TCCCATCTGC ATCTGCTCTG TGTTTCAGCT GTGGTGTGTG ATTAAGATCG 180

GGTCTCTGAG GGAC 194

(2) INFORMATION FOR SEQ ID NO: 31:

(i) SEQUENCE CHARACTERISTICS:
(A) LENGTH: 194 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..194
(D) OTHER INFORMATION:/note= "U49.4 of DRAWING F3B"

(xi) SEQUENCE DESCRIPTION: SEQ ID NO: 31:

TAGCCCCCGT GAAGATCTCA AGACCTACCG ATAGGAAGTG CCGTTTGACA CTGTAAACAG 60

CAGCTGCTGC ACACAGCTGC AGGTGTTCCA TCTCTCGACC ATAACCTTTG TGGTTTGGTC 120

AGGCTGGATC TCCCATCTGC ATCTGCTCTG TGTTTCAGCT GTGGTGTGTG ATTTAGATCG 180

GGTCTCTGAG GGAC 194

(2) INFORMATION FOR SEQ ID NO: 32:

(i) SEQUENCE CHARACTERISTICS:
(A) LENGTH: 157 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..157
(D) OTHER INFORMATION:/note= "SNOR2.1 of drawing 3C"

(xi) SEQUENCE DESCRIPTION: SEQ ID NO: 32:

AGTTTGGGCC TGATGCCACG TCTCTGGTAG CCACGAGCTT TGACCGGAGA TGCTATGATT 60

AGGCCGATAC AAAGGTGTTT CAATGTTTCA CATGTCGATA GGAACCTGGG GGCCGGCTTC 120

TATCTGTCCC ACAGAGCCGT TTTCTGTGAT TACAGTC 157

(2) INFORMATION FOR SEQ ID NO: 33:

(i) SEQUENCE CHARACTERISTICS:
(A) LENGTH: 147 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..147
(D) OTHER INFORMATION:/note= "SNOR2.3 of drawing 3C"

(xi) SEQUENCE DESCRIPTION: SEQ ID NO: 33:

AGTTTGGGCC TGATGCCACG TCTCTGGTAG CCACGAGCTT TGACCGGAGA TGCTATGATT 60

AGGCCGATAC AAAGGTGTTT CACATGTCGA TAGGAACCTG GGGACGGCTT CTATCTGTCC 120

ACAGAGCCGT TTTCTGTGAT TACAGTC 147

(2) INFORMATION FOR SEQ ID NO: 34:

(i) SEQUENCE CHARACTERISTICS:
(A) LENGTH: 150 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..150
(D) OTHER INFORMATION:/note= "SNOR2.2 of drawing F3C"

(xi) SEQUENCE DESCRIPTION: SEQ ID NO: 34:

TGTTTGGGCC TGATGCCACG TCTCTGGTAG CTACATGCTT TGACCCGAGA TGCTATGATC 60

AGGCCGATAC AAATTTGTTT CGCATGTTGA TAGGAACCTG GGGACGGCTT CTCCATCTGT 120

CCCACAGAGC CGGTTTCTGT GATCACATTC 150

(2) INFORMATION FOR SEQ ID NO: 35:

(i) SEQUENCE CHARACTERISTICS:
(A) LENGTH: 151 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..151
(D) OTHER INFORMATION:/note= "SNOR2.4 of drawing 3C"

(xi) SEQUENCE DESCRIPTION: SEQ ID NO: 35:

AGTTTGGGCC TGATGCCACG TCTCTGGTAG CTACATGCTT TGACCAGAGA TGCTATGATC 60

AGGCCGATAC AAAAGTCGTT TCACATGTTG ATAGGAACCT GGGGACCGGC TTCTCCATCT 120

GTCCACAGAG CCGGTTTCTG TGATCACATT C 151

(2) INFORMATION FOR SEQ ID NO: 36:

(i) SEQUENCE CHARACTERISTICS:
(A) LENGTH: 130 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..130
(D) OTHER INFORMATION:/note= "SNOR3.4 of drawing 3D"

(xi) SEQUENCE DESCRIPTION: SEQ ID NO: 36:

GGGTGTTGTT TTTTGATGTG ACGATCCTTT CCCCAAATTA CCGACTTTGA TTATACTGTG 60

CGATTTTTCG AGTTCTGTCT CCTCGGAGAT GGCTCTTAAG CCAGATATTC TGATCGAAAC 120

AACAACACCC 130

(2) INFORMATION FOR SEQ ID NO: 37:

(i) SEQUENCE CHARACTERISTICS:
(A) LENGTH: 76 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..76
(D) OTHER INFORMATION:/note= "SNOR3.2 of drawing 3D"

(xi) SEQUENCE DESCRIPTION: SEQ ID NO: 37:

GGGTGTTGTT TTTTGAAGTG ACGATCCTTT CCCCAAATTA CCGACTTTGA TTATACTGCG 60

CGATTTTTTG AGCTCT 76

(2) INFORMATION FOR SEQ ID NO: 38:

(i) SEQUENCE CHARACTERISTICS:
(A) LENGTH: 191 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..191
(D) OTHER INFORMATION:/note= "MzU49.1 of drawing 3E"

(xi) SEQUENCE DESCRIPTION: SEQ ID NO: 38:

TTGTCCCAGT GAAGATCCCA AGACCTACCG ATAGGAAGTG CCGTTTGACA CTGCAAACAG 60

CAGCTGCCTC TCTCAGCTGC AGGTGTTCCA TCCTTTGACC ATAACCTTTG TGGTTTGGTC 120

AGGCTGGAGC TCCCATCTGC AGCTGTTTGT TTCAGCTGTG GTGTGTGATT TTAATCGGGT 180

CTCTGAGGGA C 191

(2) INFORMATION FOR SEQ ID NO: 39:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..14
(D) OTHER INFORMATION:/note= "PLANT 18S of Drawing 4B"

(xi) SEQUENCE DESCRIPTION: SEQ ID NO: 39:

ACAUCCAAGG AAGG 14

(2) INFORMATION FOR SEQ ID NO: 40:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..18
(D) OTHER INFORMATION:/note= "U14 of drawing 4B"

(xi) SEQUENCE DESCRIPTION: SEQ ID NO: 40:

AGUCUGUAGG UUCCUUCC 18

(2) INFORMATION FOR SEQ ID NO: 41:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature

(B) LOCATION:1..14
(D) OTHER INFORMATION:/note= "HUMANS 18 S of drawing 4B"

(xi) SEQUENCE DESCRIPTION: SEQ ID NO: 41:

ACAUCCAAGG AAGG 14

(2) INFORMATION FOR SEQ ID NO: 42:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..18
(D) OTHER INFORMATION:/note= "U14 of drawing 4B"

(xi) SEQUENCE DESCRIPTION: SEQ ID NO: 42:

AGUCUGUAGG UUCCUUCC 18

(2) INFORMATION FOR SEQ ID NO: 43:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..15
(D) OTHER INFORMATION:/note= "PLANT 25S of drawing 4C"

(xi) SEQUENCE DESCRIPTION: SEQ ID NO: 43:

CUAGAGGUGU CAGAA 15

(2) INFORMATION FOR SEQ ID NO: 44:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..20
(D) OTHER INFORMATION:/note= "snoR1 of drawing F4C"

(xi) SEQUENCE DESCRIPTION: SEQ ID NO: 44:

AGUAUGUUCU CCACAGUCUU 20

(2) INFORMATION FOR SEQ ID NO: 45:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..14
(D) OTHER INFORMATION:/note= "HUMAN 28S of drawing 4C"

(xi) SEQUENCE DESCRIPTION: SEQ ID NO: 45:

AGGAGGUGUC AGAA 14

(2) INFORMATION FOR SEQ ID NO: 46:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..18
(D) OTHER INFORMATION:/note= "U60 of drawing 4C"

(xi) SEQUENCE DESCRIPTION: SEQ ID NO: 46:

AGUAUGCUCC ACAGUCUU 18

(2) INFORMATION FOR SEQ ID NO: 47:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..15
(D) OTHER INFORMATION:/note= "yeast 26S of drawing 4C"

(xi) SEQUENCE DESCRIPTION: SEQ ID NO: 47:

CUAGAGGUGC CAGAA 15

(2) INFORMATION FOR SEQ ID NO: 48:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..14
(D) OTHER INFORMATION:/note= "PLANT 25S of drawing 4D"

(xi) SEQUENCE DESCRIPTION: SEQ ID NO: 48:

CGGCUCUUCC UAUC 14

(2) INFORMATION FOR SEQ ID NO: 49:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..19
(D) OTHER INFORMATION:/note= "U49 of drawing 4D"

(xi) SEQUENCE DESCRIPTION: SEQ ID NO: 49:

AGUUUGCCGU GAAGGAUAG 19

(2) INFORMATION FOR SEQ ID NO: 50:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..13
(D) OTHER INFORMATION:/note= "HUMAN 28S of drawing 4D"

(xi) SEQUENCE DESCRIPTION: SEQ ID NO: 50:

CGGCUCUUCC UAU 13

(2) INFORMATION FOR SEQ ID NO: 51:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..18
(D) OTHER INFORMATION:/note= "U49 of drawing 4D"

(xi) SEQUENCE DESCRIPTION: SEQ ID NO: 51:

AGACUGCCGU GAAGGAUA 18

(2) INFORMATION FOR SEQ ID NO: 52:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..12
(D) OTHER INFORMATION:/note= "PLANT 25S of drawing 4E"

(xi) SEQUENCE DESCRIPTION: SEQ ID NO: 52:

UGAACGGCAC UU 12

(2) INFORMATION FOR SEQ ID NO: 53:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..13
(D) OTHER INFORMATION:/note= "U49 of drawing 4E"

(xi) SEQUENCE DESCRIPTION: SEQ ID NO: 53:

AGUUUGCCGU GAA 13

(2) INFORMATION FOR SEQ ID NO: 54:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..12
(D) OTHER INFORMATION:/note= "HUMAN 28S of drawing 4E"

(xi) SEQUENCE DESCRIPTION: SEQ ID NO: 54:

UGAACAGCAG UU 12

(2) INFORMATION FOR SEQ ID NO: 55:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..15
(D) OTHER INFORMATION:/note= "PLANT 25S of drawing 4F"

(xi) SEQUENCE DESCRIPTION: SEQ ID NO: 55:

GGGAUAACUG GCUUG 15

(2) INFORMATION FOR SEQ ID NO: 56:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..17
(D) OTHER INFORMATION:/note= "snoR3 of drawing 4F"

(xi) SEQUENCE DESCRIPTION: SEQ ID NO: 56:

AGUCUUAUAG ACCGAAU 17

(2) INFORMATION FOR SEQ ID NO: 57:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..13
(D) OTHER INFORMATION:/note= "YEAST 26S of drawing 4F"

(xi) SEQUENCE DESCRIPTION: SEQ ID NO: 57:

ACAGGGAUAA CUG 13

(2) INFORMATION FOR SEQ ID NO: 58:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: snRNA

(ix) FEATURE:
(A) NAME/KEY: misc_feature
(B) LOCATION:1..17
(D) OTHER INFORMATION:/note= "snR38 of drawing 4F"

(xi) SEQUENCE DESCRIPTION: SEQ ID NO: 58:

AAUCUGUCCC UAUUGAC 17

(2) INFORMATION FOR SEQ ID NO: 59:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..27
(D) OTHER INFORMATION:/note= "Drawing 8B"

(xi) SEQUENCE DESCRIPTION: SEQ ID NO: 59:

GCATGCATAA TCGATGTTAA CTTTAAA 27

(2) INFORMATION FOR SEQ ID NO: 60:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..39
(D) OTHER INFORMATION:/note= "Drawing 8C"

(xi) SEQUENCE DESCRIPTION: SEQ ID NO: 60:

ATGCAGCGTA AGTAGTAGCT CAATGCATAA CCTATCGAT 39

(2) INFORMATION FOR SEQ ID NO: 61:

(i) SEQUENCE CHARACTERISTICS:
(A) LENGTH: 344 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..344
(D) OTHER INFORMATION:/note= "DRAWING 16, top line"

(xi) SEQUENCE DESCRIPTION: SEQ ID NO: 61:

GCTTTCGGCT AGGCGGGCTA AACAGTGAAA AAGAACCGGT GGCCCAGGCT TATTTTAAAG 60

TCCATGGCTC GCTTACTTGC TTTGACATGT ACCGGCCCAA TTCAGCTCAG CCCAACTCAA 120

TCCGACCCCT CGTCTTTATA ACTCGGCCGT GGCCGCGCCG CATTCTCAGC GCTCACCTGG 180

CTCGCTACTC GCTAGGGTTC AATCCTTGCG CCGCCGCCAC CTACTCGAGC CCTCGCGCTA 240

GCCCCCTACG CCACCTAGCC GTTCGCCGTG CGTCGAGTCC TCCCAGCCGC CTCCCGCTCG 300

TTGCGACCTG CAGGCCTCGG TCATCCGGCG GTGTTCTTCC GGCC 344

(2) INFORMATION FOR SEQ ID NO: 62:

(i) SEQUENCE CHARACTERISTICS:
(A) LENGTH: 342 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..342
(D) OTHER INFORMATION:/note= "DRAWING 16, bottom line"

(xi) SEQUENCE DESCRIPTION: SEQ ID NO: 62:

GCTGTCTGCT AGGGGGGCTC AGCACTGAAA AGAACAAGCG AGCCAGGCTT TTCATTTGGC 60

CCAGGGCTCG CATACTTGCT TTGGTACAGG CTCGGCTCGG CCCAAGCCGT CTCAGGCCCG 120

ATCCCGATCC CACTCCCTCG GGGGGCTCGC CTTTATAACT CGTCCGTGGC CACCCACCCT 180

TCTTCACAGA GCTTCGCTCA CCTGCCTTGC TAGGGTTTAA GCCACCACCC GCCGCCGCCA 240

CCTATTCCAG CTCTCGCGCT TCGCCTTGTG GCGACGATGC ATCCGGTCTC GTTGTCGTTG 300

CGAGCTCCTC GCGTCTGTCG TCAGGCGTTG TTGTTCCGGC C 341

(2) INFORMATION FOR SEQ ID NO: 63:

(i) SEQUENCE CHARACTERISTICS:
(A) LENGTH: 3557 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..3557
(D) OTHER INFORMATION:/note= "DRAWING 17, top line"

(xi) SEQUENCE DESCRIPTION: SEQ ID NO: 63:

GAATTCATAC CTCACACCCA ATCCATGGTG GTATTAAATC CATCATTTAA TTCATGTATC 60

TCTCAAAGCT AGTTATTCTT TTGATCCATA AATTCTAATA TAAACTTGTG CAAAATTTCA 120

TGGATTTGTT TTATACGTAA TGAGCTATGG ATAAAATCCA TGCACTCCGT GGTTTTAAAA 180

AATCTCAAAC CCTTGGGTTA TAATCCATAA TGAGTTTAAT CGAATAAAAT TTTTTTAAGT 240

GAGTTTAAAT TATTTAAAGG CATGGATAGT GGCCAATTTG ATTCTAATCC ATGCTAATCC 300

AGGACCTCAG CAGCGTTCTC CCGTGCCCGT TTTTCTTCAT CGTTAATCAT CGTCGTTCAT 360

CGTTAATCAT CGTCGGCCCG GCTTTCGGCT AGGCGGGCTA AACAGTGAAA AAGAACCGGT 420

GGCCCAGGCT TATTTTAAAG TCCATGGCTC GCTTACTTGC TTTGACATGT ACCGGCCCAA 480

TTCAGCTCAG CCCAACTCAA TCCGACCCCT CGTCTTTATA ACTCGGCCGT GGCCGCGCCG 540

CATTCTCAGC GCTCACCTGG CTCGCTACTC GCTAGGGTTC AATCCTTGCG CCGCCGCCAC 600

CTACTCGAGC CCTCGCGCTA GCCCCCTACG CCACCTAGCC GTTCGCCGTG CGTCGAGTCC 660

TCCCAGCCGC CTCCCGCTCG TTGCGACCTG CAGGCCTCGG TCATCCGGCG GTGTTCTTCC 720

GGCCTTTCGT ACTGTCGCTC ATGAGTCCCG TTCTGTCGTG AATTTGCATG CAGTAGCTTG 780

AACTAGAATG CCTGCGGATT AGTCTGCCTT CTGTTTTTAT TCATCGGAGC CAGGTTACGA 840

TATGGTGGTA GTAGCTGTGA TGTGCACTGC TTTCTTCTGA CACCTCTTGT ATGAGCAGGG 900

ACTTTGTTCC CTCCGTTGAG CAGATTCAGC AACTCTCTGA GCTCCTCCCT CGCATTTTTT 960

CTGAGCTGAC CTGGCCATTT GCTCCGTCAT TTAGCATTTT ATTACGAGTT TGTGAGATCG 1020

TAGGTCTTTA CTTTTTCTTA GATGACGCCG ACTGTGCTGT GACGAGCTAC TCCTTATTTC 1080

TGACACCTTG TGTGAGCCTT GAGCAGATTA GCAACTCTCT GGATCGTCAA TTTCGTGTGC 1140

TATTCTTGAT ATGCCCTTTG TTCAATTAGT GAATTTGCAT TCGTCAATCT GACTAGTGAC 1200

TTACATGAAT CCTTTTCGTT TGGCATGAGA GGATGAAAGC TTGAAACAAT CTTAGGACAC 1260

CTTCTGACAC AGCGCGTTAC ATCACGTTGT GAATGAGAAG GCAATTCTTT TCTGATGATG 1320
CTGTGCTTCA ATATGTTACA AATCACATGT TTTTGGATTA TTTGTTGTCT ACTTTTGTTT 1380
GTGGGTTGGG AGCATGTGCG CTATTTCTTT CACCCATATT TCATTTTTTT ATGATGATGT 1440
GTTGATGTAG TTATCCTTCA ATTTGCCCTG TTTTAGTTGT GATAACTTGA TAGAAAATTG 1500
CCTAGCTTCC TGATGATTAA CCTGTTAACC AATGAAATCA GCTACATTTG CAATTTGTTT 1560
TGAACTAGAT TTGTAATGGC ATTTAATCCT AGAATTGCAT TCACCTAGAT TTGCTGTGTT 1620
TAGATTCTTC TGTGCAGTTC TAGGGAATGT TTGTTTTAGT TTCCTGAATC TGCCATGAGA 1680
GTAGTTTGGC TATGTTGTCC CAGTGAAGAT CCCAAGACCT ACCGATAGGA AGTGCCGTTT 1740
GACACTGCAA ACAGCAGCTG CCTCTCTCAG CTGCAGGTGT TCCATCCTTT GACCATAACC 1800
TTTGTGGTTT GGTCAGGCTG GAGCTCCCAT CTGCAGCTGT TTGTTTCAGC TGTGGTGTGT 1860
GATTTTAATC GGGTCTCTGA GGGACTGTAC TAAATCTGCT GTTATGTATG ATTTCATTAT 1920
CTTGATGTCA ATGCTGAAGT AGTTTGGGCC TGATGCCACG TCTCTGGTAG CCACGAGCTT 1980
TGACCGGAGA TGCTATGATT AGGCCGATAC AAAGGTGTTT CAATGTTTCA CATGTCGATA 2040
GGAACCTGGG GGCGGCTTCT ATCTGTCCAC AGAGCCGTTT TCTGTGATTA CAGTCATTGC 2100
TTTACTATAA TTATTGTTAG TGCTTTGCCT TCAGCTCATT TTGTTCCGTA TATTTGTTAT 2160
GCATATGGCA ATGATGTTGA AGTTAAAGGC TTGTTTCTCA ACATTCGCAG TAGCCGCCTA 2220
AGAGCTTTCG CCCTGCCAGG CTTGAGAGCT TGTGCTGTTT AATCCTTCCT TGGATGTCTG 2280
AGCCATACCT GTTCTTGTCC AGCTCATGAT TTTGTCAGTA CGTTCTGTAT TATCTTGCTG 2340
TTTTATTAGC TGTTGTGCAT TGCAAGTGAT GATGAAGTCA AGGCTTGTTT CTCTACATTC 2400
GCAGTTGCCG CCTAAGAGCT TTCGCCCTGC CAGGCTTGAG AGCTAGTGCT GCCAAATCCT 2460
TCCTTGGATG TCTGATGCAA TGCAATACCT ATGTTTGATA CTTGTATTTA GCTTGTGCTG 2520
TTTAATCCTT CCTTGGATGT CTGAGACATA CCTGTTCTTG TCCAGCTCAT GATTTTTTTT 2580
CCAGTACATT CTGTATATCC TGCTGTTTTA TTAGCTGTTG TGCATTGCAA GTGATGACAA 2640
AATCAAGGCT TGTTTCTCGA CATTCGCAGT TGCCGCCTAA GAGCTTTCGC CCTGCCAGGC 2700
TTGAGAGGTA GTGCTGCCAA ATCCTTCCTT GGATGTCTGA CGCAATGCAA TACCTATGTT 2760
TGATACTTGT TATTTCTGAT AATCGGTGCC CTATGATGAC AAAATCAAGG CTTGTTTCTC 2820
TACATTCGCA GTTGCCGCCT AAGAGCTTTC GCCCTGCCAG GCTTGAGAGC TAATGCTGCA 2880
GAATCCTTCC TTGGATGTCT GAGGGCCGCC GCAAAAAAGT ACTGAACTGC TGTATTTTGC 2940
TATGCTTGTA AGATCACCAT CCAGAATTTC CCAACTGTTT CTAGCGCACC TCCAGTTTAT 3000
TGCCTGTGTG AAGCAAAAGG AGCATCCAAT TCGTTTCTTG GTCATGGGTT CTGTAGTTTA 3060

TAGTTACGGC TGGTTCATTG CAATGTTGTG CGAGCGGGTA ATGTATTTTT GAACAGAGAT 3120

TGATTTGTTT TTAATGACAA TGAATCAAGT CTCGAGAATC CATCCGATTT GTATAAGTTT 3180

CATCAGTTTG AAGAAATGTG ATGACCTGAC GCAAATTCTG ATTTGTAAGT TTGCATAGAT 3240

CTGAAGATTC TAAGCATGAG CGCGGTACAA CTAAAGTTGC AAAGGCTGGC TGCATATTTT 3300

TTCTTGGTAG AATATCCTTT GATCTAATTA TGTAAAGAAA CTGGAACTTT TCTGTATTGA 3360

TCAGTCCATG TTTGGGCACA GTAGCATTGT GCCTTTGTAT GTGATGAAGT AGAGCTCAAA 3420

GTTTAATGTA TTTTTTTAAA AAAAGTACAG GCAACAGAAG TACTGTTCAT TACTATGCTC 3480

ATGTGACTGA ACCGCTATGT TTTGCACCCC AACGGACTGC CTACTGCTGT ATCTTCTTGT 3540

TTATTCTGTT GCTGCAG 3557

(2) INFORMATION FOR SEQ ID NO: 64:

(i) SEQUENCE CHARACTERISTICS:
(A) LENGTH: 2034 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: misc_feature
(B) LOCATION:1..2034
(D) OTHER INFORMATION:/note= "DRAWING 18, top line"

(xi) SEQUENCE DESCRIPTION: SEQ ID NO: 64:

GAATTCATTC AACAACAAAC TAAGGGCGGA GAGCTGGAAC CGCTCCTAAT CCAGGGGGTC 60

TTCTAGAACG TTGACCTTGC TGATCTCCGA GTAGATTTGG TCGAGATCAG AGTTGAACGG 120

TTATATAAAG CGACCATCGC CCTTTGATTT GACGCGGTGA ACCGTTAAAC CTAGGATAAT 180

TTTAGCTGGT CTCCATCTCT ACCAGTGAAA AAGTTACCGT CTACGTTCTC ACTAATATAA 240

AAGGCAGTCT TAAAAGTACT AAATCCTTAG CCATCGCATA CAAATAAAAT TCTAGAATTT 300

TTTTAATTTC TAAGAACGTG CAAGATTATC TTAGATGTAT AGTAAACACA AAGTGATACG 360

ATAGGTCATA AAACTAGTTT CTTGCTTTTC AGTTACACAT CAAAACTTAC TGTAACATTT 420

TTTAATTATC TATATCTTTT ACAAGGGATC AACGCTGTCT GCTAGGGGGG CTCAGCACTG 480

AAAAGAACAA GCGAGCCAGG CTTTTCATTT GGCCCAGGGC TCGCATACTT GCTTTGGTAC 540

AGGCTCGGCT CGGCCCAAGC CGTCTCAGGC CCGATCCCGA TCCCACTCCC TCGGGGGGGC 600

TCGCCTTTAT AACTCGTCCG TGGCCACCCA CCCTTCTTCA CAGAGCTTCG CTCACCTGCC 660
TTGCTAGGGT TTAAGCCACC ACCCGCCGCC GCCACCTATT CCAGCTCTCG CGCTTCGCCT 720
TGTGGCGACG ATGCATCCGG TCTCGTTGTC GTTGCGAGCT CCTCGCGTCT GTCGTCAGGC 780
GTTGTTGTTC CGGCCTCGTG TGCCATGGTT GCTTTCCTGA ATGCCTGCGA TTCGGCCTGA 840
GTTTTGTTTT ATACATTCAT TCATTCGTAT TCGTCACACA GGGATATGGT AGTTGTGAAG 900
AGCACTGCTT TCTTCTGACA CCTCTTGTAT GAGCAGGGAT TTCGTTCCCT CCGTTGAGCA 960
GATCCAGCAA CTCTCTGAGC TCCTCCCTAG CGGTCTGTTT CCTGAGTTTC ATCTTTGTAG 1020
TTCCAGCGTA ATTTTTTTCT ACTTTCAAGT AATTTTACTT TGCTGCATCT GCGAACTCGT 1080
GAGATGATAG TTCACCTACG TAGCCCCCGT GAAGATCTCA AGACCTACCG ATAGGAAGTG 1140
CCGTTTGACA CTGTAAACAG CAGCTGCTGC ACACAGCTGC AGGTGTTCCA TCTCTCGACC 1200
ATAACCTTTG TGGTTTGGTC AGGCTGGATC TCCCATCTGC ATCTGCTCTG TGTTTCAGCT 1260
GTGGTGTGTG ATTTAGATCG GGTCTCTGAG GGACTCTGCA GGACTGTCAC TATGTCTTGA 1320
TTTGCTGTGT TTACACCGTG CTGTTCTCAT TTACGTTTAA TAGTTTGGGC CTGATGCCAC 1380
GTCTCTGGTA GCTACATGCT TTGACCAGAG ATGCTATGAT CAGGCCGATA CAAAAGTCGT 1440
TTCACATGTT GATAGGAACC TGGGGACGGC TTCTCCATCT GTCCCACAGA GCCGGTTTCT 1500
GTGATCACAT TCTATCTTCA TTACTATGAT TATGATTGAT GTTTCACCAT TTTTTACTTA 1560
TTTTTTATAT GTATACTACA TGCATATTAT GGCAATGATG ATGAAAGATA AGGCTTGTTT 1620
CTCAACATTC GCAGTAGCCG CCTAAGAGCT TTCGCCACGC CAGGCTCGAG AGCTTGTGCT 1680
GTTGAATCCT TCCTTGGATG TCTGAGCCAT ATCTCTCTTC TTGTCCAGCT CTTCATCGTT 1740
CTTGGGTGTT GTTTTTTGAT GTGACGATCC TTTCCCCAAA TTACCGACTT TGATTATACT 1800
GTGCGATTTT TCGAGTTCTG TCTCCTCGGA GATGGCTCTT AAGCCAGATA TTCTGATCGA 1860
AACAACAACA CCCAGTTTCC TCTTAAAACA CATCATTCCA TCCGCATGTT CTTGACTTGG 1920
ATCTTGGAAA TTTTTTTGGT CTCTTTGACG ATGGAAATGA GGACCCTTTC CCCAGCTGAT 1980
CGACTACGAT TACACTGTGC GCAATTGCGA TGCCTTCTCC GCGGAGAAAG GATC 2034